(19) **Europäisches Patentamt**
**European Patent Office**
**Office européen des brevets**

(11) **EP 3 077 533 B1**

(12) **EUROPEAN PATENT SPECIFICATION**

(45) Date of publication and mention
of the grant of the patent:
**24.04.2019 Bulletin 2019/17**

(51) Int Cl.:
***C12Q 1/6883*** *(2018.01)*

(21) Application number: **14825110.1**

(86) International application number:
**PCT/EP2014/076907**

(22) Date of filing: **08.12.2014**

(87) International publication number:
**WO 2015/082721 (11.06.2015 Gazette 2015/23)**

(54) **METHODS FOR ESTABLISHING A CLINICAL PROGNOSIS OF DISEASES ASSOCIATED WITH THE FORMATION OF AGGREGATES OF ABETA1-42**

VERFAHREN ZUR ERSTELLUNG EINER KLINISCHEN PROGNOSE VON ERKRANKUNGEN IN ZUSAMMENHANG MIT DER BILDUNG VON AGGREGATEN VON ABETA1-42

PROCÉDÉS PERMETTANT D'ÉTABLIR UN PRONOSTIC CLINIQUE DE MALADIES ASSOCIÉES À LA FORMATION D'AGRÉGATS D'ABETA1-42

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.12.2013 EP 13196119**

(43) Date of publication of application:
**12.10.2016 Bulletin 2016/41**

(73) Proprietors:
• **Life & Brain GmbH**
**53127 Bonn (DE)**
• **Rheinische Friedrich-Wilhelms-Universität Bonn**
**53113 Bonn (DE)**

(72) Inventors:
• **BECKER, Tim**
**56075 Koblenz (DE)**
• **HENEKA, Michael Thomas**
**53127 Bonn (DE)**
• **KUNZ, Wolfram**
**56564 Neuwied (DE)**
• **ZUNIGA, Alfredo Ramirez**
**53757 Sankt Augustin (DE)**
• **NÖTHEN, Markus**
**50968 Köln (DE)**

(74) Representative: **Zwicker, Jörk**
**ZSP Patentanwälte PartG mbB**
**Hansastraße 32**
**80686 München (DE)**

(56) References cited:
**EP-A1- 2 009 112      WO-A1-2009/112882
WO-A1-2011/007155    WO-A2-2006/028586
WO-A2-2013/066764    WO-A2-2013/071119**

• **Anonymous: "Reference SNP (refSNP) Cluster Report: rs62256378", , 26 February 2008 (2008-02-26), XP055107912, Retrieved from the Internet: URL:http://www.ncbi.nlm.nih.gov/projects/S NP/snp_ref.cgi?rs=62256378 [retrieved on 2014-03-14]**
• **AFFYMETRIX: "Data Sheet Affymetrix(R) Genome-Wide Human SNP Array 6.0", INTERNET CITATION, 2007, pages 1-4, XP002525407, Retrieved from the Internet: URL:http://www.affymetrix.com/support/tech nical/datasheets/genomewide_snp6_datasheet . pdf [retrieved on 2009-04-10]**
• **OSTERGAARD E: "Disorders caused by deficiency of succinate-CoA ligase", JOURNAL OF INHERITED METABOLIC DISEASE, KLUWER ACADEMIC PUBLISHERS, DO, vol. 31, no. 2, 4 April 2008 (2008-04-04), pages 226-229, XP019600524, ISSN: 1573-2665**

## Description

### Field of invention

The present invention relates to novel methods for establishing the prediction of the course of neurodegenerative diseases such as Alzheimer's disease based on genotyping single nucleotide polymorphism rs62256378 and or at least one SNP in linkage disequilibrium therewith. Moreover a novel method for screening pharmaceutical compounds which may be useful for the treatment of AD are disclosed.

### Background

Alzheimer's disease (AD) is a complex disorder in which several pathways contribute to pathology and clinical phenotype. The prevalence of AD is increasing in most industrialised countries, thus creating a major burden for the health system and causing considerable suffering of the patients and their relatives. Delineation of each pathological pathway, and identification of the factors which modulate them, are crucial for the development of effective treatment. Information on individual pathological pathways is provided by biomarkers. In AD, cerebrospinal fluid (CSF) levels of $A\beta_{1-42}$ and phosphorylated Tau (pTau) reflect cerebral amyloid deposition and tau related neurodegeneration, respectively. However, a number of biological factors that influence these core AD pathways are still elusive.

International patent applications WO 2006/028586 A2 and WO 2011/007155 A1 disclose biomarkers suitable for the diagnosis and/or prognosis of Alzheimer's disease.

### Summary of the Invention

In a first aspect, the present invention relates to a method for establishing a prognosis of a patient suffering from a disease or condition associated with the formation of aggregates of $A\beta_{1-42}$ comprising the steps of

a) detecting whether an adenine or another nucleotide, preferably guanine, is present at single nucleotide polymorphism (SNP) rs62256378 and/or whether a protective allele or another allele is present at at least one SNP in linkage disequilibrium with rs62256378;

b) establishing a clinical prognosis based on the type of nucleotide present at the site of the SNP, wherein

(i) the presence of an adenine at rs62256378 and/or the presence of a protective allele at at least one SNP in linkage disequilibrium with rs62256378 is indicative of a good prognosis or
(ii) the presence of another nucleotide than adenine at rs62256378and/or the presence of a another allele at at least one SNP in linkage disequilibrium with rs62256378 is indicative of a bad prognosis,

wherein the at least one SNP in linkage disequilibrium with rs62256378 is selected from the group consisting of rs74727963, rs78609144, rs79963093, rs80028595, rs77072359, rs74680427, rs111796700, rs115298081, rs72626930, rs76856038, rs77836520, rs116341583, rs114506918 andrs745742418, and wherein the SNP in linkage disequilibrium with rs62256378 displays an r 2 of at least 0.5.

In a second aspect, the present invention relates to a suitable medicament for use in the treatment of a disease or condition associated with the formation of aggregates of $A\beta_{1-42}$ in patients having another nucleotide than adenine at rs62256378 and/or another allele at at least one SNP in linkage disequilibrium with rs62256378 as determined by the method of any one of claims 1 or 2,wherein the medicament is selected from the group consisting of donepezil, galantamine, memantine, rivastigmine, tacrine, vitamin E, citalopram, fluoxetine, paroxeine, sertraline, trazodone, lorazepam, oxazepam, aripiprazole, clozapine, haloperidol, olanzapine, quetiapine, risperidone, and ziprasidone.

In a third aspect, the present invention relates to the use of a kit for establishing a clinical prognosis of a patient suffering from a disease or condition associated with the formation of aggregates of $A\beta_{1-42}$, which comprises at least one means for detecting the presence of adenine or guanine at SNP rs62256378, comprising an oligonucleotide comprising at least 8 bases of SEQ ID NO: 1 or a complement thereof, wherein said at least 8 bases comprise position 27.

In a fourth aspect, the present invention relates to a method for screening compounds to identify therapeutic candidates for the modulation of a disease or condition associated with the formation of aggregates of $A\beta_{1-42}$ comprising the steps of

a) contacting (i) a brain, blood, or liver cell which is capable of expressing the GTP-specific beta subunit of the succinyl-CoA ligase (SUCLG2) in vitro or (ii) a cell which expresses SUCLG2 in vitro or (iii) a solution comprising SUCLG2 with a test compound or (iv) administering the test compound to an experimental animal, wherein the animal is a non-human animal;

b) detecting in case of (i) whether said test compound increases the expression level or the activity of SUCLG2 in vitro, in case of (ii) whether said test compound increases the expression level or the activity of SUCLG2 in vitro, in case of (iii) whether said test compound increases the activity of SUCLG2, in case (iv) whether said test compound increases the expression level or activity of SUCLG2, wherein an increase of the expression level of activity indicates that said test compound is a candidate compound for treating the disease or condition in vivo.

Description of the Figures

**Figure 1:** Q-Q plots (A) for $A\beta_{1-42}$ and (B) for $pTau_{181}$

**Figure 2:** Genome-wide signal intensity (Manhattan) Plots.
A. Results for the association with cerebrospinal fluid (CSF) $A\beta_{1-42}$. B. Results for the association with CSF $pTau_{181}$. Both plots show the individual p-values against genomic position. Chromosome number is shown on the x axis. The results within each chromosome are plotted left to right, with left being the p-terminal end of the corresponding chromosome. Horizontal dashed lines indicate a p-value threshold of $5\times10^{-8}$ (i.e. genome-wide significance). Green dots depict single nucleotide polymorphisms that are genome-wide significant in the genome-wide association study step.

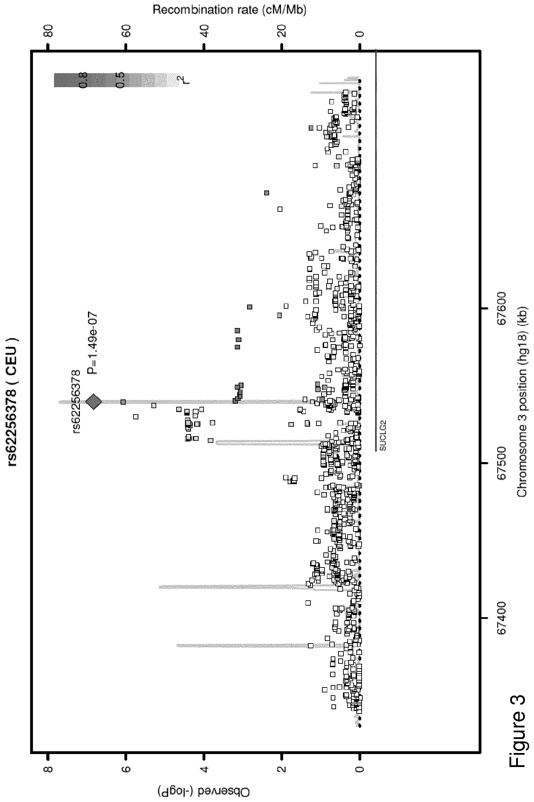

**Figure 3:** Regional plot for rs62256378 locus. Regional plots for the *SUCLG2* (rs62256378) locus. The diamond shows rs62256378. Degree of LD ($r^2$) between SNPs is shown in a greyscale chart.

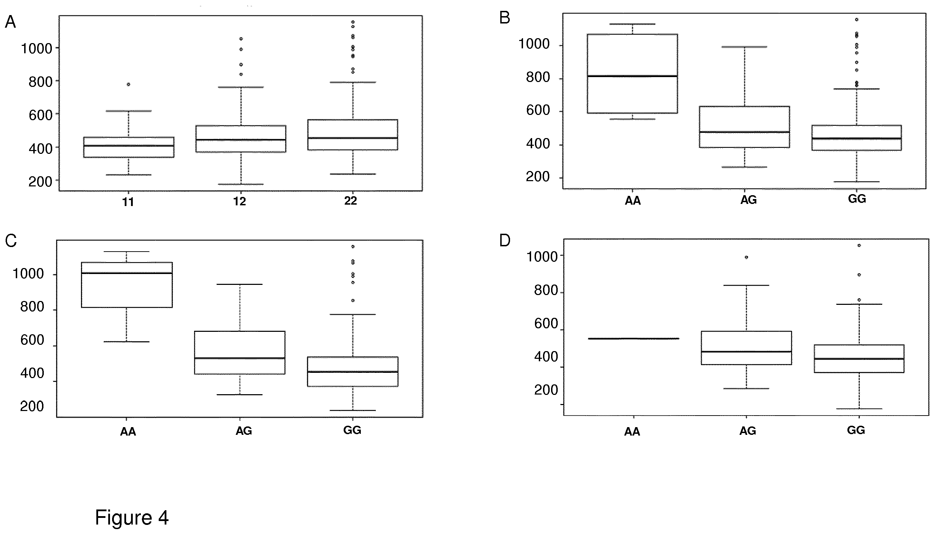

**Figure 4:** Effect of the interaction between APOE-$\varepsilon$4 status and rs62256378 on CSF A$\beta$1-42 levels
Effect of the interaction between APOE $\varepsilon$4 status and rs62256378 genotype on cerebrospinal fluid (CSF) $A\beta_{1-42}$ levels in AD patients. A. Box plot showing the effect of the APOE-$\varepsilon$4 status upon $A\beta_{1-42}$ levels. APOE-$\varepsilon$4 homozygous is coded as (11), APOE-$\varepsilon$4 heterozygous is coded (12), and APOE-$\varepsilon$4 noncarriers are coded (22). B. Box plot depicting levels of CSF $A\beta_{1-42}$ depending on rs62256378 genotypes. C. Box plot depicting levels of CSF $A\beta_{1-42}$ depending on rs62256378 genotypes within the AD patients negative for APOE-$\varepsilon$4. D. Box plot depicting levels of CSF $A\beta_{1-42}$ depending on rs62256378 genotypes within the AD patients group heterozygous for APOE-$\varepsilon$4. The lower, upper and the middle lines of boxes correspond to 25th and 75th percentiles and medians, respectively. The whiskers at the top and bottom extend from the 95th and 5th percentile, respectively.

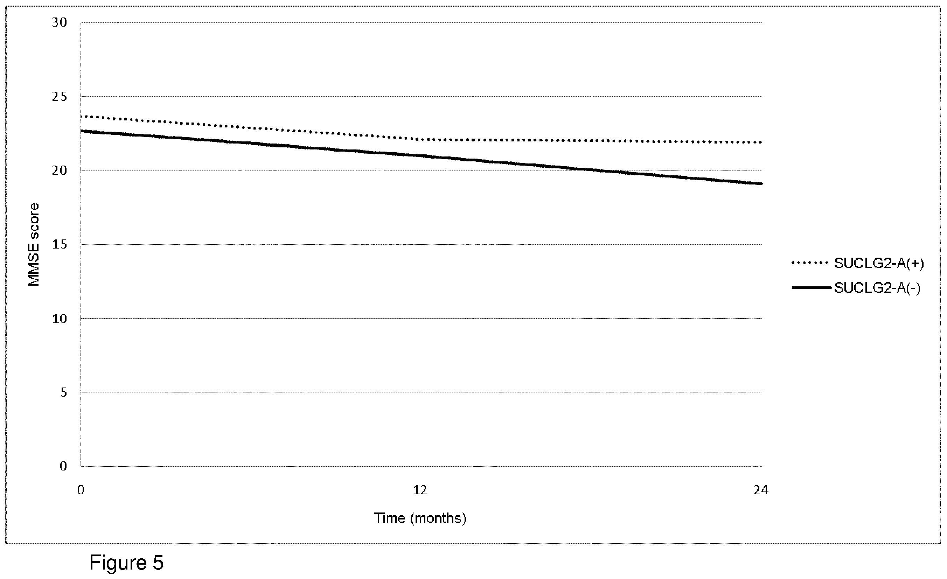

**Figure 5:** rs62256378 allele A shows protective effect on the rate of cognitive decline. Estimated regression lines of the Mini-Mental State Examination (MMSE) score by time. Patients were stratified according to presence or not of the A allele at rs62256378 (SUCLG2-A(+) or SUCLG2-A(-), respectively). The SUCLG2-A(+) group showed slower rate of cognitive decline compared to SUCLG2-A(-).

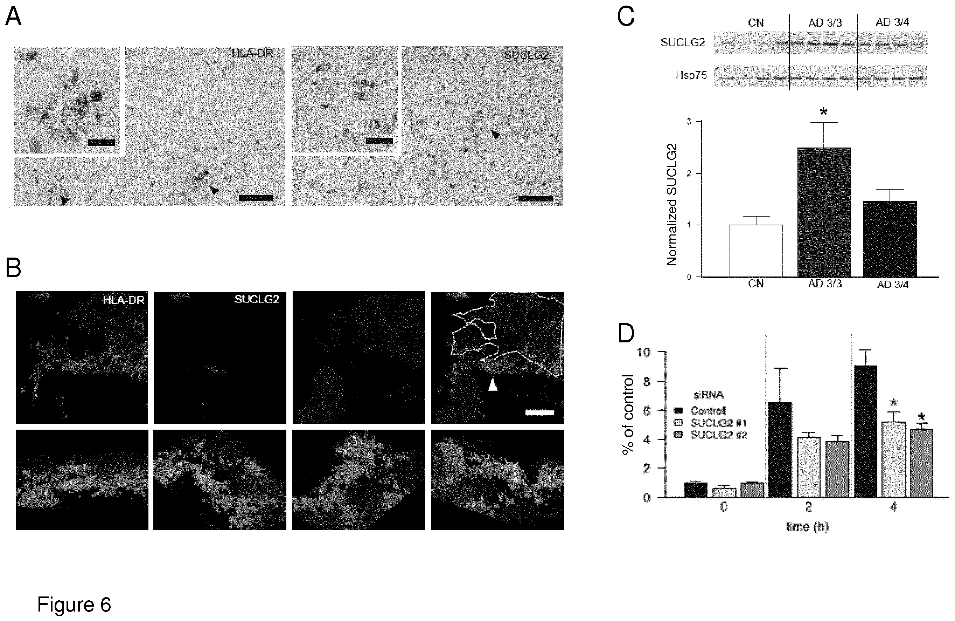

**Figure 6.** Functional studies on SUCLG2.
**A and B. SUCLG2 localized to microglia in Alzheimer's disease (AD) brains. A.** Representative diaminobenzidine (DAB) immunostaining with SUCLG2 and HLA-DR (as a human microglial marker) in brain cortex of AD subjects. DAB-positive cells with characteristic romboidal nuclei (typically microglial cells) are indicated by arrows. Scale bars are 100$\mu$m and 20 $\mu$m on the main and small images, respectively. **B.** Hi-resolution confocal image of an area of frontal cortex from an AD patient immune-labelled with HLA-DR (green) and SUCLG2 (red). Plaque autofluorescence is visible in the blue channel and it is highlighted with a discontinuous line. Most of the SUCLG2 is enclosed by the HLA-DR (arrow). The top panel shows several viewpoints of a 3D reconstruction of the same area. (Scale is 10 microns). **C. Increased levels of SUCLG2 in AD ApoE 3/3 brains.** Soluble extracts from the brain cortex of AD patients with ApoE 3/4 and ApoE 3/3 status plus age-matched healthy controls (CN) were probed with an antibody to SUCLG2. Expression levels were normalized to Hsp75. (CN, n=6; AD, 3/4, n=8; AD 3/3 n=4). D. **SUCLG2 silencing is detrimental to extracellular A$\beta_{1-42}$.** FAM-A$\beta_{1-42}$ phagocytosis was impaired in BV2 cells in which SUCLG2 was silenced by two distinct siRNA compared to negative control siRNA. FAM-A$\beta_{1-42}$ was added at a 500nM final concentration and the amount of internalized A$\beta_{1-42}$ was assessed at the indicated time points (n=6 respectively), showing that cells contain less A$\beta_{1-42}$ in the absence of SUCLG2.

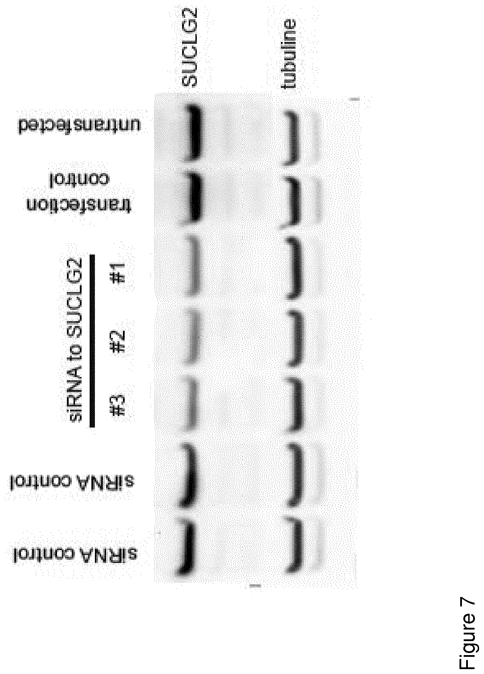

**Figure 7:** Effect upon SUCLG2 protein expression of different siRNA against the SUCLG2 mRNA.

Soluble extracts from BV2 cells transfected with two control siRNA, three siRNA directed to distinct areas of the SUCLG2 gene, or the transfection controls were probed with an antibody to SUCLG2 to demonstrate siRNA-mediated blockage of SUCLG2 expression. Beta-tubulin indicates equivalent protein loading.

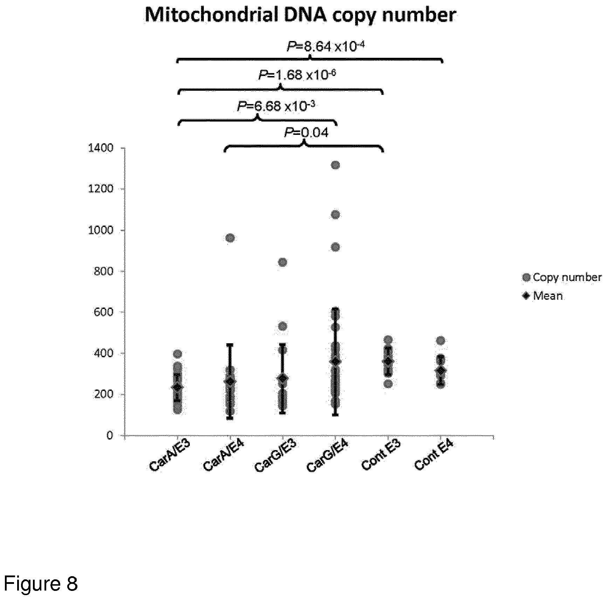

**Figure 8:** Correlation between the SUCLGL2 allele, the ApoE E3/4 allele as well as the mtDNA copy number; CarA/E3 is carrier of Allele A and ApoE E4 negative; CarG/E4 is carrier of Allele G and ApoE E4 positive, CarG/E3 is carrier of Allele G and ApoE E4 negative; CarA/E4 is carrier of Allele A and ApoE E4 positive, Cont E3 is healthy controls ApoE E4 negative, Cont E4 is healthy controls ApoE E4 positive; Each red dot represents one single person; Black diamonds represent mean for each group with its corresponding standard deviation; Each person was analyzed in triplicates; Each run was controlled using a positive fibroblast sample; T-test was used to compare groups

## Description

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

To practice the present invention, unless otherwise indicated, conventional methods of chemistry, biochemistry, cell biology, and recombinant DNA techniques are employed which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. As used in this specification and the appended claims, the singular forms "a", "an", and "the" include plural referents, unless the content clearly dictates otherwise.

The present invention relates to a method for establishing a clinical prognosis of a patient suffering from a disease or condition associated with the formation of aggregates of $A\beta_{1-42}$ comprising the steps of

a) detecting whether an adenine or another nucleotide, preferably guanine, is present at single nucleotide polymorphism (SNP) rs62256378 and/or whether a protective allele or another allele is present at at least one SNP in linkage disequilibrium with rs62256378;

b) establishing a clinical prognosis based on the type of nucleotide present at the site of the SNP, wherein

(i) the presence of an adenine at rs62256378r and/or the presence of a protective allele at at least one SNP in linkage disequilibrium with rs62256378 is indicative of a good prognosis or

(ii) the presence of another nucleotide than adenine at rs62256378 and/or the presence of a another allele at at least one SNP in linkage disequilibrium with rs62256378 is indicative of a bad prognosis, wherein the at least one SNP in linkage disequilibrium with rs62256378 is selected from the group consisting of rs74727963, rs78609144, rs79963093, rs80028595, rs77072359, rs74680427, rs111796700, rs115298081, rs72626930, rs76856038, rs77836520, rs116341583, rs114506918 andrs745742418, and wherein the SNP in linkage disequilibrium with rs62256378 displays an r2 of at least 0.5.

The method of the present invention is an *in vitro* method which is practiced on an isolated sample of the patient.

The term "$A\beta_{1-42}$" refers to a group of peptide fragments which are generated by proteolytic cleavage of amyloid precursor protein (APP). This group includes $A\beta_{1-42}$ as defined by SEQ ID NO: 16, $A\beta_{1-43}$ as defined by SEQ ID NO: 17 and $A\beta_{x-42}$. The term "$A\beta_{x-42}$" refers to fragments starting at positions other than 1 from $A\beta_{1-42}$, i.e. it is characterized by deletion of at least 1, at least 2 at least 3 or at least 4 contiguous N-terminal amino acid residues of SEQ ID NO: 17

**Patient**

The patient is, preferably, a primate. More preferably, the patient is a human.

Since the method of the invention is used for establishing a prognosis of Alzheimer's disease in a patient, it is preferred that the patient is suspected to suffer or known to suffer from Alzheimer's disease. Most preferably, the patient is known to suffer from Alzheimer's disease.

Preferably, the patient is heterozygous for isoform 4 of apolipoprotein E. More preferably, the patient does not carry a gene for isoform of apolipoprotein E. Apolipoprotein E is an important part of the chylomicrons of the vertebrates. It is involved in the transport of lipoproteins, fat-soluble vitamins and cholesterol. Three important isoforms are known: 2, 3 and 4. Individuals carrying one or two copies of isoform 4 are at an increased risk of developing Alzheimer's disease.

**Single Nucleotide Polymorphism**

Single nucleotide polymorphisms (SNPs) are numerically the most important variations in the human genome. In a SNP different versions of the same gene in the same species differ due to exchanges of a single base at a given position. SNPs may be found in coding as well as non-coding regions of the genome. The effects of SNPs differ depending on their position and the type of base exchange. SNPs in non-coding regions, for example, may influence gene expression by enhancing or attenuating the binding of regulatory genes. Such SNPs may also influence mRNA, thus generating different splice variants depending on the base present. SNPs in coding regions may be synonymous, i.e. not changing the amino acid sequence of the encoded peptide, or non-synonymous, i.e. causing incorporation of a different amino acid. SNPs may also introduce a missense into a coding region, thus leading to the synthesis of a shortened peptide.

SNPs may be used as genetic markers for various traits such as increased risk of certain diseases or difference in drug metabolism.

Depending on the frequency of SNPs in a given populations, one allele may be designated the "major" (i.e. more frequent) allele and the other allele or alleles may be designated as "minor" (i.e. less frequent) alleles.

The term "protective allele" is used in the context of the present invention to mean a SNP that is associated with a good clinical prognosis, e.g. slower disease progression, of a patient suffering from a disease or condition associated with the formation of aggregates of $A\beta_{1-42}$.

Any other allele, particularly the major allele, than the "protective allele" is typically associated with a worse clinical prognosis, e.g. a faster disease progression than found in the average of patients carrying at least one protective allele.

**Detection of a SNP**

Methods for discriminating between single nucleotide polymorphisms are well known in the art. In principle, all methods for analysing nucleotide sequences may be used. Particularly preferred are sequencing, southern blotting, quantitative real time PCR and single nucleotide extension.

The SNP may be detected in any sample obtained from the patient provided that the sample contains a sufficient amount of the patient's genomic DNA for detecting the SNP. Preferred samples are blood or blood cells and oral mucosa.

**rs62256378**

SNP rs62256378 is located on chromosome 3p14.1. The nucleotide sequence surrounding the SNP is defined by SEQ ID NO: 1. Base position 22 may be an adenine (minor allele) or a guanine (major allele).

As will be set forth below, the presence of an adenine at nucleotide position 22 of rs62256378 as defined by SEQ ID NO: 1 is associated with slower disease progression of a disease or condition associated with the formation of aggregates of $A\beta_{1-42}$ than the presence of any other nucleotide, particularly a guanine. In patients suffering from Alzheimer's disease a lower rate of cognitive decline was found if at least one copy of the minor allele was present. Thus, this allele is a protective allele indicating a slower progression of a disease or condition associated with the formation of aggregates of $A\beta_{1-42}$, in particular AD while any other base, and particularly guanine, at this position is associated with the average speed of disease progression.

**Linkage Disequilibrium**

It is well understood by the person skilled in the art that genetic recombination of loci in close proximity on the same chromosome is restricted because the incidence of crossing over during meiosis is decreased. Consequently, the frequency of recombination is lower than for two loci on different chromosomes or different ends of the same chromosome. If two loci A and B are independent, the frequency of the combination of alleles $A_1$ and $B_1$ is calculated by multiplying their frequencies within the population in question. Any deviation from this expected value indicates that both loci are in linkage disequilibrium. If the observed frequency $F(A_1, B_1)$ of a combination of alleles $A_1$ and $B_1$ is smaller than the expected frequency $E(A_1, B_1)$ it can be concluded that those alleles are found on different chromosomes or in such a great distance on the same chromosome that crossing over leads to a random distribution during meiosis. Conversely, if the frequency of the combination is higher than expected, this indicates that both alleles are located on the same chromosome in relatively close proximity.

A measure for linkage disequilibrium is $r^2$. A value of 0 indicates complete independence of two loci while a value of 1 indicates no incidence of segregation during meiosis.

The $r^2$ measure of linkage disequilibrium is defined as

$$r2(pa, pb, pab) = \frac{(pab - papb)^2}{pa(1 - pa)pb(1 - pb)},$$

where $p_{ab}$ is the frequency of haplotypes having allele a at locus 1 and allele b at locus 2. As the square of a correlation coefficient, $r^2(p_a, p_b, p_{ab})$ can range from 0 to 1 as $p_a$, $p_b$ and $p_{ab}$ vary. More details regarding the mathematical properties of $r^2$ can be found in VanLiere and Rosenberg, 2008, Theoretical Population Biology, 74: 130-137.

Preferably, the term "SNP in linkage disequilibrium with rs62256378" refers to a SNP which has a $r^2$ larger than 0.5, more preferably 0.6, even more preferably 0.7, even more preferably 0.8 and most preferably 0.9.

It follows from the method for calculating $r^2$ that for all SNPs with a high degree of linkage disequilibrium with rs62256378 the minor allele is the protective allele which is associated with a better clinical prognosis than the major allele or any other allele.

Moreover, based on the association of higher concentrations of $A\beta_{1-42}$ in the cerebrospinal fluid with the presence of at least one copy of the minor allele of SNP rs62256378 and a slower progression of Alzheimer's disease the theoretical prediction of the protective allele can easily be confirmed. This is, preferably, done by measuring the concentration of a statistically relevant number of AD patients carrying at least one copy of the minor allele, measuring said concentration in a statistically relevant number of AD patients carrying no copy of the minor allele and comparing the average concentrations found in both groups. If the former group has a higher average concentration of $A\beta_{1-42}$ in the cerebrospinal fluid, the minor allele is confirmed as the protective allele.

In the method of the invention the "at least one SNP in linkage disequilibrium with rs62256378 is selected from the group consisting of rs74727963, rs78609144, rs79963093, rs80028595, rs77072359, rs74680427, rs111796700, rs115298081, rs72626930, rs76856038, rs77836520, rs116341583, rs114506918 and rs74574241. Characteristics of these SNPs are given in Table 1 below.

Table 1: Overview over SNPs in linkage disequilibrium with rs62256378

| SNP | Proxy | Distance [nucleotides] | R2 | Allele | Nucleotide | Coordinate_HG18[a] | SEQ ID NO: |
|---|---|---|---|---|---|---|---|
| rs62256378 | rs62256378 | 0 | 1 | Minor | A | 67539723 | 1 |
| | | | | Major | G | | 2 |
| rs62256378 | rs74727963 | 487 | 0,818 | Minor | T | 67540210 | 3 |
| | | | | Major | C | | 4 |
| rs62256378 | rs78609144 | 2,043 | 0,818 | Minor | T | 67541766 | 5 |
| | | | | Major | C | | 6 |
| rs62256378 | rs79963093 | 3,733 | 0,818 | Minor | A | 67543456 | 7 |
| | | | | Major | G | | 8 |
| rs62256378 | rs80028595 | 6,182 | 0,818 | Minor | C | 67545905 | 9 |
| | | | | Major | T | | 10 |
| rs62256378 | rs77072359 | 9,357 | 0,818 | Minor | T | 67549080 | 11 |
| | | | | Major | C | | 12 |
| rs62256378 | rs74680427 | 3,5267 | 0,818 | Minor | T | 67574990 | 13 |
| | | | | Major | C | | 14 |
| rs62256378 | rs111796700 | 4,0016 | 0,73 | Minor | A | 67579739 | 15 |
| | | | | Major | G | | 16 |
| rs62256378 | rs115298081 | 4,5727 | 0,73 | Minor | T | 67585450 | 17 |
| | | | | Major | A | | 18 |

(continued)

| SNP | Proxy | Distance [nucleotides] | R2 | Allele | Nucleotide | Coordinate_HG18[a] | SEQ ID NO: |
|---|---|---|---|---|---|---|---|
| rs62256378 | rs72626930 | 1,360 | 0,596 | Minor | C | 67541083 | 19 |
| | | | | Major | T | | 20 |
| rs62256378 | rs76856038 | 4,293 | 0,558 | Minor | C | 67544016 | 21 |
| | | | | Major | T | | 22 |
| rs62256378 | rs77836520 | 10,443 | 0,558 | Minor | T | 67550166 | 23 |
| | | | | Major | G | | 24 |
| rs62256378 | rs116341583 | 61,147 | 0,558 | Minor | T | 67600870 | 25 |
| | | | | Major | C | | 26 |
| rs62256378 | rs114506918 | 134,799 | 0,554 | Minor | T | 67674522 | 27 |
| | | | | Major | C | | 28 |
| rs62256378 | rs74574241 | 176,969 | 0,509 | Minor | C | 67716692 | 29 |
| | | | | Major | T | | 30 |

[a]Position of the SNP in release 18 of the human genome

It can be seen from the table that SNPs in a distance of up to 176,969 bases display significant linkage disequilibrium with rs62256378. Therefore, a "SNP in linkage disequilibrium with rs62256378" is in an also preferred definition of the term not more than 176,000 nucleotides, more preferably not more than 46,000 nucleotides and most preferably not more than 10,000 bases distant from rs62256378.

**Diseases or conditions associated with the formation of aggregates of Aβ$_{1-42}$**

Diseases or conditions associated with the formation of aggregates of Aβ$_{1-42}$ are all diseases or conditions which are characterized by intra- or extracellular formation of insoluble complexes of Aβ$_{1-42}$ intra- or extracellularly.

At least in some of the diseases and conditions it is not entirely clear whether the formation of insoluble complexes is a cause or a consequence of the disease. Therefore, it is not required that the formation of aggregates of Aβ$_{1-42}$ is causal for the disease or condition in question.

The disease or condition associated with the formation of aggregates of Aβ$_{1-42}$ is preferably selected from the group consisting of Alzheimer's disease, sporadic inclusion body myositis and Lewy body dementia.

Alzheimer's disease (AD) is progressive neurodegenerative disease. It is the most common form of dementia. Early stages of AD are characterized by memory loss, especially the inability to remember recent events. Disease progression affects linguistic abilities, particularly to a shrinking vocabulary and decreased fluency. Further progression of the disease is marked by increasing memory losses which also affect the long-term memory, decreasing coordination of complex motor sequences and further exacerbation of linguistic difficulties. Neuropsychiatric problems such as labile affect, irritability and wandering are common. In the final stage of the disease, the patient depends on external care even for the simplest tasks. Linguistic impairment progresses to a point, where language may reduced to single words or even be lost completely. Patients in the final stages of AD are typically exhausted and apathic.

Anatomically, AD is characterized by loss of neurons and synapses in the cerebral cortex resulting in atrophy of the affected regions. Histologic examination of brain tissue of AD patients reveals the presence of extracellular amyloid plaques and intracellular neurofibrillar tangles. The former mainly consist of beta-amyloid peptide, the latter mainly consist of the microtubule-associated protein tau which is hyperphosphorylated.

The pathophysiology of AD has still not been completely elucidated. Currently, the so called "amyloid hypothesis" enjoys the widest support. According to this hypothesis, fragments of the amyloid precursor protein which are generated β- and γ-secretases form aggregates which are believed to be neurotoxic and to mediate the loss of neurons observed in brains of AD patients. The most relevant fragments of APP appear to be Aβ$_{1-42}$, Aβ$_{1-43}$ and Aβ$_{x-42}$ as these peptides are most frequently found in amyloid plaques.

Sporadic inclusion body myositis is an inflammatory muscle disease which is characterized by a slowly progressing weakness of proximal and distal muscles. Histologically, inclusion bodies inside the muscle fibres and infiltration of the muscle by cytotoxic T-cells. The inclusion bodies contain protein aggregations which comprise Aβ$_{1-42}$. In a mouse model of the disease it could be shown that antibodies against Aβ attenuate decrease the intracellular amount of insoluble Aβ and attenuate the disease (Kitazawa et al., 2009, The Journal of Neuroscience, 29: 6131-611).

Lewy body dementia dementia characterized by the presence of Lewy bodies in neural cells. These bodies mainly comprise synuclein, frequently associated with neurofilament protein, ubiquitin and alpha B crystallin. If these inclusions occur in the Cortex, they are often associated with extracellular aggregations of $A\beta_{1-42}$ in the Hippocampus.

## Clinical Prognosis

The term "clinical prognosis" refers to the prediction of the future development of the disease or condition the patient suffers from. Preferably, the increase or onset of clinically observable signs and symptoms is predicted. In case of sporadic inclusion body myositis the symptoms are, preferably, associated with weakness of the affected muscles. In case of Alzheimer's disease and Lewy body dementia the symptoms are, preferably, related to cognitive impairment.

Preferably, a "good clinical prognosis" as indicated by the detection of at least one protective allele of at least one of the SNPs recited above is the prognosis of disease progression which is slower than disease progression in a statistically relevant number of patients not carrying the protective allele. Preferably, disease progression is defined by the onset and/or exacerbation of the symptoms recited in the paragraph above.

Thus, a "good" as well as a "bad" prognosis are relative terms, derived from sufficiently large collectives of patients suffering from a disease or condition associated with the formation of aggregates of $A\beta_{1-42}$ and either carrying at least one protective allele of a SNP of the present invention or not carrying it. Given the fact, that the protective allele is the minor allele, the bad prognosis can be viewed as the most frequent or typical prognosis of the disease in question and the good prognosis can be viewed as the exception from this rule.

Methods for determining cognitive impairment are well known to the neurologist. Preferred methods include mini-mental state examination (MMSE), blessed dementia rating scale (BDRS) and the clinical dementia rating sum of boxes

Most preferably, the method of the present invention predicts the decline of cognitive abilities as measured by the MMSE. This is a brief questionnaire used for screening cognitive impairment (Folstein MF, Folstein SE, McHugh PR, 1975, Journal of Psychiatric Research 12 (3): 189-98). It covers the areas of memory, orientation and arithmetic. Up to 30 points can be awarded, wherein at least 27 points indicate normal cognition, 19 to 24 points indicate mild cognitive impairment, scores between 10 and 18 points indicate moderate cognitive impairment and scores below 10 points indicate severe cognitive impairment.

In case of AD, the presence of the protective allele is associated with a cognitive decline of no more than 2 or 3 points as measured with the MMSE in 24 months, while the absence of the protective allele is associated with a decline of more than 3, preferably more than 4 and, most preferably, more than 5 points in 24 months.

As will be understood by those skilled in the art, such a prognosis is usually not intended to be correct for 100% of the subjects to be analysed. The term, however, requires that the prognosis is correct for a statistically significant portion of the subjects (e.g. a cohort in a cohort study). Thus, the method of the present invention at least provides an aid for establishing a clinical prognosis. Whether a portion is statistically significant, can be determined without further ado by the person skilled in the art using various well known statistic evaluation tools, e.g., determination of confidence intervals, p-value determination, Student' s t-test, Mann-Whitney test etc.. Details are found in Dowdy and Wearden, Statistics for Research, John Wiley & Sons, New York 1983. Useful confidence intervals are at least 90%, at least 95%, at least 97%, at least 98% or at least 99 %. The p-values are, typically, 0.1, 0.05, 0.01, 0.005, or 0.0001.

## Treatment

Since the prognosis established according the method of the present invention has obvious consequences for the treatment of a patient suffering from a disease associated with the formation of aggregates of $A\beta_{1-42}$, the present invention also relates to a suitable medicament for use in the treatment of a disease or condition associated with the formation of aggregates of $A\beta_{1-42}$ in patients having another nucleotide than adenine at rs62256378 and/or another allele at at least one SNP in linkage disequilibrium with rs62256378 as determined by the method of any one of claims 1 or 2, wherein the medicament is selected from the group consisting of donepezil, galantamine, memantine, rivastigmine, tacrine, vitamin E, citalopram, fluoxetine, paroxeine, sertraline, trazodone, lorazepam, oxazepam, aripiprazole, clozapine, haloperidol, olanzapine, quetiapine, risperidone, and ziprasidone.

If the clinical prognosis is the bad prognosis, pharmaceutical as well as non-pharmaceutical preventive measures should be taken in order to slow down the loss cognitive function, the loss of the ability to perform daily life activities and the onset of neuropsychiatric complications.

All definitions given above also apply to this embodiment of the invention.

Suitable medicaments belonging to the above-mentioned groups which are suitable for the treatment of AD are well known to the person skilled in the art.

It is understand by the person skilled in the art, that a physician will take further variables, such as pre-existing diseases of the patient, into account when establishing a prognosis. Thus, the method of the present invention may be integrated into and assist a more complex process of clinical decision making.

Advantageously, the method of the present invention allows the early determination of the future course of a disease associated with the formation of aggregates of $A\beta_{1-42}$, particularly AD. Patients with a predicted slower progression of the disease may spared aggressive treatment, thus decreasing the risk of side effects which is often associated with aggressive treatment. Moreover, a reliable prediction of the speed of disease progression allows more informed decisions of the patient and/or his relatives about the general management of the disease. This may affect, e.g., the decision about home care or institutional care.

Kit

In another embodiment, the present invention relates to the use of a kit for establishing a clinical prognosis of a patient suffering from a disease or condition associated with the formation of aggregates of $A\beta_{1-42}$, which comprises at least one means for detecting the presence of adenine or guanine at SNP rs62256378, comprising an oligonucleotide comprising at least 8 bases of SEQ ID NO: 1 or a complement thereof, wherein said at least 8 bases comprise position 27.

The term "kit" refers to a composition of parts which are useful for determining the presence of a low risk allele at any of the SNPs defined further above in this application.

In one preferred embodiment, said oligonucleotide comprises at least 10, at least 12, at least 14, at least 16, at least 18 or at least 20 bases of the genomic region surrounding the position of the SNP in question and includes the position of the SNP. Such oligonucleotides are useful as specific probes for hybridization-based detection of the SNP or as specific primers in a PCR reaction which either generates or does not generate a product depending on the nucleotide present at the position of the SNP.

In another preferred embodiment, the oligonucleotide comprises at least 10, at least 12, at least 14, at least 16, at least 18 or at least 20 bases and is located with its 3'-end immediately before the position of the SNP. Such nucleotides are particularly useful for detecting SNPs by single base extension and single nucleotide primer extension.

Said oligonucleotides may be labelled with any detectable label known in the art.

In one preferred embodiment, the kit comprises in addition to the means for detecting the presence of adenine or guanine at SNP rs62256378 or for detecting whether a protective allele is present at at least one SNP in linkage disequilibrium with rs62256378 at least one means selected from the group consisting of a container for collecting and/or storing a sample comprising the patient's genomic DNA, means for extracting genomic DNA from the sample and a DNA polymerase.

The present disclosure also relates to an oligonucleotide as described above as part of the kit for use in establishing a prognosis of a patient suffering from a disease or condition associated with the formation of aggregates of $A\beta_{1-42}$.

In yet another embodiment, the present dislcosure relates to the use of an oligonucleotide as described for establishing a clinical prognosis of a patient suffering from a disease or condition associated with the formation of aggregates of $A\beta_{1-42}$.

In yet another embodiment, the present disclosure relates to an oligonucleotide as described for use in establishing a clinical prognosis of a patient suffering from a disease or condition associated with the formation of aggregates of $A\beta_{1-42}$.

In another embodiment, the present invention relates to a method for screening compounds to identify therapeutic candidates for the modulation of a disease or condition associated with the formation of aggregates of $A\beta_{1-42}$ comprising the steps of

a) contacting (i) a brain, blood, or liver cell which is capable of expressing the GTP-specific beta subunit of the succinyl-CoA ligase (SUCLG2) in vitro or (ii) a cell which expresses SUCLG2 in vitro or (iii) a solution comprising SUCLG2 with a test compound or (iv) administering the test compound to an experimental animal, wherein the animal is a non-human animal;

b) detecting in case of (i) whether said test compound increases the expression level or the activity of SUCLG2 in vitro, in case of (ii) whether said test compound increases the expression level or the activity of SUCLG2 in vitro, in case of (iii) whether said test compound increases the activity of SUCLG2, in case (iv) whether said test compound increases the expression level or activity of SUCLG2, wherein an increase of the expression level of activity indicates that said test compound is a candidate compound for treating the disease or condition in vivo.

It is preferred that in embodiments wherein the cell capable of expressing SUCLG2, does not express SUCLG2 before being contacted with said test compound, the expression of SUCLG2 is initiated, i.e. increased, upon being contacted with the test compound. It is thus, also preferred that in embodiments wherein the cell capable of expressing SUCLG2, does not express SUCLG2 before being contacted with said test compound, the activity of SUCLG2 is initiated, i.e. increased, upon being contacted with the test compound.

All definitions of "disease or condition associated with the formation of aggregates of $A\beta_{1-42}$ given above also apply to this embodiment of the invention.

Preferably, the GTP-specific beta subunit of the succinyl-CoA ligase expressed by the cell or comprised by has an amino acid sequence as defined by SEQ ID NOs: 33 (isoform 1) or 34 (isoform 2).

The term "contacting" refers to the addition of the test compound to the growth medium of the cell or the buffer in which SUCLG2 is dissolved so that the screening compound can interact with the cell or the SUCLG2 and exert its effect thereon.

If a cell is used in the screening method of the present invention, it is, preferably, a cell which expresses or is capable of expressing SUCLG2 without genetic modification of the cell.

The cell is, preferably, a liver cell or a brain cell.

Preferably, the cell is a brain cell. The term "brain cell" refers to any type of cell which is found in the brain, particularly to neurons and glial cells. In the present application, the term "neuron" refers to any cell which can be electrically excited and transmits information through electrochemical signals.

More preferably, the cell is a glial cell. This term refers to all non-neuronal cells found in the brain with the exception of blood cells. Preferably, glial cells are selected from the group consisting of microglia, astrocytes, oligodendrocytes and ependymal cells. Most preferably the glial cell is a microglial cell.

Microglia are specialised macrophages found in the brain and the spinal cord. They are the main constituents of the immune response in the central nervous system. Thy remove dead cells and foreign materials. They act as antigen presenting cells which activate T-cells and they can destroy pathogens by releasing cytotoxic substances, most prominently $H_2O_2$ and NO. Importantly, they also produce amyloid precursor protein.

Astrocytes anchor neurons to their blood supply, take up and recycle secreted neurotransmitters and regulate the external chemical environment of neurons.

Oligodendrocytes coat axons and produce the myelin sheaf which isolates the axons.

Ependymal cells line the walls of the cavities of the central nervous system, secrete cerebrospinal fluid, help by beating with their cilia to circulate it and make up a part of the blood-brain-barrier.

In a preferred embodiment of the screening method the cell is heterozygous for isoform 4 of apolipoprotein E. More preferably, it does not carry a copy of isoform 4 of apolipoprotein 4.

In another preferred embodiment of the screening method, (i) the cell is also capable of expressing the alpha subunit of SUCLG2 (SUCLG1) or expresses SUCLG1 or (ii) the solution further comprises SUCLG1. Preferably, the amino acid sequence of SUCLG1 is defined by SEQ ID NO: 35

**Expression of SUCLG2**

The expression of SUCLG2 refers to the transcriptional activity of the gene and is, preferably, determined based on the amount of mRNA or protein produced by the cell. It is also preferred to use the activity of said peptide or the succinate CoA-ligase holoenzyme.

Particular methods for determining the amount of an mRNA are hybridization-based or amplification-based. Such methods are well known in the art. The required devices and reagents are commercially available.

Hybrididization-based methods employ nucleic acid molecules which are completely or partially complementary to the mRNA to be detected. Said nucleic acid molecules bind specifically to the complete or a partial sequence of the nucleic acid molecule to be detected. Specific binding of both of the aforementioned nucleic acid molecules is then detected using suitable means and methods. The detection of the target mRNA may be qualitatively or quantitatively. Suitable hybridization-based methods are fluorescent in-situ hybridization (FISH), Northern Blot and DNA microarrays.

Amplification-based methods employ means and methods which amplify the complete or a partial sequence of the mRNA to be detected. Said amplification is specific, i.e. only those sequences are amplified which are identical or highly similar to the target nucleic acid. The presence of the target nucleic acid in a sample is detected qualitatively or quantitatively based on the presence or the amount of amplificates generated. A useful amplification-based method is quantitative real time PCR.

FISH employs short (typically 10 to 40 nucleotides) single-stranded DNA molecules (probes) covalently coupled to a fluorescent dye (label). Cells or tissue comprising the mRNA of interest are fixed and contacted with the probe under conditions which allow hybridization of the probe and the target sequence. Unbound probes are then removed by washing. After washing, the amount of bound probes can be determined by detecting the fluorescence emitted by the bound probes.

Northern blotting involves separation of the mRNA according to its size on a gel and the transfer of the separated DNA to a membrane (typically made of nylon). Once the RNA is linked to the membrane, it is contacted with a probe consisting of single-stranded nucleic acid which is completely or partially complementary to a part of the mRNA sequence. Said probe is covalently linked to an enzyme or comprises radioactive isotopes. The probe is contacted with the membrane carrying the mRNA under conditions suitable for specific binding of the probe to its target sequence. After washing of the membrane in order to remove unbound probes, the label may be detected with suitable methods. Radioactively labelled probes may be detected with light-sensitive paper or a phosphor imager. Suitable enzymatic labels are recited below. Means and methods for their detection are conventional in the art.

Microarrays are solid supports covalently to single-stranded nucleic acid molecules which are completely or partially complementary to the sequence of specific mRNAs. Nucleic acid molecules having different sequences are spatially separated on the array so that molecules of a given sequence form a spot. The mRNA comprised by a sample of interest is converted to cDNA by a reverse transcriptase and covalently coupled to a detectable label. Typically, said label is fluorescent dye. The cDNA is contacted with the array under conditions suitable for specific hybridization of the cDNA to the complementary nucleic acid molecules carried by the solid support. After washing in order to remove unbound cDNA the bound cDNA can be detected by detecting the label.

Quantitative real-time PCR is performed similarly to the conventional polymerase chain reaction, i.e. a target nucleic acid sequence is amplified using two specific primers, a suitable DNA polymerase and the required deoxynucleotides in a thermocycler. Rather than determining the amount of amplificate at the end, the amount of amplificate is determined during the reaction. It may be repeatedly determined at each amplification cycle. There are two principle methods for detecting the amplificate. Intercalating fluorescent dyes can be used to determine the total amount of double-stranded DNA in the reaction vessel. Said dyes must only display fluorescence when bound to double-stranded DNA. A suitable dye is, for example, SYBR Green. For more specific detection of the amplificate single-stranded nucleic acid molecules covalently coupled to a fluorescent dye on one and a suitable quencher at the other one are employed. Due to its spatial proximity to the fluorescent dye the quencher inhibits fluorescence of the dye as long as both molecules are bound to the probe. The probes are completely or partially complementary to the amplified sequence and, therefore, hybridize specifically to the amplificate. Bound probe molecules are degraded by the 5' to 3' exonuclease activity of the DNA polymerase. After this degradation the quencher molecule is too far removed from the fluorescent dye to prevent fluorescence. Thus, the amount of fluorescence observed in the vessel increases with the amount of PCR product generated.

In accordance with the present invention, determining the amount of a peptide or polypeptide can be achieved by all known means for determining the amount of a peptide in a sample. Said means comprise immunoassay devices and methods which may utilize labelled molecules in various sandwich, competition, or other assay formats. The reagents and devices employed in these methods are conventional in the art and commercially available.

Said assays will develop a signal which is indicative for the presence or absence of the peptide or polypeptide. Moreover, in one embodiment the signal strength can be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise biosensors, optical devices coupled to immunoassays, biochips, analytical devices such as mass-spectrometers, NMR-analyzers, or chromatography devices. Further methods include micro-plate ELISA-based methods, fully-automated or robotic immunoassays, CBA (an enzymatic Cobalt Binding Assay), and latex agglutination assays.

In one embodiment, determining the amount of a peptide or polypeptide comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and noncovalent binding. A ligand according to the present invention can be any compound, e.g. a peptide, polypeptide, nucleic acid, or small molecule, binding to the peptide or polypeptide described herein. Suitable ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. In one embodiment, the ligand or agent binds specifically to the peptide or polypeptide. Specific binding according to the present invention means that the ligand or agent should not bind substantially to ("cross-react" with) another peptide, polypeptide or substance present in the sample to be analyzed. In one embodiment, the specifically bound peptide or polypeptide is bound with at least 3 times higher, at least 10 times higher or at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Said method may be semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, the amount of substrate is typically saturating. The substrate may also be labelled with a detectable label prior to the reaction. The sample is contacted with the substrate for an adequate period of time. An adequate period of time refers to the time necessary for a detectable amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, Green Fluorescent Protein (GFP), myc-tag, influenza A virus haemagglutinin (HA), maltose binding protein, and the like. In the case of a peptide or polypeptide, the tag is may be attached at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a coloured reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include 35S, 125I, 32P, 33P and the like. A radioactive label can be detected by any method known and appropriate, e.g. a light-sensitive film or a phosphor imager. Suitable measurement methods according the present invention also include precipitation enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The activity of SUCLG2 which is measured in the screening method of the present invention is, preferably, the reaction between succinyl-CoA, inorganic phosphate and GDP to generate succinate, coenzyme A and GTP. If a screening compound increases said activity, it is a candidate compound as understood by the present application.

Preferably, the above-defined activity of SUCLG increases the stability and/or function of mitochondria.

Preferably, the expression and/or activity of SUCLG2 is compared between a cell or enzyme or experimental animal which is contacted with the test compound and a control experiment which comprises the same cell or enzyme incubated under identical conditions except for the addition of the test compound.

Preferably, the test compound is selected from the group consisting of peptides, small molecules and agonistic antibodies.

Peptides as understood by the present invention are macromolecules comprising at least 2, 3, 5, 10, 25, 50, 75, 100, 150 or 200 amino acids linked by peptide bonds. Preferably, the peptides comprise only proteinogenic amino acids. However, peptides comprising amino acids which are not proteinogenic are also comprised by the term "peptide".

Small molecules as understood by the present application are organic molecules having a molecular weight of about 50 g/mol, about 100 g/mol, about 200 g/mol, about 300 g/mol, about 400 g/mol, about 500/mol, about 600 g/mol, about 700 g/mol, about 800 g/mol, about 900 g/mol or about 1000 g/mol. The term "about" refers to a deviation of +/- 5 % of the values recited above.

An agonistic antibody as understood by the present application is an antibody which increases the activity and/expression of SUCLG2. The term "antibody" antibodies as well as antigen-binding fragments of antibodies as defined below.

The term "antibody", as used herein, is intended to refer to immunoglobulin molecules comprised of four polypeptide chains, two heavy (H) chains and two light (L) chains interconnected by disulfide bonds. The term "antibody" also includes all recombinant forms of antibodies, in particular of the antibodies described herein, e.g. antibodies expressed in prokaryotes, unglycosylated antibodies, and any antigen-binding antibody fragments and derivatives as described below. Each heavy chain is comprised of a heavy chain variable region ("HCVR" or "VH") and a heavy chain constant region (comprised of domains CH1, CH2 and CH3). Each light chain is comprised of a light chain variable region ("LCVR or "VL") and a light chain constant region (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

Substitution of one or more CDR residues or omission of one or more CDRs is also possible. Antibodies have been described in the scientific literature in which one or two CDRs can be dispensed with for binding. Padlan et al. (1995 FASEB J. 9:133-139) analyzed the contact regions between antibodies and their antigens, based on published crystal structures, and concluded that only about one fifth to one third of CDR residues actually contact the antigen. Padlan also found many antibodies in which one or two CDRs had no amino acids in contact with an antigen (see also, Vajdos et al. 2002 J Mol Biol 320:415-428).

CDR residues not contacting antigen can be identified based on previous studies (for example residues H60-H65 in CDRH2 are often not required), from regions of Kabat CDRs lying outside Chothia CDRs, by molecular modeling and/or empirically. If a CDR or residue(s) thereof is omitted, it is usually substituted with an amino acid occupying the corresponding position in another human antibody sequence or a consensus of such sequences. Positions for substitution within CDRs and amino acids to substitute can also be selected empirically. Empirical substitutions can be conservative or non-conservative substitutions.

The term "antigen-binding fragment" of an antibody (or simply "binding portion"), as used herein, refers to one or more fragments of an antibody that retain the ability to specifically bind to its antigen, such as SUCLG2. It has been shown that the antigen-binding function of an antibody can be performed by fragments of a full-length antibody. Examples of binding fragments encompassed within the term "antigen-binding fragment" of an antibody include (i) Fab fragments, monovalent fragments consisting of the VL, VH, CL and CH domains; (ii) F(ab')$_2$ fragments, bivalent fragments comprising two Fab fragments linked by a disulfide bridge at the hinge region; (iii) Fd fragments consisting of the VH and CH domains; (iv) Fv fragments consisting of the VL and VH domains of a single arm of an antibody, (v) dAb fragments (Ward et al., (1989) Nature 341: 544-546), which consist of a VH domain; (vi) isolated complementarity determining regions (CDR), and (vii) combinations of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242: 423-426; and Huston et al. (1988) Proc. Natl. Acad. Sci. USA 85: 5879-5883). Such single chain antibodies are also intended to be encompassed within the term "antigen-binding fragment" of an antibody. A further example is a binding-domain immunoglobulin fusion protein comprising (i) a binding domain polypeptide that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. The binding domain polypeptide can be a heavy chain variable region or a light chain variable region. The binding-domain immunoglobulin fusion proteins are further disclosed in US 2003/0118592 and US 2003/0133939. These antibody fragments are obtained using conventional techniques known to those with skill in the art, and the fragments are screened for utility in the same manner as are intact antibodies. Further examples of "antigen-binding fragments" are so-called microantibodies, which are derived from single CDRs. For example, Heap et al. describe a 17 amino acid residue microantibody derived from the heavy chain CDR3 of an antibody directed against the gp120 envelope glycoprotein of HIV-1 (Heap CJ et al. (2005) J. Gen. Virol. 86:1791-1800). Other examples include small antibody mimetics comprising two or more CDR regions that are fused to each other, preferably by cognate framework regions. Such a small antibody mimetic comprising VH CDR1 and VL CDR3 linked by the cognate VH FR2 has been described by Qiu et al. (Qiu X-Q, et al. (2007) Nature biotechnology 25(8):921-929).

The term "experimental animal" refers to any non-human animal which can be used in research to study the mechanism and course of Alzheimer's disease. The experimental animal is, preferably a mouse. Particularly preferred are mice. Particularly preferred mice which serve as animal models for Alzheimer's disease belong to the strains recited below.

B6;129-Psen 1$^{tm 1 Mpm}$ TG(APPSwe, tauP301L)1Lfa/Mmjax (MMRRC ID 034830-JAX)

B6C3-Tg(APPswe,PSEN1dE9)85Dbo/Mmjax (MMRRC ID 034829-JAX)
B6.Cg-Tg(APP swe,PSEN1dE9)85Dbo/Mmjax (MMRRC ID 034832-JAX)
B6.129-Tg(APPsw)40Btla/Mmjax (MMRRC ID 034831-JAX)
B6.Cg-Nos2$^{tm1Lau}$ Tg(Thy1-APPSwDutlowa)BWevn/Mmjax (MMRRC ID 034849-JAX)
B6.Cg-TgPDGFB-APPwlnd)20Lms/2J/Mmjax (MMRRC ID 034836-JAX)
B6SJL-Tg(APPSwFILon,PSEN1*M146L*L286V)6799Vas/Mmjax (MMRRC ID 034840-JAX)
C57BU6-Tg(Thy1-APPSwDutlowa)BWevn/Mmjax (MMRRC ID 034843-JAX)
B6;C3-Tg(Prnp-MAPT*P301S)PS19VIe (Stock No. 008169)
B6.Cg-Mapf$^{tm1(EGFP)KI}$Tg(MAPT)8cPfav/J (Stock No. 005491)
B6.Cg-Tg(Camk2a-tTA)1Mmay Tg(tetO-ABL1*P242E*P249)CPdav/J (Stock No. 015838)
FVB-Tg(tetO-MAPT*P301L)#Kha/J (Stock No. 015815

These strains are available from the Jackson Laboratory, Bar Harbor, Main, United States of America.

Expression and/or activity of SUCLG2 are determined in brain tissue, blood and liver cells. The brain tissue is preferably taken from the frontal cortex.

The test compound may be administered to the experimental non-human animal via any route which is suitable. Preferred routes of administration are intravenous injection, intramuscular injection, intrathecal injection, peritoneal injection, subcutaneous injection and oral administration.

The test compound may be formulated as pharmaceutically acceptable salt. The formulation may comprise any pharmaceutically acceptable filler.

The following examples are intended to illustrate the invention. They shall not limit the scope of the claims in any way.

## Examples

## Methods

## Online Methods

## Genome-wide association study (GWAS) samples

GWAS data on the cerebrospinal fluid (CSF) biomarkers amyloid-beta 1-42 ($A\beta_{1-42}$) and phosphorylated Tau at position 181 ($pTau_{181}$) from three independent Alzheimer's disease (AD) cohorts were analyzed (Bonn1CSF, n=113; Bonn2CSF, n=167; ADNI, n=83). All AD patients included in the GWAS analysis fulfilled NINCDA/ADRDA criteria for probable AD (16). The demographic data of the GWAS AD patients are shown in Table 2.

The Bonn1CSF and Bonn2CSF GWAS samples were derived from a larger German GWAS cohort (n=1,079) which was recruited from the following three sources: (i) the German Dementia Competence Network (17) (DCN; www.kompetenznetz-demenzen.de, n=391); (ii) the German study on Aging, Cognition, and Dementia in primary care patients (18) (AgeCoDe, n=64); and (iii) the interdisciplinary Memory Clinic at the University Hospital of Bonn (n=624). These German patients were classified as two separate samples (i.e. Bonn1, n=438; and Bonn2, n=641), as they were genotyped using two different platforms (see below). The Bonn1CSF and Bonn2CSF samples were comprised of AD patients from Bonn1 and Bonn2 respectively for whom CSF data were available. The Bonn1CSF included 61 patients from the Memory Clinic and 52 from the DCN. The Bonn2CSF consisted of 84 patients from the Memory Clinic and 83 from the DCN.

Between 2003 and 2005, the DCN recruited a large cohort of patients with MCI (n=1,095 patients) or early AD (n=391). Baseline analysis comprised extensive neuropsychological tests including, the Consortium to Establish a Registry for Alzheimer's Disease (CERAD); the MMSE; and the Clinical Dementia rating (CDR-SB). Follow-up assessments were performed at 12 and 24 months (17). Whole blood, genomic DNA, plasma, and CSF were collected from participants.

AgeCoDe is a German multicenter prospective cohort study. The recruitment source was the registries of general practitioners (GP). From these registries a sample was randomly drawn and contacted by mail. Main inclusion criteria were age of 75 years and older and absence of dementia according to the GP's judgment. A total of 3,327 AgeCoDe participants were recruited between January 2002 and November 2003 in six German cities. Each participant is assessed at baseline and at 18 months intervals thereafter. All assessments are performed at the participant's home by a trained study psychologist or physician. At all visits, assessment includes the Structured Interview for Diagnosis of Dementia of Alzheimer type, Multi-infarct Dementia, and Dementia of other etiology according to DSM-IV and ICD-10 (20). The SIDAM comprises: (1) a 55-item neuropsychological test battery, including all 30 items of the MMSE and assessment of several cognitive domains (orientation, verbal and visual memory, intellectual abilities, verbal abilities/calculation, visual-spatial constructional abilities, aphasia/ apraxia); (2) a 14-item scale for the assessment of the activities of daily living (SIDAM-ADL-Scale); and (3) the Hachinski Rosen-Scale. Dementia was diagnosed according to DSM-IV criteria. The AgeCoDe study was approved by all of the respective ethics committees. Written informed consent was obtained from all study participants or their legal guardians. At the time of writing, four waves of follow-up have been completed in the AgeCoDe cohort.

The interdisciplinary Memory Clinic of the Department of Psychiatry and Department of Neurology at the University Hospital in Bonn provided further patients (n=624). Diagnoses were assigned according the NINCDS/ADRDA criteria (16) and on the basis of clinical history, physical examination, neuropsychological testing (using the CERAD neuropsychological battery, including the MMSE), laboratory assessments, and brain imaging.

The third GWAS sample was a subsample of the Alzheimer's Disease Neuroimaging Initiative (ADNI) cohort. This comprised 83 AD patients of non-Hispanic Caucasian origin for whom CSF biomarker data were available. The ADNI is a $60 million, 5 year public-private partnership which was launched in 2003 by the National Institute on Aging, the National Institute of Biomedical Imaging and Bioengineering, the Food and Drug Administration, and various private pharmaceutical companies and non-profit making organizations. The Principal Investigator of this initiative is Michael W. Weiner, MD. ADNI is the result of the efforts of many coinvestigators from a broad range of academic institutions and private corporations, and subjects have been recruited from over 50 sites across the USA and Canada. The initial goal of ADNI was to recruit 800 adults aged 55 to 90 years, i.e. 200 cognitively normal individuals for 3 year follow-up, 400 individuals with MCI for 3 year follow-up, and 200 individuals with early AD for 2 year follow-up. Up-to-date information on the cohort is available at www.adni-info.org. All AD patients fulfilled NINCDS/ADRDA criteria (16), and their magnetic resonance imaging (MRI) data were consistent with this diagnosis. ADNI participants undergo evaluation at 6-month to 12-month intervals through a battery of assessments including cognitive and neuropsychological testing, neuroimaging (MRI with or without positron emission tomography), and measurement of plasma and CSF biomarkers. Among other instruments, cognitive status is assessed using the MMSE score. The ADNI study is approved by the Institutional Review Board at each of the participating centers, and all participants provide written informed consent. Data used in the preparation of the present study were obtained from the ADNI database (www.loni.ucla.edu/ADNI).

**Replication sample**

These patients were drawn from the ongoing memory-clinic-based Amsterdam Dementia Cohort (ADC) of the VU University Medical Center. All patients underwent a standardized one-day assessment, comprising medical history, informant based history, physical and neurological examination, laboratory tests, neuropsychological assessment, and brain MRI. Probable AD diagnoses were assigned according to both NINCDS/ADRDA criteria (16), and the core clinical NIA-AA criteria (21). Follow-up took place during routine clinical attendance at the memory clinic, and involved patient history, cognitive testing including the MMSE, and a general physical and neurological examination. Patients who failed to attend routine follow-up for at least 2 years after baseline were contacted by telephone, and their cognitive status was evaluated using a standardized interview that included questions concerning all cognitive domains, physical complaints, and medical history, complemented by the Telephone Interview for Cognitive Status (TICS) (22). In cases where the telephone interview suggested clinical progression, patients underwent a detailed evaluation at the outpatient clinic.

**Investigation of rs62256378 in AD risk**

A case-control sample was used to investigate whether rs62256378 confers AD risk. The AD patients were derived from the Bonn1 and Bonn2 samples (n= 1, 079). The control sample was mainly comprised of individuals from three population-based studies: the Heinz Nixdorf Recall (HNR) study cohort (n=1,188) (23); the POPGEN biobank (n=471) (24); and the KORA study (n=458) (25). These controls were not screened for cognitive impairment. Furthermore, 287 healthy elderly individuals from the AgeCoDe cohort, in whom dementia and MCI had been excluded at the last follow-up visit, were also included. The demographic data for these samples are provided in Table 3.

**Investigation of rs62256378 in cognitive decline**

The effect of rs62256378 on cognitive decline in AD patients was explored using longitudinal MMSE scores in AD patients from: the Bonn1 (n=80); Bonn2 (n=110); ADNI (n=140); and the ADC (n=339). The effect of APOE-$\varepsilon$4 carrier/noncarrier status on the rs62256378 effect on MMSE progression was also analyzed.

**CSF collection and analysis**

For the Bonn1CSF and Bonn2CSF samples, diagnostic lumbar punctures were performed at the respective Department of Neurology or Psychiatry using a standardized technique. The subject was placed in a sitting or supine position, and a 22G atraumatic spinal needle was inserted. The CSF samples were kept on ice for a maximum of 1 hour (h) and then centrifuged for 10 minutes (min) (2000g at 4°C). Samples were aliquoted to 0.25 ml and stored in polypropylene tubes at -80°C until analysis. All CSF samples were sent to the Department of Psychiatry in Erlangen for quantification. Levels of $A\beta_{1-42}$ and $pTau_{181}$ were measured using commercially available ELISA immunoassays INNOTEST® β-amyloid$_{(1-42)}$ and INNOTEST® PhosphoTAU$_{(181p)}$ (Innogenetics), in accordance with the protocols described by Popp et al. (26).

In the ADNI cohort, lumbar puncture was performed using a 20G or 24G spinal needle, as described in the ADNI procedures manual (http://www.adni-info.org/). In brief, CSF was collected into collection tubes provided to each site. The CSF samples were then transferred into polypropylene transfer tubes and placed on dry ice within 1 h of collection. They were then shipped overnight to the ADNI Biomarker Core laboratory at the University of Pennsylvania Medical Center on dry ice. After thawing for 1h at room temperature and gentle mixing, aliquots (0.5ml) of these samples were prepared. The aliquots were stored in bar code-labelled polypropylene vials at -80°C. CSF levels of $A\beta_{1-42}$ and $pTau_{181}$ were measured using the multiplex xMAP Luminex technology (Luminex Corp) and INNO-BIA AlzBio3 immunoassays (Innogenetics) according to protocols described elsewhere[1].

In the ADC cohort, lumbar puncture was performed using a 25G needle. The CSF was collected in polypropylene tubes and centrifuged at 1800 x g for 10 min at 4°C. The CSF was immediately stored at -20°C until further analysis (maximum two months). CSF levels of $A\beta_{1-42}$ and $pTau_{181}$ were measured using similar commercial ELISA immunoassays as those used for Bonn1CSF and Bonn2CSF. Quantification was performed in the neurological laboratory of the VU University Medical Center in Amsterdam in accordance with the protocols described by Mulder et al. (27).

Standardization of CSF biomarker levels was necessary since values in the ADNI cohort differed substantially from those observed in the Bonn1CSF, Bonn2CSF, and ADC cohorts, due to the different platforms used (regular ELISA versus Luminex). Individual CSF levels were standardized within each study using a standard normal log transformation.

### Genotyping

The Bonn1CSF sample was genotyped on the Illumina 610-quad chip, and was part of the sample investigated by Harold et al. (28). The Bonn2CSF sample was genotyped using the Illumina Omni 1M-quad chip. The ADNI cohort was genotyped using the Illumina 610 chip. The control sample of Bonn1 was genotyped using the Illumina HumanHap550k chip. The control sample of Bonn2 was genotyped with the Illumina 1M-quad chip.

Genotyping in the ADC sample was performed using the MassArray system and a Sequenom Compact MALDI-TOF device (Sequenom). SNPs were selected for the replication step if they modulated either $A\beta_{1-42}$ or $pTau_{181}$ with a p-value of <5x10$^{-6}$ and showed consistent allele direction across all three GWAS. A total of 163 SNPs representing 36 novel loci met these criteria. Successful Sequenom assay design was achieved for 40 SNPs. These included rs429358, which characterizes APOE-ε4 vs. APOE-ε4 noncarriers, and 30 SNPs representing 29 potentially novel loci. For seven of the 36 novel loci, design of a Sequenom assay failed due to technical reasons and a lack of additional proxies for the respective locus. SNPs representing the nine other known AD susceptibility genes were also included (Table 2) (28). Primer sequences and assay conditions are available upon request. Thus a total of 40 SNPs were genotyped in the ADC cohort.

Since rs62256378 was imputed in the GWAS step, the SNP was genotyped directly in GWAS patients for whom DNA was available using Sequenom (Bonn1CSF n=112, and Bonn2CSF n=152).

### Quality control (QC)

The Bonn1 sample was part of the sample investigated by Harold et al. (28) which comprised 19,000 samples prior to QC. Harold et al. eliminated 53,383 SNPs and 2,850 subjects from data analysis after stringent QC. No German subjects were excluded. For the present analyses, a check was made for deviations from Hardy-Weinberg equilibrium (HWE) within the German subgroup, and 55 SNPs with a HWE p-value <10$^{-6}$ were excluded.

In the Bonn2 sample, seven individuals were excluded due to potential relatedness, as indicated by high genome-wide identity-by-state values (4 standard deviations greater than overall mean IBS). SNPs with a genotyping rate of <99% were excluded. Four individuals were also excluded due to a genotyping rate of <99%. Finally, 88 SNPs were excluded due to deviation from HWE (p <10$^{-6}$), and 80,000 SNPs due to low minor allele frequency (<1%). The QC criteria used for Bonn2 were applied to the entire ADNI cohort. In total, 24 individuals and 42,199 SNPs were excluded due to a low genotyping rate, 80 SNPs due to deviation from HWE, and 5,961 SNPs due to low minor allele frequency.

The QC applied to the population-based Bonn1 controls is described elsewhere (32). In this sample, 49 controls were removed due to a low genotyping rate. In addition, 80 controls were removed because they overlapped with the HNR control sample used in Bonn2.

In the ADC replication cohort, three samples were excluded due to a genotyping rate of <95%. In addition, 101 individuals were excluded because their covariate *APOE* status was not available. A total of five SNPs were excluded due to a low genotyping rate (<95%), strong deviation from HWE (p=1x10$^{-38}$), or monomorphic status.

**Statistical analyses**

For each of the three GWAS (Bonn1 CSF, Bonn2CSF, ADNI) genotype data were imputed using the 1,000 Genomes (33) reference data, May 2012 release, and the IMPUTEv2 software package (34). Imputed SNPs with an information score of <0.4 or a minor allele frequency of <3% were excluded. After imputation, a total of 6,812,394 SNPs were included in the initial GWAS step and the meta-analysis of the three GWAS.

To identify possible stratification within each cohort, a multidimensional scaling analysis was performed using PLINK (35). The three leading principal components were retained as covariates.

In each cohort, CSF levels were analyzed as quantitative traits (QT) using linear regression, as implemented in PLINK. Age, sex, and APOE status - as defined by the number of APOE-$\varepsilon$4 copies - were additional covariates.

The association results were combined in a meta-analysis using the fixed effects model (36), as implemented in YAMAS (37). In addition, heterogeneity measures (38) were computed and meta-analysis was performed under a random effects model (36).

The real genotypes of the replication ADC cohort were analyzed with INTERSNP (39), and combined with the GWAS results in the meta-analysis fashion described above. The explained variance was determined using the coefficient of determination $R^2$ of the linear regression model.

Two SNPs showing association with $A\beta_{1-42}$ level were replicated in the ADC, i.e. rs429358 at the APOE locus and rs62256378 at a novel locus containing the *SUCLG2* gene. A possible genetic interaction between these two association signals was explored as an indicator of a possible biological link. For this, SNP-SNP interaction was analyzed using an allelic interaction test (one degree of freedom test [1.d.f.]). The standard genotype coding and definition of interaction parameters provided by Cordell and Clayton (40) were used, as implemented in INTERSNP.

Case-control analysis was performed using a standard logistic regression test (1.d.f.).

Cognitive decline was computed as $\Delta MMSE/\Delta t$, where $\Delta MMSE$ was the difference in the MMSE scores between time point A and time point B, and where $\Delta t$ was the time in days between time points A and B. $\Delta MMSE/\Delta t$ was then analyzed as a QT. Educational status and MMSE at time point A were used as additional covariates.

**Human brain tissue**

Post-mortem human brain tissue specimens from patients with histologically confirmed AD and from age-matched controls who had died from non-neurological disease ($75\pm10$ years at the age of death) were obtained from the Neurological Tissue Bank of the Biobank from the Hospital Clinic-Institut d'Investigacions Biomèdiques August Pi i Sunyer in Barcelona. Post-mortem duration varied from 1.5 to 5 h; neither age nor post-mortem sampling time differed significantly between controls and AD patients.

**Immunohistochemistry**

For co-localization of SUCLG2 in human brain cortex tissue sections were subjected to paraffin removal at 60°C for 30 min, xylene treatment, and rehydration through a graded ethanol series. Antigen unmasking was performed by heating the tissue sample to 100°C for 20 min in 10mM sodium citrate buffer pH=6. Sections were incubated with rabbit anti-SUCLG2 antibody (Novus Biologicals, NBP1-32521) diluted to 1:400 and mouse anti-HLA-DR (Thermo scientific, MA1-35420) diluted to 1:100. Consecutive sections were further incubated with biotinylated secondary antibody, and signal amplification is achieved with HRP-streptavidin-biotin conjugates (Vectastain Elite ABC system). Diaminobenzidine-stained (DAB) sections where then counterstained with hematoxylin. Brightfield images were then obtained using an axioplan microscope (Zeiss). In parallel, sections were incubated with Alexa 488 and Alexa 546-conjugated secondary antibodies and mounted on a dapi-containing mounting media (Vectorlabs). Any attempt to quench autofluorescence was done. Hi-Res confocal images were obtained using a Leica SP5 Laser Scanning Confocal Microscope (Leica). Following deconvolution, 3D reconstruction was performed using Huygens Scientific Volume Imaging (SVI).

**Cell culture and transfection**

BV2 murine microglial cells were obtained from the American Type Culture Collection (ATCC). The cells were maintained at 37°C in 5%CO2 DMEM medium, supplemented with 10% fetal calf serum and 1% penicillin/streptomycin. The following were obtained from Invitrogen: three different Stealth siRNA directed to exons 4, 5, and 6-7

5'-GCGGUUUGAAAGGAGGUGUUCAUU-3' (SEQ ID NO: 36),
5'-CCCUGGAUAUUUCUAGAGAAACAUA-3' (SEQ ID NO: 37), and
5'-ACCAGGCUGCAGAUCAGAUUACAAA-3' (SEQ ID NO: 38);

the recommended Stealth negative control; and the positive (GAPDH) control siRNA duplexes. Transfections were performed using lipofectamine 2000 (Invitrogen) in accordance with the manufacturer's recommendations.

**Western blot**

Protein extracts from BV2 cells or frozen human brain tissue were homogenized in TNE buffer (10mM Tris-HCl pH 7.4; 150mM NaCl, 5mM EDTA; 0.5% NP-40; 1x complete protease inhibitor cocktail (Roche); 1x phosphatase inhibitor cocktail I; and II (Sigma-Aldrich). Protein concentration was determined using the BCA method (Pierce Biotechnology). A total of 20mg of protein was resolved by SDS-PAGE, transferred to PVDF, and probed with rabbit anti-SUCLG2 antibody (Novus Biologicals, NBP1-32521) diluted to 1:500. Following overnight incubation, the membranes were rinsed three times in TTBS and incubated at 22°C in 800CW donkey anti-rabbit secondary antibody in 5% nonfat milk in TTBS pH=7.5. The blots were visualized using a LI-COR Biosciences Odyssey infrared imaging system (Lincoln). After visualization, the blots were stripped through rinsing with TBS. They were then incubated at 55°C for 30 min in a buffer containing 200mM glycine, 2% SDS and 100mM mercaptoethanol. Membranes were rinsed in TBS and then blocked and reprobed with mouse monoclonal anti-Hsp75 (UC David/NIH NeuroMab Facility, clone N52A/42) diluted to 1:1000, rinsed three times in TTBS, incubated at 22°C in 800CW donkey antimouse secondary antibody in 5% nonfat milk in TTBS pH=7.5, and visualized using the LI-COR system (Lincoln).

**Phagocytosis of FAM-labeled $A\beta_{1-42}$ (FAM-$A\beta_{1-42}$)**

Following incubation of $AP_{i-42}$ at 37°C for three days, microglial phagocytosis of aggregated FAM-$A\beta_{1-42}$ (Anaspec) was measured using a plate-based assay. Control or SUCLG2-silenced BV2 cells were plated at 50000 cells per 100$\mu$l in black 96 well plates, and FAM-$A\beta_{1-42}$ was added to a final concentration of 500nM and incubated for 0, 2, or 4 h. The FAM-$A\beta_{1-42}$-containing medium was then removed and the remaining extracellular FAM-$A\beta_{1-42}$ was quenched for 1 min with 100$\mu$l 0.2% trypan blue in PBS pH=4.4. Following aspiration, fluorescence was measured at 485nm excitation/535nm emission using an Infinite 200 reader (Tecan). To normalize for cell numbers, 100$\mu$l at 50[$\mu$g/ml] Hoechst Dye 33342 in PBS was added and the sample was incubated for 30 min. Fluorescence was measured at 360nm excitation/465nm emission using the Infinite 200 reader (Tecan). In addition, microglial FAM-$A\beta_{1-42}$ phagocytosis was verified by confocal microscopy using a BX61 microscope equipped with a disc-spinning unit (Olympus).

**Mitochondrial DNA (mtDNA) Copy Number Analysis**

The experiments were performed on DNA extracted from peripheral blood samples. A segment of the mtDNA outside the 7S region was amplified using primers MT3922F25 and MT4036R26 (first number, 5' nucleotide of primer; second number, length of primer; F, forward primer; R, reverse primer; numbering according to reference sequence NC_012920). CT values were defined at the inflection points of fitted sigmoid curves (four-parameter Chapman curves) and were compared to those of the single-copy nuclear gene KCNJ10 amplified by primers KIR835F19 and KIR903R19 (numbering according to sequence U52155). The quantitative PCR was performed on the MyiQ real-time PCR system using the iQ SYBR Green Supermix (Bio-Rad, Munich, Germany) according to the instructions of the manufacturer. Amplification conditions were as follows: 95 °C for 3 minutes, and 45 cycles of 95 °C for 15 seconds and 60 °C for 1 minute. The exact cycle number values were calculated as ln(c) / b, which correspond to the inflection point of nonlinear fitted regression of the sigmoidal amplification curves applying the following equation:

$$y = y_0 + a \, (1 - \exp^{-bx})^c$$

($y_0$: background fluorescence intensity; y: measured fluorescence intensity; x: cycle number; and a, b, and c: curve parameters to be fitted). Calculations were performed using the SigmaPlot 2001 statistical analysis software. For each value, 3 independent experiments were performed with different concentrations of template DNA, each experiment in triplicate, and primer efficiencies were calculated. Serial dilutions from counted fibroblasts or mitochondrial PCR fragments were used to verify absolute copy numbers.

**Results**

**Study design**

GWAS data on CSF biomarkers $A\beta_{1-42}$ and $pTau_{181}$ from three independent AD cohorts were analyzed (Bonn1 CSF, n=113; Bonn2CSF, n=167; ADNI, n=83). The resulting data were then combined in a meta-analysis. The three GWAS cohorts are described in Table 3

For the replication step, SNPs were selected that showed consistent allele direction across all three GWAS and which modulated either $A\beta_{1-42}$ or $pTau_{181}$ with a p-value<$5x10^{-6}$ and which showed consistent allele direction across all studies. These 30 SNPs, together with 10 SNPs representing known AD susceptibility genes, were then genotyped in an independent sample of 515 AD patients (ADC). These 40 SNPs represented a total of 39 loci. Two SNPs showing association with CSF levels of $A\beta_{1-42}$ in the GWAS meta-analysis were replicated in the ADC, i.e. rs429358 at the *APOE* locus and rs62256378 at a novel locus containing the *SUCLG2* gene. The ADC sample is described in detail in the Online Methods and Table 3. Since these two replicated loci both modulated $A\beta_{1-42}$ levels in the CSF a possible genetic interaction was explored, since this might suggest a biological link between the two loci. It was also investigated whether rs62256378: (i) confers AD risk , using a case-control sample; and (ii) has a possible effect on cognitive decline in AD patients, using prospective data on Mini-mental State Examination (MMSE) scores. The samples used in these two analyses are described in detail in the Online Methods.

Functional experiments were then performed to elucidate the functional role of SUCLG2 in $A\beta_{1-42}$ homeostasis. Firstly, the cellular distribution of SUCLG2 was examined in post mortem human brain tissue from individuals who had died from non-neurological disease. Secondly, to follow up the finding concerning genetic interaction with APOE, the effect of APOE-ε4 carrier status on the protein expression of SUCLG2 was investigated using protein extracts from human AD and control post mortem frontal cortex. Thirdly, the role of SUCLG2 in microglial $A\beta_{1-42}$ phagocytosis was analyzed by reducing SUCLG2 expression through siRNA approaches.

**GWAS meta-analysis**

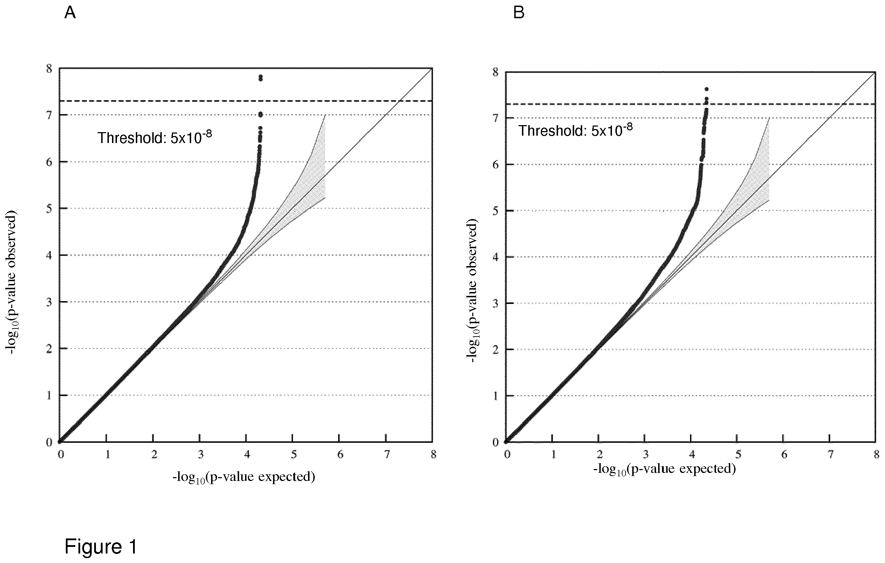

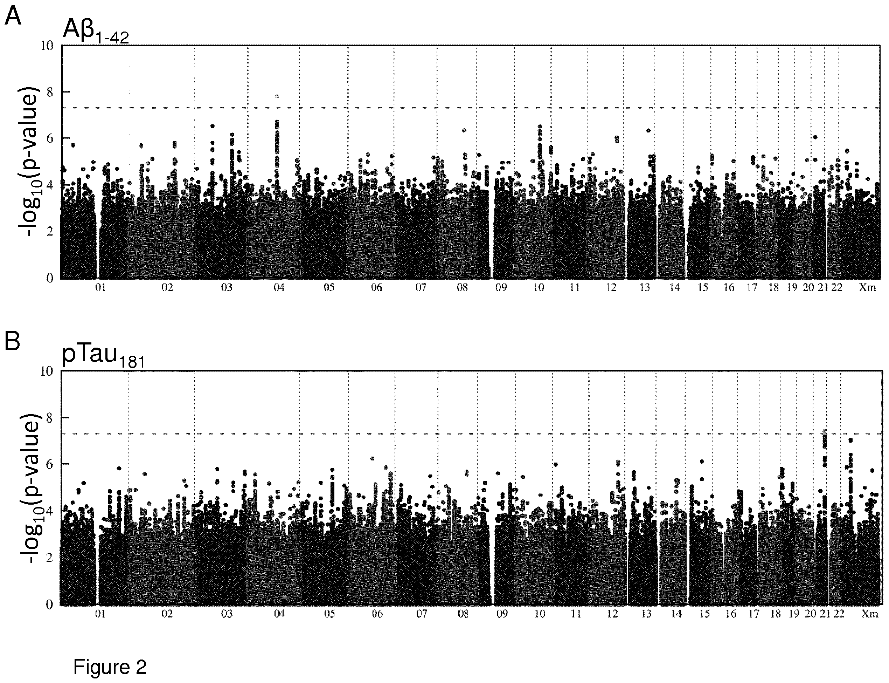

Meta-analysis of the three GWAS yielded genome-wide inflation factors of λ=1.05 for $A\beta_{1-42}$ and λ=1.03 for $pTau_{181}$ (Fig. 1). After A-adjustment, a genome-wide significant association was observed between APOE-ε4 (rs429358) and $A\beta_{1-42}$ (p=$1.8x10^{-10}$), thus confirming published data (Table 2). A total of 335 SNPs were below the threshold of genome-wide significance but had a promising p-value of <$5x10^{-6}$ (Fig. 2). Of these, 163 showed consistent allele direction across all three GWAS.

**Replication and meta-analysis of combined data**

Thirty-five of the 40 SNPs included in the replication step were successfully genotyped (Table 4).

The replication step revealed a strong association between rs429358 (APOE-ε4) and $A\beta_{1-42}$ levels (p=$1.1x10^{-5}$, β=$-0.7\pm0.015$, Table 2). This association became stronger in a meta-analysis of the combined GWAS and replication data (p=$4.3x10^{-17}$, β=$-0.4\pm0.05$, Table 2).

The second strongest replication signal was for association between a SNP on chromosome 3p14.1 (rs62256378, minor allele frequency [MAF] =6.1%), and $A\beta_{1-42}$ levels (p=$1.9x10^{-6}$, β=$0.61\pm0.13$, Fig. 3). The meta-analysis of the GWAS and replication data generated a highly significant finding (p=$2.5x10^{-12}$, β=$0.71\pm0.10$), with the low frequency allele being associated with higher $A\beta_{1-42}$ levels and showing an effect size that was larger than that of APOE-ε4. Since rs62256378 was imputed, we genotyped this SNP in 264 individuals from the GWAS step for whom DNA was available. The genotyping of rs62256378 using Sequenom technology revealed a concordance between imputed and real genotypes of >99%, demonstrating the validity of the imputed rs62256378 genotypes in the GWAS step (the results of the statistical calculations that involved imputed and real genotypes are provided in Table 5).

With the exception of *APOE*, none of the known AD susceptibility genes showed genome-wide significant association with $A\beta_{1-42}$ or $pTau_{181}$ levels in the meta-analysis of the three GWAS and the replication sample (Table 6). Both *CD2AP* (rs9349407) and *APOE* (rs429358) showed a nominally significant association with increased $pTau_{181}$ levels (p=0.031 and p=0.029, respectively).

In a recent GWAS by Cruchaga et al5 the following three novel genome-wide significant findings were found for total Tau and $pTau_{181}$ levels: (i) rs9877502 at *SNAR-I,* p=$4.89x10^{-9}$ and p=$1.68x10^{-7}$ for total Tau and $pTau_{181}$, respectively; (ii) rs514716 at *GLIS3,* p=$1.07x10^{-8}$ and p=$3.22x10^{-9}$ for Tau and $pTau_{181}$, respectively; and (iii) rs6922617 at *NCR2,* p=$2.55x10^{-5}$ and p=$3.58x10^{-8}$ for total Tau and $pTau_{181}$, respectively. Of these three SNPs, only rs9877502 and $pTau_{181}$ showed a nominally significant association in the present GWAS meta-analysis of $pTau_{181}$ (p=$9.21x10^{-4}$). Interestingly, a non-significant trend for rs6922617 was observed (p=0.07). This locus received further support from the adjacent SNP rs11966476 (p=0.04) (Table 7).

**Effect of rs62256378 on AD risk**

In the case-control analysis, no association was found between rs62256378 and AD risk (p=0.797, OR=$1.03\pm0.12$).

**Genetic interaction between rs429358 and rs62256378**

Both of the presently replicated loci modulated $A\beta_{1-42}$ levels. APOE-$\epsilon$4 was associated with lower $A\beta_{1-42}$ levels, and the minor allele A of rs62256378 was associated with higher $A\beta_{1-42}$ levels. Investigation of a possible interaction between the two association signals revealed strong evidence for allelic interaction (p=9.5x10$^{-5}$, Table 8). The effect of the minor allele A of rs62256378 varied substantially depending on APOE-$\epsilon$4 carrier/noncarrier status (Table 9). The strongest effect of rs62256378-A in terms of elevation of $A\beta_{1-42}$ levels was observed in APOE-$\epsilon$4 noncarriers (p=1.5x10$^{-8}$, $\beta$=0.97$\pm$0.17, Table 9, Fig. 4). In heterozygous APOE-$\epsilon$4 carriers, the effect remained significant, although the level of significance was reduced (p=1.5x10$^{-3}$, $\beta$=0.35$\pm$0.11, Table 9, Fig. 4). In APOE-$\epsilon$4 homozygotes, no significant effect was detectable for rs62256378-A (p=0.84). This finding was supported by the consistent pattern of interaction observed across all four sub-samples (Table 9).

**rs62256378 and cognitive decline**

The SNP rs62256378-A significantly attenuated cognitive decline in AD patients (p=3.1x10$^{-3}$, $\beta$=0.32$\pm$0.12, Table 10, Fig. 5). After stratification for APOE genotype, this effect was significant in APOE-$\epsilon$4 noncarriers (p=0.021, $\beta$=0.32$\pm$0.16, Table 10). A non-significant trend was observed in heterozygous APOE-$\epsilon$4 carriers (p=0.07, $\beta$=0.31$\pm$0.21). There was no evidence for interaction between *SUCLG2* (rs62256378) and APOE genotype (p=0.456).

**Conditional analysis of the SUCLG2 locus**

This marker is located within a recombination hotspot. This hotspot defines two linkage disequilibrium blocks that span several exons of *SUCLG2* on chromosome 3p14.1 (Fig. 3). Conditional analysis showed that all additional signals in this region were dependent on rs62256378.

**Functional investigation of the role of SUCLG2**

Immunohistochemistry revealed that SUCLG2 expression was mainly confined to microglial cells (6) in the post-mortem brains of AD patients and age-matched controls (Fig. 6A and 6B). The strongest association between rs62256378 and higher $A\beta_{1-42}$ levels and MMSE stabilization was observed in APOE-$\epsilon$4 noncarriers. To determine whether *APOE* status influenced SUCLG2 levels, SUCLG2 protein levels were analyzed in human postmortem samples from the frontal cortex, a brain region affected at an early stage of AD. Here, ApoE3/3 carriers showed significantly higher SUCLG2 levels compared to ApoE4/3 carriers (Fig. 6C). Controls in turn showed the lowest levels, reinforcing the hypothesis that rs62256378 may influence the disease-course in AD, rather than overall disease-risk. SUCLG2 levels in microglial cells were then lowered using various siRNA approaches (Fig. 7). Decreasing microglial SUCLG2 compromised $A\beta_{1-42}$ phagocytosis in a time dependent manner (Fig. 6D).

**mtDNA copy number in AD patients**

In DNA obtained from peripheral blood of Alzheimer patients, we observed that mtDNA copy number fluctuates depending on the specific allele combinations for *APOE* and rs62256378. Thus in patients APOE-$\epsilon$4 noncarriers and carriers of the protective allele A variant in rs62256378, we detected a significant reduction of mtDNA copy number when compared to AD patients APOE-$\epsilon$4 carriers and carriers of the common G allele in rs62256378 and healthy controls (Fig. 8).

**Discussion**

The present study demonstrated that genetic variation in *SUCLG2* modulated $A\beta_{1-42}$ levels in AD dementia patients. Furthermore, it was found that *SUCLG2* was associated with AD clinical course, as measured by change in MMSE over time.

The negative result of the case-control analysis suggests that this gene does not confer AD susceptibility, which would explain why it has not been identified in previous case-control GWAS of AD. Possible reasons for the failure of previous GWAS of CSF markers to identify *SUCLG2* are threefold. Firstly, they included unaffected subjects and individuals with MCI. Secondly, they analyzed non-imputed datasets. Thirdly, APOE was not used as a covariate in the analyses. In contrast, in this study the analysis was restricted to AD dementia only, the genetic data were imputed and APOE was included as covariate.

The data also confirm the previously reported association between the APOE locus and $A\beta_{1-42}$ levels. Interestingly, the effect of *SUCLG2* was mainly observed in APOE-$\epsilon$4 noncarriers.

The effect of *SUCLG2* on $A\beta_{1-42}$ levels was surprisingly high, since it was not expected to identify a novel gene with an effect comparable to *APOE*. *SUCLG2* explained 10.7% of the variance in $A\beta_{1-42}$ levels in APOE-$\varepsilon$4 noncarriers, which is more than the variance explained by APOE-$\varepsilon$4 in the overall sample (7.1%, Table 11).

None of the presently investigated SNPs showed genome-wide significant association with $pTau_{181}$. To date, genome-wide significant association with total $Tau/pTau_{181}$ levels has been reported for four loci, i.e. *APOE, SNAR-I, GLIS3,* and *NCR2* (1, 4, 5). A nominally significant association between the $pTau_{181}$ level and three SNPs was observed: rs429358 (*APOE* locus); rs9877502 (*SNAR-I*); and rs11966476 (*NCR2*). A possible reason for the failure to replicate the finding for *GLIS3* is that in addition to AD patients, the study by Cruchaga et al. also included MCI patients and controls. If *GLIS3* only contributes to $pTau_{181}$ variance in unaffected individuals or during the preclinical stages of disease, this effect may not be present in this sample.

With the exception of *APOE,* none of the known AD susceptibility genes showed genome-wide significant association with either $A\beta_{1-42}$ or $pTau_{181}$ levels in the present study. Previous studies have reported associations between $A\beta_{1-42}$ or $pTau_{181}$ levels and the AD susceptibility genes *PICALM, CLU, MS4A4A,* and *TREM2 (2, 3, 5).* Again, the failure to replicate these findings, even at a nominal significant level, may have been due to the fact that this study focussed on AD patients only. The present study is the first to report an association between the AD susceptibility gene *CD2AP* (rs9349407) and $pTau_{181}$ level (p=0.031), although this finding was only nominally significant and requires replication. In the case of *TREM2,* a genuine association between the SNP originally associated with AD (rs75932628; p.R47H) and $pTau_{181}$ cannot be ruled out, since none of the present SNPs in this region displayed sufficient linkage disequilibrium with rs75932628, and this SNP cannot be imputed adequately using available variant databases.

*SUCLG2* encodes a GTP-specific beta subunit of the succinyl-CoA ligase (SUCL). The mitochondrial matrix enzyme SUCL is a heterodimer, which is formed by an invariable alpha-subunit (encoded by *SUCLG1*), and a variable beta subunit which confers nucleoside specificity. This beta subunit is encoded by either *SUCLA2* or *SUCLG2.* In the case of SUCLG2, the SUCL enzyme catalyzes the reversible conversion of succinyl-CoA and GDP to succinate and GTP respectively (7). Previous studies have identified mutations in *SUCLG1* and *SUCLA2* in patients with mitochondrial DNA (mtDNA) depletion syndromes (MDS). MDS are characterized by a decrease in mtDNA content and a reduction in the activity of mitochondrial respiratory chain complexes I, II, IV, and V (8). Interestingly, both features have also been found, to a lesser extent, in AD (9). In line with this, Miller et al. demonstrated that reducing the expression of *SUCLG2* led to a decrease in both mtDNA and the activity mitochondrial complex IV (10).

Our observation of higher SUCLG2 expression in APOE-$\varepsilon$4 noncarriers may be explained in terms of the amyloid cascade hypothesis. While hereditary AD is caused by an overproduction of $A\beta_{1-42}$ secondary to autosomal dominant mutations in genes coding for APP and presenilin 1 and 2, sporadic AD is more likely to arise from limited clearance of $A\beta_{1-42}$ (11). Limited clearance by microglia is likely to contribute to increased $A\beta_{1-42}$ burden in the brains of sporadic AD patients, as described recently for the CD33 risk allele rs3865444 (12). In the present study, the association between rs62256378 and $A\beta_{1-42}$ CSF levels was modified by ApoE-$\varepsilon$4. The latter also decreased the level of SUCLG2 in microglia, and this in turn impaired $A\beta_{1-42}$ phagocytosis. Removal of $A\beta_{1-42}$ by microglia is likely to decrease the propensity of $A\beta_{1-42}$ to aggregate and deposit. Therefore, stabilized SUCLG2 levels and concomitant microglial clearance may maintain higher $A\beta_{1-42}$ CSF levels by reducing the tendency of $A\beta_{1-42}$ to aggregate. Furthermore, microglial function may also be of relevance to cognitive performance, as suggested by recent experimental data, which showed that improved microglial clearance was protective in terms of memory function (13). A plausible hypothesis is that improved microglial function may account for the stabilization of MMSE scores in rs62256378-A carriers. These findings are consistent with a number of previous reports, which have implicated innate immune activation and microglial clearance in sporadic AD.

Microglial phagocytosis of $A\beta_{1-42}$ is highly energy-dependent, and most of this energy is provided by mitochondria. The maintenance of mtDNA is crucial for mitochondrial function, since it encodes the key enzymes of mitochondrial respiration. In AD, several lines of evidence have confirmed changes in the quantity and quality of mitochondrial mtDNA (9). These changes may lead to mitochondrial impairment in the AD brain, as demonstrated by an elevation in the number of mitochondria with disrupted cristae and accumulated osmiophilic material (14). Interestingly, a previous report showed that *SUCLG2* silencing caused a significant reduction in both mitochondrial DNA (mtDNA), and the activity of cytochrome oxidase (COX or complex IV) in fibroblasts from MDS patients with *SUCLA2* mutations and healthy controls (10). Hence, the beneficial effect of the *SUCLG2* A allele on the clinical course of AD may be mediated by improved mtDNA maintenance, a compensatory mechanism which increases the clearance of $A\beta_{1-42}$ and dying neurons. Interestingly, while the evidence for interaction of *SUCLG2* with APOE was strong in the effect on $A\beta_{1-42}$ levels (p=9.5x10$^{-5}$), such an interaction was not evident in the analysis of the clinical course (p=0.456).

This may suggest that *SUCLG2* contributes to $A\beta_{1-42}$ and clinical course through at least partially independent pathways, and that the pathway leading to variance in clinical course is not or at least not strongly influenced by APOE. This hypothesis would be consistent with the lack of consistent evidence that APOE genotype might confer an effect on the clinical course, despite its very strong effect on $A\beta_{1-42}$ levels (and on disease susceptibility).

**References**

1. Kim, S. et al. Genome-wide association study of CSF biomarkers Abeta1-42, t-tau, and p-tau181p in the ADNI cohort. Neurology 76, 69-79 (2011).

2. Schjeide, B.M. et al. The role of clusterin, complement receptor 1, and phosphatidylinositol binding clathrin assembly protein in Alzheimer disease risk and cerebrospinal fluid biomarker levels. Arch Gen Psychiatry 68, 207-13 (2011).

3. Elias-Sonnenschein, L.S. et al. Genetic loci associated with Alzheimer's disease and cerebrospinal fluid biomarkers in a Finnish case-control cohort. PLoS One 8, e59676 (2013).

4. Han, M.R., Schellenberg, G.D. & Wang, L.S. Genome-wide association reveals genetic effects on human Abeta42 and tau protein levels in cerebrospinal fluids: a case control study. BMC Neurol 10, 90 (2010).

5. Cruchaga, C. et al. GWAS of cerebrospinal fluid tau levels identifies risk variants for Alzheimer's disease. Neuron 78, 256-68 (2013).

6. Mattiace, L.A., Davies, P. & Dickson, D.W. Detection of HLA-DR on microglia in the human brain is a function of both clinical and technical factors. Am J Pathol 136, 1101-14 (1990).

7. Johnson, J.D., Mehus, J.G., Tews, K., Milavetz, B.I. & Lambeth, D.O. Genetic evidence for the expression of ATP- and GTP-specific succinyl-CoA synthetases in multicellular eucaryotes. J Biol Chem 273, 27580-6 (1998).

8. Ostergaard, E. Disorders caused by deficiency of succinate-CoA ligase. J Inherit Metab Dis 31, 226-9 (2008).

9. Silva, D.F. et al. Mitochondrial abnormalities in Alzheimer's disease: possible targets for therapeutic intervention. Adv Pharmacol 64, 83-126 (2012).

10. Miller, C., Wang, L., Ostergaard, E., Dan, P. & Saada, A. The interplay between SUCLA2, SUCLG2, and mitochondrial DNA depletion. Biochim Biophys Acta 1812, 625-9 (2011).

11. Mawuenyega, K.G. et al. Decreased clearance of CNS beta-amyloid in Alzheimer's disease. Science 330, 1774 (2010).

12. Bradshaw, E.M. et al. CD33 Alzheimer's disease locus: altered monocyte function and amyloid biology. Nat Neurosci 16, 848-50 (2013).

13. Heneka, M.T. et al. NLRP3 is activated in Alzheimer's disease and contributes to pathology in APP/PS1 mice. Nature 493, 674-8 (2013).

14. Baloyannis, S.J. Mitochondrial alterations in Alzheimer's disease. JAlzheimers Dis 9, 119-26 (2006).

15. van der Flier, W.M., Pijnenburg, Y.A., Fox, N.C. & Scheltens, P. Early-onset versus late-onset Alzheimer's disease: the case of the missing APOE varepsilon4 allele. Lancet Neurol 10, 280-8 (2011).

16. McKhann, G. et al. Clinical diagnosis of Alzheimer's disease: report of the NINCDS-ADRDA Work Group under the auspices of Department of Health and Human Services Task Force on Alzheimer's Disease. Neurology 34, 939-44 (1984).

17. Kornhuber, J. et al. Early and differential diagnosis of dementia and mild cognitive impairment: design and cohort baseline characteristics of the German Dementia Competence Network. Dement Geriatr Cogn Disord 27, 404-17 (2009).

18. Jessen, F. et al. Prediction of dementia in primary care patients. PLoS One 6, e16852 (2011).

19. Luck, T. et al. Mild cognitive impairment in general practice: age-specific prevalence and correlate results from the German study on ageing, cognition and dementia in primary care patients (AgeCoDe). Dement Geriatr Cogn Disord 24, 307-16 (2007).

20. Zaudig, M. etal. SIDAM--A structured interview for the diagnosis of dementia of the Alzheimer type, multi-infarct dementia and dementias of other aetiology according to ICD-10 and DSM-III-R. Psychol Med 21, 225-36 (1991).

21. McKhann, G.M. etal. The diagnosis of dementia due to Alzheimer's disease: recommendations from the National Institute on Aging-Alzheimer's Association workgroups on diagnostic guidelines for Alzheimer's disease. Alzheimers Dement 7, 263-9 (2011).

22. Kempen, G.I., Meier, A.J., Bouwens, S.F., van Deursen, J. & Verhey, F.R. [The psychometric properties of the Dutch version of the Telephone Interview Cognitive Status (TICS)]. Tijdschr Gerontol Geriatr 38, 38-45 (2007).

23. Schmermund, A. et al. Assessment of clinically silent atherosclerotic disease and established and novel risk factors for predicting myocardial infarction and cardiac death in healthy middle-aged subjects: rationale and design of the Heinz Nixdorf RECALL Study. Risk Factors, Evaluation of Coronary Calcium and Lifestyle. Am Heart J 144, 212-8 (2002).

24. Krawczak, M. et al. PopGen: population-based recruitment of patients and controls for the analysis of complex genotype-phenotype relationships. Community Genet 9, 55-61 (2006).

25. Wichmann, H.E., Gieger, C. & Illig, T. KORA-gen--resource for population genetics, controls and a broad spectrum of disease phenotypes. Gesundheitswesen 67 Suppl 1, S26-30 (2005).

26. Popp, J. et al. Cerebrospinal fluid markers for Alzheimer's disease over the lifespan: effects of age and the APOEepsilon4 genotype. J Alzheimers Dis 22, 459-68 (2010).

27. Mulder, C. et al. Amyloid-beta(1-42), total tau, and phosphorylated tau as cerebrospinal fluid biomarkers for the diagnosis of Alzheimer disease. Clin Chem 56, 248-53 (2010).

28. Harold, D. et al. Genome-wide association study identifies variants at CLU and PICALM associated with Alzheimer's disease. Nat Genet 41, 1088-93 (2009).

29. Hollingworth, P. et al. Common variants at ABCA7, MS4A6A/MS4A4E, EPHA1, CD33 and CD2AP are associated with Alzheimer's disease. Nat Genet 43, 429-35 (2011).

30. Lambert, J.C. et al. Genome-wide association study identifies variants at CLU and CR1 associated with Alzheimer's disease. Nat Genet 41, 1094-9 (2009).

31. Naj, A.C. et al. Common variants at MS4A4/MS4A6E, CD2AP, CD33 and EPHA1 are associated with late-onset Alzheimer's disease. Nat Genet 43, 436-41 (2011).

32. Mangold, E. et al. Genome-wide association study identifies two susceptibility loci for nonsyndromic cleft lip with or without cleft palate. Nat Genet 42, 24-6 (2010).

33. Abecasis, G.R. etal. A map of human genome variation from population-scale sequencing. Nature 467, 1061-73 (2010).

34. Howie, B.N., Donnelly, P. & Marchini, J. A flexible and accurate genotype imputation method for the next generation of genome-wide association studies. PLoS Genet 5, e1000529 (2009).

35. Purcell, S. et al. PLINK: a tool set for whole-genome association and population-based linkage analyses. Am J Hum Genet 81, 559-75 (2007).

36. de Bakker, P.I. et al. Practical aspects of imputation-driven meta-analysis of genome-wide association studies. Hum Mol Genet 17, R122-8 (2008).

37. Meesters, C. etal. Quick, "imputation-free" meta-analysis with proxy-SNPs. BMC Bioinformatics 13, 231 (2012).

38. Huedo-Medina, T.B., Sanchez-Meca, J., Marin-Martinez, F. & Botella, J. Assessing heterogeneity in meta-analysis: Q statistic or I2 index? Psychol Methods 11, 193-206 (2006).

39. Herold, C., Steffens, M., Brockschmidt, F.F., Baur, M.P. & Becker, T. INTERSNP: genome-wide interaction analysis guided by a priori information. Bioinformatics 25, 3275-81 (2009).

40. Cordell, H.J. & Clayton, D.G. A unified stepwise regression procedure for evaluating the relative effects of polymorphisms within a gene using case/control or family data: application to HLA in type 1 diabetes. Am J Hum Genet 70, 124-41 (2002).

Table 2: Summary of GWAS, replication, and overall meta-analysis for SNPs with $p<5x10^{-6}$ and the same effect direction in all three GWAS samples

GWAS: Genome-wide association study.

a

| Phenotype[a] | | QC[b] | Chr[c] | rs[d] | bp[e] | minor[f] | major[g] | maf[h] | Missing Rate[i] | pHWE[j] | beta[k] | se[l] | p[m] |
|---|---|---|---|---|---|---|---|---|---|---|---|---|---|
| Aβ1-42 | | OK | 1 | rs3818361 | 207784968 | T | C | 0.223 | 0.027 | 0.076 | -0.190 | 0.084 | 0.024 |
| | | out | 1 | rs10779606 | 213841768 | C | G | 0.443 | 0.050 | 2.53x10⁻³⁸ | -0.151 | 0.053 | 0.005 |
| | | OK | 2 | rs6753292 | 46002160 | T | C | 0.251 | 0.000 | 0.862 | 0.009 | 0.075 | 0.908 |
| | | OK | 2 | rs72804029 | 59273581 | T | C | 0.089 | 0.005 | 0.146 | 0.173 | 0.118 | 0.144 |
| | | OK | 2 | rs744373 | 127894615 | C | T | 0.328 | 0.009 | 0.359 | -0.035 | 0.072 | 0.623 |
| | | out | 2 | rs11884035 | 169247034 | 0 | T | 0.000 | 0.000 | 1.000 | - | - | 1.000 |
| | | OK | 2 | rs34737109 | 172905689 | A | G | 0.383 | 0.000 | 0.878 | -0.080 | 0.066 | 0.231 |
| | | OK | 3 | rs62256378 | 67457033 | A | G | 0.061 | 0.000 | 0.052 | 0.608 | 0.126 | 1.9x10⁻⁶ |
| | | OK | 3 | rs4407373 | 139255012 | T | G | 0.099 | 0 | 0.472 | -0.045 | 0.111 | 0.683 |
| | | OK | 3 | rs2222464 | 165289424 | C | T | 0.274 | 0.000 | 0.599 | -0.023 | 0.073 | 0.757 |
| | | OK | 4 | rs388575 | 107973286 | C | T | 0.252 | 0.016 | 0.886 | 0.049 | 0.074 | 0.504 |
| | | OK | 4 | rs376496 | 107974207 | G | C | 0.249 | 0.000 | 0.886 | 0.086 | 0.074 | 0.245 |
| | | OK | 6 | rs9349407 | 47453378 | C | G | 0.263 | 0.007 | 0.392 | -0.054 | 0.073 | 0.462 |
| | | OK | 6 | rs12527308 | 63061935 | T | C | 0.150 | 0.000 | 0.963 | 0.092 | 0.090 | 0.309 |
| | | OK | 6 | rs9360453 | 72154332 | A | G | 0.291 | 0.000 | 0.412 | -0.042 | 0.070 | 0.543 |
| | | OK | 6 | rs4896367 | 138807281 | C | T | 0.268 | 0.000 | 0.405 | -0.023 | 0.071 | 0.748 |
| | | OK | 6 | rs6911312 | 154761284 | C | A | 0.356 | 0.000 | 0.694 | 0.042 | 0.068 | 0.542 |
| | | OK | 6 | rs9397794 | 155575942 | A | G | 0.245 | 0.000 | 0.690 | 0.091 | 0.074 | 0.222 |
| | | OK | 7 | rs11767557 | 143109139 | C | T | 0.180 | 0.007 | 0.390 | -0.026 | 0.084 | 0.757 |
| | | OK | 8 | rs11136000 | 27464519 | T | C | 0.343 | 0.009 | 0.446 | 0.030 | 0.071 | 0.669 |
| | | OK | 9 | rs73402275 | 10369118 | C | T | 0.129 | 0.000 | 0.876 | -0.061 | 0.097 | 0.530 |
| | | OK | 10 | rs77131199 | 26798222 | A | G | 0.037 | 0.000 | 0.613 | 0.126 | 0.168 | 0.455 |
| | | OK | 10 | rs2258946 | 93624672 | T | C | 0.145 | 0.000 | 0.154 | -0.095 | 0.089 | 0.286 |
| | | OK | 11 | rs12418935 | 11863410 | A | G | 0.107 | 0.016 | 0.140 | -0.098 | 0.108 | 0.362 |
| | | OK | 11 | rs610932 | 59939307 | A | C | 0.392 | 0.016 | 0.678 | -0.026 | 0.068 | 0.706 |
| | | OK | 11 | rs3851179 | 85868640 | A | G | 0.356 | 0.009 | 0.430 | -0.017 | 0.070 | 0.811 |
| | | OK | 12 | rs7304039 | 108736352 | A | G | 0.295 | 0.002 | 0.925 | -0.013 | 0.071 | 0.857 |

EP 3 077 533 B1

pTau181

| | | | | | | | | | | | |
|---|---|---|---|---|---|---|---|---|---|---|---|
| OK | 13 | rs4942417 | 32887389 | C | A | 0.154 | 0.000 | 0.859 | 0.047 | 0.090 | 0.599 |
| OK | 13 | rs74922937 | 91829564 | A | G | 0.026 | 0.000 | 0.189 | 0.019 | 0.198 | 0.923 |
| OK | 15 | rs143825603 | 61848856 | C | T | 0.053 | 0.000 | 0.812 | -0.046 | 0.146 | 0.751 |
| OK | 18 | rs7230126 | 13890199 | C | T | 0.317 | 0.000 | 0.222 | -0.038 | 0.067 | 0.575 |
| OK | 18 | rs3794851 | 74709351 | C | G | 0.046 | 0.000 | 0.960 | -0.018 | 0.153 | 0.908 |
| OK | 19 | rs3764650 | 1046520 | G | T | 0.109 | 0.009 | 0.903 | -0.002 | 0.106 | 0.983 |
| OK | 19 | rs429358 (APOE-ε4) | 45411941 | ε4 | non-ε4 | 0.442 | 0.000 | 0.732 | -0.701 | 0.149 | $1.1 \times 10^{-5}$ |
| OK | 19 | rs3865444 | 51727962 | T | G | 0.304 | 0.020 | 0.626 | 0.053 | 0.072 | 0.460 |
| OK | 21 | rs11700591 | 41507695 | C | T | 0.068 | 0.009 | 0.144 | 0.015 | 0.126 | 0.903 |
| OK | 23 | rs217969 | 118527877 | T | A | 0.256 | 0.002 | 0.188 | -0.084 | 0.061 | 0.164 |
| OK | 1 | rs3818361 | 207784968 | T | C | 0.223 | 0.027 | 0.076 | 0.126 | 0.087 | 0.150 |
| out | 1 | rs10779606 | 213841768 | C | G | 0.443 | 0.050 | $2.53 \times 10^{-38}$ | 0.031 | 0.056 | 0.582 |
| OK | 2 | rs6753292 | 46002160 | T | C | 0.251 | 0.000 | 0.862 | -0.113 | 0.080 | 0.156 |
| OK | 2 | rs72804029 | 59273581 | T | C | 0.089 | 0.005 | 0.146 | 0.044 | 0.126 | 0.725 |
| OK | 2 | rs744373 | 127894615 | C | T | 0.328 | 0.009 | 0.359 | 0.080 | 0.075 | 0.284 |
| out | 2 | rs11884035 | 169247034 | 0 | T | 0.000 | 0.000 | 1.000 | - | - | 1.000 |
| OK | 2 | rs34737109 | 172905689 | A | G | 0.383 | 0.000 | 0.878 | -0.064 | 0.071 | 0.365 |
| OK | 3 | rs62256378 | 67457033 | A | G | 0.061 | 0.000 | 0.052 | -0.005 | 0.138 | 0.974 |
| OK | 3 | rs4407373 | 139255012 | T | G | 0.099 | 0 | 0.472 | 0.012 | 0.116 | 0.921 |
| OK | 3 | rs2222464 | 165289424 | C | T | 0.274 | 0.000 | 0.599 | -0.038 | 0.078 | 0.628 |
| OK | 4 | rs388575 | 107973286 | C | T | 0.252 | 0.016 | 0.886 | 0.003 | 0.080 | 0.970 |
| OK | 4 | rs376496 | 107974207 | G | C | 0.249 | 0.000 | 0.886 | -0.027 | 0.079 | 0.736 |
| OK | 6 | rs9349407 | 47453378 | C | G | 0.263 | 0.007 | 0.392 | 0.103 | 0.076 | 0.177 |
| OK | 6 | rs12527308 | 63061935 | T | C | 0.150 | 0.000 | 0.963 | -0.060 | 0.096 | 0.533 |
| OK | 6 | rs9360453 | 72154332 | A | G | 0.291 | 0.000 | 0.412 | 0.019 | 0.074 | 0.800 |
| OK | 6 | rs4896367 | 138807281 | C | T | 0.268 | 0.000 | 0.405 | -0.038 | 0.076 | 0.619 |
| OK | 6 | rs6911312 | 154761284 | C | A | 0.356 | 0.000 | 0.694 | -0.010 | 0.073 | 0.895 |
| OK | 6 | rs9397794 | 155575942 | A | G | 0.245 | 0.000 | 0.690 | -0.012 | 0.079 | 0.883 |
| OK | 7 | rs11767557 | 143109139 | C | T | 0.180 | 0.007 | 0.390 | -0.080 | 0.087 | 0.360 |
| OK | 8 | rs11136000 | 27464519 | T | C | 0.343 | 0.009 | 0.446 | -0.062 | 0.074 | 0.401 |
| OK | 9 | rs73402275 | 10369118 | C | T | 0.129 | 0.000 | 0.876 | 0.105 | 0.103 | 0.309 |
| OK | 10 | rs77131199 | 26798222 | A | G | 0.037 | 0.000 | 0.613 | 0.011 | 0.180 | 0.951 |
| OK | 10 | rs2258946 | 93624672 | T | C | 0.145 | 0.000 | 0.154 | 0.213 | 0.094 | 0.024 |
| OK | 11 | rs12418935 | 11863410 | A | G | 0.107 | 0.016 | 0.140 | -0.053 | 0.118 | 0.653 |
| OK | 11 | rs610932 | 59939307 | A | C | 0.392 | 0.016 | 0.678 | -0.103 | 0.071 | 0.150 |
| OK | 11 | rs3851179 | 85868640 | A | G | 0.356 | 0.009 | 0.430 | -0.043 | 0.073 | 0.560 |
| OK | 12 | rs7304039 | 108736352 | A | G | 0.295 | 0.002 | 0.925 | -0.073 | 0.076 | 0.340 |

| | | | | | | | | | | | |
|---|---|---|---|---|---|---|---|---|---|---|---|
| OK | 13 | rs4942417 | 32887389 | C | A | 0.154 | 0.000 | 0.859 | -0.077 | 0.095 | 0.419 |
| OK | 13 | rs74922937 | 91829564 | A | G | 0.026 | 0.000 | 0.189 | -0.171 | 0.211 | 0.417 |
| OK | 15 | rs143825603 | 61848856 | C | T | 0.053 | 0.000 | 0.812 | -0.027 | 0.155 | 0.862 |
| OK | 18 | rs7230126 | 13890199 | C | T | 0.317 | 0.000 | 0.222 | 0.031 | 0.072 | 0.670 |
| OK | 18 | rs3794851 | 74709351 | C | G | 0.046 | 0.000 | 0.960 | -0.014 | 0.163 | 0.934 |
| OK | 19 | rs3764650 | 1046520 | G | T | 0.109 | 0.009 | 0.903 | -0.090 | 0.110 | 0.416 |
| OK | 19 | rs429358 (APOE-ε4) | 45411941 | ε4 | non-ε4 | 0.442 | 0.000 | 0.732 | 0.059 | 0.068 | 0.386 |
| OK | 19 | rs3865444 | 51727962 | T | G | 0.304 | 0.020 | 0.626 | -0.049 | 0.076 | 0.519 |
| OK | 21 | rs11700591 | 41507695 | C | T | 0.068 | 0.009 | 0.144 | -0.018 | 0.132 | 0.891 |
| OK | 23 | rs217969 | 118527877 | T | A | 0.256 | 0.002 | 0.188 | 0.044 | 0.065 | 0.495 |

[a]rs Number; [b] Chromosome; [c] Physical position in base pairs, according to dbSNP build 37; [d] Effect allele: allele the effect estimate is reported for; [e] Other allele; [f] Effect estimate (fixed effects meta-analysis) for step 1 meta-analysis; [g] Corresponding standard error; [h] Effect directions in 3 initial studies; [i] Lambda-adjusted p-value (fixed effects meta-analysis), GWAS meta-analysis; [j] minor allele frequency; [k] Effect estimate from regression analysis; [L] p-value of regression analysis; m Effect directions for step 1 (joint direction) and replication analysis; [n] Effect estimate (fixed effects meta-analysis) GWAS meta-analysis plus replication; [o] Corresponding standard error; [p] p-value (fixed effects meta-analysis) GWAS meta-analysis plus replication.

Table 3:

| | Bonn1CSF | Bonn2CSF | ADNI | ADC | AD patients | | Healthy controls | |
|---|---|---|---|---|---|---|---|---|
| | | | | | Bonn1: 438 | Bonn2:641 | Bonn1:1179 | Bonn2:1096 |
| N | 113 | 167 | 83 | 515 | 1079 | | 2275 | |
| Age [years ± S.D.] | 71.6 [±7.4] | 71.8 [±7.7] | 74.8 [±7.5] | 66.4 [±8.8] | 73.3 [±8.8] | | 56.7 [±13.8] | |
| F:M ratio | 1.26 | 1.19 | 1 | 1.05 | 1.7 | | 1.3 | |
| $A\beta_{1\text{-}42}$ [± S.D] | 478.3 [±186.5] | 529.2 [±238.1] | 149.4 [±51.1] | 462.5 [±147.9] | N.A. | N.A. | N.A. | N.A. |
| $pTau_{181}$ [± S.D.] | 87.5 [±36.5] | 88.6 [±45.2] | 39.5 [±20.1] | 88.2 [±38.8] | N.A. | N.A. | N.A. | N.A. |
| Genotype platform | Illumina 610 | Illumina 1M-quad | Illumina 610 | Sequenom | Bonn1: 438 Illumina 610 | Bonn2:641 Illumina 1M-quad | Bonn1:1179 Illumina HumanHap550k | Bonn2:1096 Illumina 1M-quad |

S.D.: Standard deviation; F:M ratio: Female:male ratio; $A\beta_{1\text{-}42}$: amyloid-beta 1-42; $pTau_{181}$: phosphorylated Tau at position 181; AD: Alzheimer's Disease; CSF: Cerebrospinal fluid; ADNI: Alzheimer's Disease Neuroimaging Initiative cohort; ADC: Amsterdam Dementia Cohort

[a] Cerebrospinal fluid biomarker; [b] Final quality control judgement; [c] Chromosome; [d] rs Number; [e] Physical position in base pairs, according to dbSNP build 37; f SNP minor allele; [g] SNP major allele; [h] minor allele frequency; [i] Missing genotype rate; [j] P-value for deviation from Hardy-Weinberg equilibrium; [k] Effect estimate from regression analysis; [l] Corresponding standard error; [m] p-value of regression analysis.

Tab: 4: **Replication results on the ADC sample**

| Phenotype[a] | QC[b] | Chr[c] | rs[d] | bp[e] | minor[f] | major[g] | maf[h] | Missing Rate[i] | pHWE[j] | beta[k] | se[l] | p[m] |
|---|---|---|---|---|---|---|---|---|---|---|---|---|
| | OK | 1 | rs3818361 | 207784968 | T | C | 0.223 | 0.027 | 0.076 | -0.190 | 0.084 | 0.024 |
| | out | 1 | rs10779606 | 213841768 | C | G | 0.443 | 0.050 | $2.53 \times 10^{-38}$ | -0.151 | 0.053 | 0.005 |
| | OK | 2 | rs6753292 | 46002160 | T | C | 0.251 | 0.000 | 0.862 | 0.009 | 0.075 | 0.908 |
| | OK | 2 | rs72804029 | 59273581 | T | C | 0.089 | 0.005 | 0.146 | 0.173 | 0.118 | 0.144 |
| | OK | 2 | rs744373 | 127894615 | C | T | 0.328 | 0.009 | 0.359 | -0.035 | 0.072 | 0.623 |
| | out | 2 | rs11884035 | 169247034 | 0 | T | 0.000 | 0.000 | 1.000 | - | - | 1.000 |
| | OK | 2 | rs34737109 | 172905689 | A | G | 0.383 | 0.000 | 0.878 | -0.080 | 0.066 | 0.231 |
| | OK | 3 | rs62256378 | 67457033 | A | G | 0.061 | 0.000 | 0.052 | 0.608 | 0.126 | $1.9 \times 10^{-6}$ |
| | OK | 3 | rs4407373 | 139255012 | T | G | 0.099 | 0 | 0.472 | -0.045 | 0.111 | 0.683 |
| | OK | 3 | rs2222464 | 165289424 | C | T | 0.274 | 0.000 | 0.599 | -0.023 | 0.073 | 0.757 |
| | OK | 4 | rs388575 | 107973286 | C | T | 0.252 | 0.016 | 0.886 | 0.049 | 0.074 | 0.504 |
| | OK | 4 | rs376496 | 107974207 | G | C | 0.249 | 0.000 | 0.886 | 0.086 | 0.074 | 0.245 |
| $A\beta_{1-42}$ | OK | 6 | rs9349407 | 47453378 | C | G | 0.263 | 0.007 | 0.392 | -0.054 | 0.073 | 0.462 |
| | OK | 6 | rs12527308 | 63061935 | T | C | 0.150 | 0.000 | 0.963 | 0.092 | 0.090 | 0.309 |
| | OK | 6 | rs9360453 | 72154332 | A | G | 0.291 | 0.000 | 0.412 | -0.042 | 0.070 | 0.543 |
| | OK | 6 | rs4896367 | 138807281 | C | T | 0.268 | 0.000 | 0.405 | -0.023 | 0.071 | 0.748 |
| | OK | 6 | rs6911312 | 154761284 | C | A | 0.356 | 0.000 | 0.694 | 0.042 | 0.068 | 0.542 |
| | OK | 6 | rs9397794 | 155575942 | A | G | 0.245 | 0.000 | 0.690 | 0.091 | 0.074 | 0.222 |
| | OK | 7 | rs11767557 | 143109139 | C | T | 0.180 | 0.007 | 0.390 | -0.026 | 0.084 | 0.757 |
| | OK | 8 | rs11136000 | 27464519 | T | C | 0.343 | 0.009 | 0.446 | 0.030 | 0.071 | 0.669 |
| | OK | 9 | rs73402275 | 10369118 | C | T | 0.129 | 0.000 | 0.876 | -0.061 | 0.097 | 0.530 |
| | OK | 10 | rs77131199 | 26798222 | A | G | 0.037 | 0.000 | 0.613 | 0.126 | 0.168 | 0.455 |
| | OK | 10 | rs2258946 | 93624672 | T | C | 0.145 | 0.000 | 0.154 | -0.095 | 0.089 | 0.286 |
| | OK | 11 | rs12418935 | 11863410 | A | G | 0.107 | 0.016 | 0.140 | -0.098 | 0.108 | 0.362 |
| | OK | 11 | rs610932 | 59939307 | A | C | 0.392 | 0.016 | 0.678 | -0.026 | 0.068 | 0.706 |
| | OK | 11 | rs3851179 | 85868640 | A | G | 0.356 | 0.009 | 0.430 | -0.017 | 0.070 | 0.811 |
| | OK | 12 | rs7304039 | 108736352 | A | G | 0.295 | 0.002 | 0.925 | -0.013 | 0.071 | 0.857 |
| | OK | 13 | rs4942417 | 32887389 | C | A | 0.154 | 0.000 | 0.859 | 0.047 | 0.090 | 0.599 |
| | OK | 13 | rs74922937 | 91829564 | A | G | 0.026 | 0.000 | 0.189 | 0.019 | 0.198 | 0.923 |
| | OK | 15 | rs143825603 | 61848856 | C | T | 0.053 | 0.000 | 0.812 | -0.046 | 0.146 | 0.751 |

| | | | | | | | | | | | |
|---|---|---|---|---|---|---|---|---|---|---|---|
| OK | 18 | rs7230126 | 13890199 | C | T | 0.317 | 0.000 | 0.222 | -0.038 | 0.067 | 0.575 |
| OK | 18 | rs3794851 | 74709351 | C | G | 0.046 | 0.000 | 0.960 | -0.018 | 0.153 | 0.908 |
| OK | 19 | rs3764650 | 1046520 | G | T | 0.109 | 0.009 | 0.903 | -0.002 | 0.106 | 0.983 |
| OK | 19 | rs429358 (APOE-ε4) | 45411941 | ε4 | non-ε4 | 0.442 | 0.000 | 0.732 | -0.701 | 0.149 | $1.1 \times 10^{-5}$ |
| OK | 19 | rs3865444 | 51727962 | T | G | 0.304 | 0.020 | 0.626 | 0.053 | 0.072 | 0.460 |
| OK | 21 | rs11700591 | 41507695 | C | T | 0.068 | 0.009 | 0.144 | 0.015 | 0.126 | 0.903 |
| OK | 23 | rs217969 | 118527877 | T | A | 0.256 | 0.002 | 0.188 | -0.084 | 0.061 | 0.164 |
| OK | 1 | rs3818361 | 207784968 | T | C | 0.223 | 0.027 | 0.076 | 0.126 | 0.087 | 0.150 |
| out | 1 | rs10779606 | 213841768 | C | G | 0.443 | 0.050 | $2.53 \times 10^{-38}$ | 0.031 | 0.056 | 0.582 |
| OK | 2 | rs6753292 | 46002160 | T | C | 0.251 | 0.000 | 0.862 | -0.113 | 0.080 | 0.156 |
| OK | 2 | rs72804029 | 59273581 | T | C | 0.089 | 0.005 | 0.146 | 0.044 | 0.126 | 0.725 |
| OK | 2 | rs744373 | 127894615 | C | T | 0.328 | 0.009 | 0.359 | 0.080 | 0.075 | 0.284 |
| out | 2 | rs11884035 | 169247034 | O | T | 0.000 | 0.000 | 1.000 | - | - | 1.000 |
| OK | 2 | rs34737109 | 172905689 | A | A | 0.383 | 0.000 | 0.878 | -0.064 | 0.071 | 0.365 |
| OK | 3 | rs62256378 | 67457033 | A | G | 0.061 | 0.000 | 0.052 | -0.005 | 0.138 | 0.974 |
| OK | 3 | rs4407373 | 139255012 | T | T | 0.099 | 0 | 0.472 | 0.012 | 0.116 | 0.921 |
| OK | 3 | rs2222464 | 165289424 | C | T | 0.274 | 0.000 | 0.599 | -0.038 | 0.078 | 0.628 |
| OK | 4 | rs388575 | 107973286 | C | T | 0.252 | 0.016 | 0.886 | 0.003 | 0.080 | 0.970 |
| OK | 4 | rs376496 | 107974207 | G | C | 0.249 | 0.000 | 0.886 | -0.027 | 0.079 | 0.736 |
| OK | 6 | rs9349407 | 47453378 | C | G | 0.263 | 0.007 | 0.392 | 0.103 | 0.076 | 0.177 |
| OK | 6 | rs12527308 | 63061935 | T | C | 0.150 | 0.000 | 0.963 | -0.060 | 0.096 | 0.533 |
| OK | 6 | rs9360453 | 72154332 | A | G | 0.291 | 0.000 | 0.412 | 0.019 | 0.074 | 0.800 |
| OK | 6 | rs4896367 | 138807281 | C | T | 0.268 | 0.000 | 0.405 | -0.038 | 0.076 | 0.619 |
| OK | 6 | rs6911312 | 154761284 | C | A | 0.356 | 0.000 | 0.694 | -0.010 | 0.073 | 0.895 |
| OK | 6 | rs9397794 | 155575942 | A | G | 0.245 | 0.000 | 0.690 | -0.012 | 0.079 | 0.883 |
| OK | 7 | rs11767557 | 143109139 | C | T | 0.180 | 0.007 | 0.390 | -0.080 | 0.087 | 0.360 |
| OK | 8 | rs11136000 | 27464519 | T | C | 0.343 | 0.009 | 0.446 | -0.062 | 0.074 | 0.401 |
| OK | 9 | rs73402275 | 10369118 | C | G | 0.129 | 0.000 | 0.876 | 0.105 | 0.103 | 0.309 |
| OK | 10 | rs77131199 | 26798222 | A | G | 0.037 | 0.000 | 0.613 | 0.011 | 0.180 | 0.951 |
| OK | 10 | rs2258946 | 93624672 | T | C | 0.145 | 0.000 | 0.154 | 0.213 | 0.094 | 0.024 |
| OK | 11 | rs12418935 | 11863410 | A | G | 0.107 | 0.016 | 0.140 | -0.053 | 0.118 | 0.653 |
| OK | 11 | rs610932 | 59939307 | A | C | 0.392 | 0.016 | 0.678 | -0.103 | 0.071 | 0.150 |
| OK | 11 | rs3851179 | 85868640 | A | G | 0.356 | 0.009 | 0.430 | -0.043 | 0.073 | 0.560 |
| OK | 12 | rs7304039 | 108736352 | A | A | 0.295 | 0.002 | 0.925 | -0.073 | 0.076 | 0.340 |
| OK | 13 | rs4942417 | 32887389 | C | A | 0.154 | 0.000 | 0.859 | -0.077 | 0.095 | 0.419 |
| OK | 13 | rs74922937 | 91829564 | A | G | 0.026 | 0.000 | 0.189 | -0.171 | 0.211 | 0.417 |
| OK | 15 | rs143825603 | 61848856 | C | T | 0.053 | 0.000 | 0.812 | -0.027 | 0.155 | 0.862 |

pTau181

| | | | | | | | | | | | |
|---|---|---|---|---|---|---|---|---|---|---|---|
| OK | 18 | rs7230126 | 13890199 | C | T | 0.317 | 0.000 | 0.222 | 0.031 | 0.072 | 0.670 |
| OK | 18 | rs3794851 | 74709351 | C | G | 0.046 | 0.000 | 0.960 | -0.014 | 0.163 | 0.934 |
| OK | 19 | rs3764650 | 1046520 | G | T | 0.109 | 0.009 | 0.903 | -0.090 | 0.110 | 0.416 |
| OK | 19 | rs429358 (APOE-ε4) | 45411941 | ε4 | non-ε4 | 0.442 | 0.000 | 0.732 | 0.059 | 0.068 | 0.386 |
| OK | 19 | rs3865444 | 51727962 | T | G | 0.304 | 0.020 | 0.626 | -0.049 | 0.076 | 0.519 |
| OK | 21 | rs11700591 | 41507695 | C | T | 0.068 | 0.009 | 0.144 | -0.018 | 0.132 | 0.891 |
| OK | 23 | rs217969 | 118527877 | T | A | 0.256 | 0.002 | 0.188 | 0.044 | 0.065 | 0.495 |

[a] Cerebrospinal fluid biomarker; [b] Final quality control judgement; [c] Chromosome; [d] rs Number; [e] Physical position in base pairs, according to dbSNP build 37; f SNP minor allele; [g] SNP major allele; [h] minor allele frequency; [i] Missing genotype rate; [j] P-value for deviation from Hardy-Weinberg equilibrium; [k] Effect estimate from regression analysis; [l] Corresponding standard error; [m] p-value of regression analysis.

## Table 5. Imputed vs Genotyped

| Sample[a] | Chr[b] | rs[c] | EA[d] | OA[e] | Imputed Data | | | | Direct Genotyping | | | |
|---|---|---|---|---|---|---|---|---|---|---|---|---|
| | | | | | maf[f] | beta[g] | se[h] | p[i] | maf[f] | beta[g] | se[h] | p[i] |
| Bonn1CSF | 3 | rs62256378 | A | G | 0.049 | 2.13 | 0.46 | $2.5 \times 10^{-5}$ | 0.049 | 1.57 | 0.45 | $1.6 \times 10^{-4}$ |
| Bonn2CSF | 3 | rs62256378 | A | G | 0.066 | 0.82 | 0.24 | $6.0 \times 10^{-4}$ | 0.063 | 0.79 | 0.21 | $2.4 \times 10^{-4}$ |
| GWAS (incl. ADNI) | 3 | rs62256378 | A | G | 0.058 | 0.89 | 0.17 | $3.0 \times 10^{-7}$ | 0.057 | 0.80 | 0.16 | $4.7 \times 10^{-7}$ |
| GWAS + Replication | 3 | rs62256378 | A | G | 0.060 | 0.71 | 0.10 | $2.5 \times 10^{-12}$ | 0.059 | 0.68 | 0.10 | $5.1 \times 10^{-12}$ |

[a] Sample analyzed; [b] Chromosomal location; [c] rs Number; [d] Effect allele: allele the effect estimate is reported for; [e] Other allele; [f] Minor allele frequency; [g] Effect estimate; [h] Its standard error (fixed effects meta-analysis); [i] p-value.

Table 6: Effect of know susceptibility Alzheimer's disease genes on cerebrospinal fluid levels of Aβ$_{1-42}$ and pTau$_{181}$

| Reference: Case-Control Analysis (GWAS catalog) | | | | | | | CSF-Analysis, covariates*: APOE, age, sex | | | | | |
| --- | --- | --- | --- | --- | --- | --- | --- | --- | --- | --- | --- | --- |
| | | | | | | | Aβ$_{1-42}$ | | | pTau$_{181}$ | | |
| Gene | Reported SNP | Risk allele frequency | OR | Minor Allele | Major allele | Risk allele | effect of susceptibility allele | consistency[a] | P | effect of susceptibility allele | consistency[a] | P |
| APOE | rs429358 (APOE-ε4) | 0.09 | 3-4 | C | T | C | -0.404 | yes | 4.3x10$^{-17}$ | 0.113 | yes | 0.024 |
| ABCA7 | rs3764650 | 0.1 | 1.23 | G | T | G | -0.038 | yes | 0.649 | 0.004 | yes | 0.96 |
| CR1 | rs6656401 | 0.19 | 1.21 | A | G | A | -0.104 | yes | 0.094 | 0.121 | yes | 0.06 |
| PICALM | rs3851179 | 0.63 | 1.16 | T | C | C | 0.006 | no | 0.909 | 0.031 | yes | 0.581 |
| CLU | rs11136000 | 0.6 | 1.16 | T | C | C | 0 | no | 0.995 | -0.007 | no | 0.909 |
| BIN1 | rs744373 | 0.29 | 1.15 | G | A | G | 0.007 | no | 0.895 | 0.078 | yes | 0.17 |
| EPHA1 | rs11767557 | 0.79 | 1.11 | C | T | T | 0.003 | no | 0.964 | 0.015 | yes | 0.818 |
| CD2AP | rs9349407 | 0.29 | 1.11 | C | G | C | -0.086 | yes | 0.111 | 0.119 | yes | 0.031 |
| CD33 | rs3865444 | 0.69 | 1.1 | A | C | C | 0.003 | no | 0.959 | 0.005 | yes | 0.936 |
| MS4A | rs610932 | 0.58 | 1.1 | T | G | G | 0.024 | no | 0.652 | 0.009 | yes | 0.871 |

GWAS: Genome-wide association study; CSF: Cerebrospinal fluid; OR: Odd ratio.

[a] Consistency in the allele direction between the OR observed in the present study and the OR reported in the GWAS-catalog.

# Table 7: **Previous CSF GWASs results**

| Study[a] | rs[b] | Chr[c] | bp[d] | EA[e] | OA[f] | A$\beta_{42}$ | | | | pTau | | | |
|---|---|---|---|---|---|---|---|---|---|---|---|---|---|
| | | | | | | dir[g] | beta[h] | se[i] | p[j] | dir[g] | beta[h] | se[i] | p[j] |
| | rs11206801 | 1 | 56850686 | C | A | --+ | -0.122 | 0.132 | 0.354 | ++- | 0.027 | 0.135 | 0.842 |
| | rs4431886 | 1 | 56852267 | T | C | --+ | -0.122 | 0.132 | 0.354 | ++- | 0.027 | 0.135 | 0.842 |
| | rs10911736 | 1 | 185422604 | G | A | ++- | 0.016 | 0.080 | 0.846 | --+ | 0.013 | 0.081 | 0.872 |
| | rs893769 | 2 | 119840084 | A | C | -+- | -0.020 | 0.075 | 0.791 | ++- | 0.001 | 0.077 | 0.989 |
| | rs17189298 | 2 | 119845317 | G | A | -+- | -0.019 | 0.079 | 0.811 | +-- | 0.025 | 0.081 | 0.760 |
| | rs895401 | 2 | 119867708 | G | A | +-+ | 0.071 | 0.080 | 0.373 | --- | -0.051 | 0.082 | 0.533 |
| | rs2074955 | 2 | 158977794 | A | G | +++ | 0.090 | 0.109 | 0.411 | -++ | -0.019 | 0.111 | 0.863 |
| | rs7558386 | 2 | 227562139 | A | G | ++- | 0.033 | 0.082 | 0.689 | --- | -0.121 | 0.083 | 0.145 |
| | rs7631605 | 3 | 37234589 | C | T | -+- | -0.113 | 0.082 | 0.167 | --+ | -0.104 | 0.084 | 0.214 |
| | rs6850199 | 4 | 49000850 | A | G | --- | -0.049 | 0.087 | 0.571 | -++ | 0.092 | 0.089 | 0.302 |
| Han et al.[k] | rs2768975 | 4 | 49029901 | C | T | +-+ | -0.052 | 0.087 | 0.552 | -++ | 0.122 | 0.089 | 0.171 |
| | rs12643654 | 4 | 96159817 | A | G | --- | -0.128 | 0.126 | 0.309 | ++- | 0.119 | 0.128 | 0.356 |
| | rs12203791 | 6 | 67721488 | T | C | ++- | 0.043 | 0.113 | 0.704 | +++ | 0.094 | 0.116 | 0.418 |
| | rs1727638 | 6 | 72139572 | G | A | -+- | -0.189 | 0.081 | 0.019 | +-- | 0.025 | 0.082 | 0.761 |
| | rs121724 | 7 | 29241445 | G | T | --- | -0.154 | 0.078 | 0.047 | --+ | 0.053 | 0.079 | 0.502 |
| | rs12534221 | 7 | 131287990 | C | A | +++ | 0.131 | 0.130 | 0.316 | +-- | 0.035 | 0.131 | 0.787 |
| | rs7842088 | 8 | 17585129 | G | A | -+- | -0.149 | 0.080 | 0.062 | --- | -0.042 | 0.082 | 0.606 |
| | rs2928826 | 8 | 109614094 | C | T | --+ | -0.035 | 0.095 | 0.713 | +-+ | 0.177 | 0.099 | 0.073 |
| | rs2935776 | 8 | 109629903 | A | G | --+ | -0.035 | 0.095 | 0.716 | +-+ | 0.178 | 0.099 | 0.071 |
| | rs11015839 | 10 | 19002887 | G | A | --+ | -0.060 | 0.084 | 0.473 | --+ | -0.036 | 0.087 | 0.676 |
| | rs11015860 | 10 | 19005039 | T | G | --+ | -0.061 | 0.084 | 0.472 | --+ | -0.036 | 0.087 | 0.678 |

EP 3 077 533 B1

| | | | | | | | | | | | | |
|---|---|---|---|---|---|---|---|---|---|---|---|---|
| rs11015939 | 10 | 19017942 | G | A | --+ | -0.086 | 0.093 | 0.351 | --- | -0.149 | 0.096 | 0.120 |
| rs10763625 | 10 | 19034951 | C | T | --+ | -0.085 | 0.092 | 0.356 | --- | -0.145 | 0.095 | 0.128 |
| rs2493624 | 10 | 19062256 | A | C | --+ | -0.057 | 0.084 | 0.496 | --+ | -0.022 | 0.086 | 0.798 |
| rs2495832 | 10 | 19074461 | A | C | --+ | -0.106 | 0.092 | 0.252 | --- | -0.130 | 0.095 | 0.174 |
| rs2986971 | 10 | 30096628 | A | G | -++ | 0.020 | 0.075 | 0.794 | -+- | -0.028 | 0.077 | 0.715 |
| rs10784496 | 12 | 66160971 | A | G | +-+ | 0.058 | 0.078 | 0.459 | +-- | -0.017 | 0.079 | 0.825 |
| rs11069178 | 12 | 119367827 | A | C | ++- | -0.064 | 0.094 | 0.495 | +-+ | 0.014 | 0.096 | 0.885 |
| rs1997111 | 12 | 119387918 | A | C | --+ | 0.064 | 0.083 | 0.439 | ++- | 0.000 | 0.086 | 0.999 |
| rs2899472 | 15 | 51516055 | C | A | ++- | -0.020 | 0.086 | 0.817 | -+- | -0.002 | 0.088 | 0.979 |
| rs1021599 | 18 | 30215056 | G | A | +-- | -0.038 | 0.087 | 0.659 | ++- | 0.018 | 0.089 | 0.837 |
| rs1445093 | 18 | 49770823 | G | A | +-+ | 0.044 | 0.083 | 0.595 | -+- | -0.165 | 0.084 | 0.049 |
| rs1943816 | 18 | 71058622 | A | G | ?++ | 0.384 | 0.186 | 0.039 | -+- | -0.296 | 0.141 | 0.036 |
| rs2075650 | 19 | 45395619 | A | G | ++- | 0.077 | 0.128 | 0.550 | -+- | -0.122 | 0.131 | 0.350 |
| rs4925189 | 20 | 59947044 | G | A | -++ | 0.090 | 0.095 | 0.343 | ++- | 0.158 | 0.099 | 0.110 |
| rs1022442 | 21 | 22355846 | G | A | +-- | -0.054 | 0.080 | 0.502 | -++ | -0.002 | 0.083 | 0.978 |
| rs239713 | 21 | 28696476 | T | C | +++ | 0.143 | 0.089 | 0.110 | --+ | -0.022 | 0.092 | 0.810 |
| rs5998432 | 22 | 32745916 | C | T | --+ | -0.101 | 0.125 | 0.419 | ++- | 0.034 | 0.128 | 0.791 |
| rs10191411 | 2 | 149387968 | T | C | -+- | -0.092 | 0.085 | 0.276 | --+ | 0.072 | 0.086 | 0.406 |
| rs6759083 | 2 | 149438211 | G | A | +-- | 0.104 | 0.090 | 0.244 | +-- | -0.048 | 0.093 | 0.610 |
| rs7564433 | 2 | 149446638 | G | T | -++ | -0.096 | 0.089 | 0.284 | -++ | 0.052 | 0.093 | 0.572 |
| rs10197267 | 2 | 149486909 | C | T | -++ | -0.102 | 0.089 | 0.256 | -++ | 0.045 | 0.093 | 0.629 |
| Kim et al.[l]  rs11724427 | 4 | 89103172 | A | G | +++ | 0.123 | 0.096 | 0.200 | --- | -0.140 | 0.098 | 0.153 |
| rs2583693 | 8 | 20665979 | A | C | --- | -0.215 | 0.081 | 0.008 | +++ | 0.139 | 0.084 | 0.098 |
| rs10974334 | 9 | 4102339 | G | T | -+- | -0.011 | 0.092 | 0.900 | +-+ | 0.096 | 0.094 | 0.308 |
| rs2208558 | 9 | 4103345 | G | A | -+- | -0.015 | 0.095 | 0.871 | +-+ | 0.109 | 0.097 | 0.262 |
| rs17157052 | 10 | 1863551 | A | G | +-+ | 0.187 | 0.092 | 0.042 | -+- | -0.183 | 0.096 | 0.058 |

| | rs Number | Chr | Position | Effect allele | Other allele | Dir | Effect | SE | p-value | Dir | Effect | SE | p-value |
|---|---|---|---|---|---|---|---|---|---|---|---|---|---|
| | rs7123160 | 11 | 80118588 | A | G | +-- | -0.042 | 0.088 | 0.630 | ++- | -0.047 | 0.089 | 0.596 |
| | rs2448281 | 11 | 80125592 | C | A | -++ | 0.007 | 0.086 | 0.934 | --+ | 0.079 | 0.087 | 0.363 |
| | rs1408888 | 13 | 72428647 | T | G | +-- | 0.013 | 0.095 | 0.894 | -++ | -0.161 | 0.097 | 0.096 |
| | rs11628929 | 14 | 24844054 | A | G | +++ | 0.102 | 0.084 | 0.224 | +-+ | 0.051 | 0.086 | 0.549 |
| | rs6573766 | 14 | 24852046 | T | G | --- | -0.188 | 0.100 | 0.061 | ++- | 0.072 | 0.102 | 0.485 |
| | rs3936367 | 16 | 82549285 | C | A | -+- | -0.116 | 0.081 | 0.155 | --+ | 0.082 | 0.083 | 0.319 |
| | rs3813089 | 18 | 48346024 | G | T | +-- | 0.007 | 0.095 | 0.942 | -++ | 0.091 | 0.097 | 0.349 |
| | rs1885082 | 20 | 17593984 | G | A | ?-+ | -0.066 | 0.124 | 0.593 | ?-+ | 0.044 | 0.125 | 0.725 |
| | rs6089510 | 20 | 60377424 | C | A | ++- | 0.028 | 0.085 | 0.738 | -+- | -0.003 | 0.085 | 0.973 |
| | rs4444 | 22 | 31205334 | T | C | --- | -0.105 | 0.077 | 0.176 | +-+ | 0.059 | 0.079 | 0.451 |
| | rs136245 | 22 | 31208544 | G | A | --- | -0.103 | 0.077 | 0.181 | +-+ | 0.059 | 0.079 | 0.450 |
| | rs136243 | 22 | 31209135 | C | T | +-- | -0.100 | 0.078 | 0.197 | +-+ | 0.055 | 0.079 | 0.484 |
| | rs2269657 | 22 | 42264269 | G | T | +-- | 0.023 | 0.091 | 0.801 | -+- | -0.080 | 0.094 | 0.392 |
| | rs1316356 | 3 | 190657163 | T | C | N.A. | N.A. | N.A. | N.A. | --- | -0.268 | 0.079 | $7.22 \times 10^{-4}$ |
| | rs9877502 | 3 | 190669518 | G | A | N.A. | N.A. | N.A. | N.A. | --- | -0.262 | 0.079 | $9.21 \times 10^{-4}$ |
| | rs6922617 | 6 | 41336101 | G | A | N.A. | N.A. | N.A. | N.A. | +++ | 0.287 | 0.162 | 0.076 |
| | rs11966476 | 6 | 41340572 | G | A | N.A. | N.A. | N.A. | N.A. | +++ | 0.327 | 0.159 | 0.041 |
| Cruchaga et al.[m] | rs622536 | 9 | 3927704 | T | C | N.A. | N.A. | N.A. | N.A. | +-- | -0.124 | 0.114 | 0.280 |
| | rs59860681 | 9 | 3927804 | C | T | N.A. | N.A. | N.A. | N.A. | +-- | -0.123 | 0.114 | 0.281 |
| | rs623295 | 9 | 3927841 | A | C | N.A. | N.A. | N.A. | N.A. | +-- | -0.124 | 0.114 | 0.277 |
| | rs624290 | 9 | 3928115 | C | T | N.A. | N.A. | N.A. | N.A. | +-- | -0.125 | 0.114 | 0.275 |
| | rs514716 | 9 | 3929424 | C | T | N.A. | N.A. | N.A. | N.A. | +-- | -0.155 | 0.107 | 0.148 |

[a] Original publication where result were taken from; [b] rs Number; [c] Chromosome; [d] Base pair position according to dbSNP build 37; [e] Effect allele: allele the effect estimate is reported for; [f] Other allele; [g] Direction of effect in the studies, Bonn1CSF, Bonn2CSF, and ADNI; [h] Effect estimate (fixed effects meta-analysis); [i] Its standard error (fixed effects meta-analysis). [j] p-value (fixed effects meta-analysis).

[k]Han, M.R., Schellenberg, G.D. & Wang, L.S. Genome-wide association reveals genetic effects on human Abeta42 and tau protein levels in cerebrospinal fluids: a case control study. BMC Neurol 10, 90 (2010); [l] Kim, S. et al. Genome-wide association study of CSF biomarkers Abeta1-42, t-tau, and p-tau181p in the ADNI cohort. Neurology 76, 69-79 (2011); [m] Cruchaga, C. et al. GWAS of cerebrospinal fluid tau levels identifies risk variants for Alzheimer's disease. Neuron 78, 256-68 (2013).

Table 8: Analysis of interaction between $A\beta_{1-42}$ levels, rs492358, and rs62256378.

| Alleles SNP1 | | | Alleles SNP2 | | | Bonn1 CSF | | | Bonn2CSF | | | ADNI | | | ADC | | | Meta-Analysis | | |
|---|---|---|---|---|---|---|---|---|---|---|---|---|---|---|---|---|---|---|---|---|
| SNP1 | minor | major | SNP2 | minor | Major | beta[a] | se[b] | p[c] | beta[a] | se[b] | p[c] | beta[a] | se[b] | p[c] | beta[a] | se[b] | p[c] | beta[a] | se[b] | p[c] |
| rs62256378 | A | G | rs429358 | $\varepsilon 4$ | $\varepsilon 4(-)$ | -1.32 | 0.58 | 0.02 | -0.55 | 0.31 | 0.08 | -0.09 | 0.40 | 0.82 | -0.56 | 0.18 | $2.0 \times 10^{-3}$ | -0.55 | 0.14 | $9.5 \times 10^{-5}$ |

[a] Effect estimate for allelic interaction term $x_1 x_2$; [b] Standard error of effect estimate; [c] p-value.

CSF: Cerebrospinal fluid; ADNI: Alzheimer's Disease Neuroimaging Initiative cohort; ADC: Amsterdam Dementia Cohort

Table 9: Association results of $A\beta_{1\text{-}42}$ and rs62256378 by sample and APOE-strata.

| Stratum[a] | rs[b] | Chr[c] | bp[d] | minor[e] | major[f] | Bonn1CSF | | | Bonn2CSF | | | ADNI | | | ADC | | | Meta-Analysis | | |
|---|---|---|---|---|---|---|---|---|---|---|---|---|---|---|---|---|---|---|---|---|
| | | | | | | beta[g] | se[h] | P[i] | beta[g] | se[h] | P[i] | beta[g] | se[h] | P[i] | beta[g] | se[h] | P[i] | beta[g] | se[h] | P[i] |
| ALL | rs429358 (e4) | 19 | 45411941 | $\varepsilon4$ | $\varepsilon4(-)$ | -0.51 | 0.11 | $6.1 \times 10^{-6}$ | -0.33 | 0.07 | $5.9 \times 10^{-7}$ | -0.39 | 0.15 | 0.01 | -0.70 | 0.15 | $11 \times 10^{-5}$ | -0.40 | 0.05 | $4.3 \times 10^{-17}$ |
| ALL | rs62256378 | 3 | 67457033 | A | G | 2.13 | 0.46 | $2.5 \times 10^{-5}$ | 0.82 | 0.24 | $60 \times 10^{-4}$ | 0.50 | 0.29 | 0.09 | 0.61 | 0.13 | $1.9 \times 10^{-6}$ | 0.71 | 0.10 | $25 \times 10^{-12}$ |
| APOE-$\varepsilon4$ noncarriers | rs62256378 | 3 | 67457033 | A | G | 1.68 | 0.57 | $4.8 \times 10^{-3}$ | 0.96 | 0.36 | $8.8 \times 10^{-3}$ | 0.66 | 0.43 | 0.14 | 0.94 | 0.24 | $11 \times 10^{-4}$ | 0.97 | 0.17 | $15 \times 10^{-8}$ |
| APOE-$\varepsilon4$ hetero-zygous | rs62256378 | 3 | 67457033 | A | G | 0.24 | 0.30 | 0.44 | 0.45 | 0.22 | 0.04 | 0.48 | 0.49 | 0.33 | 0.32 | 0.15 | 0.03 | 0.35 | 0.11 | $15 \times 10^{-3}$ |
| APOE-$\varepsilon4$ ho-mozygous | rs62256378 | 3 | 67457033 | A | G | - | - | - | -0.13 | 0.33 | 0.71 | -0.10 | 0.76 | 0.90 | 0.01 | 0.25 | 0.96 | -0.04 | 0.19 | 0.83 |

a Analysis of all patients and within strata defined by APOE-$\varepsilon4$ carrier status; b rs Number; c Chromosome; d Physical position in base pairs, according to dbSNP build 37; e minor allele; f major allele g Effect estimate from regression analysis. Represents increase in $A\beta1\text{-}42$ levels in standard deviations per minor allele; h Standard error of effect estimate; i p-value; ADNI: Alzheimer's Disease Neuroimaging Initiative cohort; ADC: Amsterdam Dementia Cohort

Table 10: Effect of rs62256378 on mini-mental states examination decline by sample and APOE-strata.

| | | Allele | | Bonn1 | | | Bonn2 | | | ADNI | | | ADC | | | **Meta-analysis** | | |
|---|---|---|---|---|---|---|---|---|---|---|---|---|---|---|---|---|---|---|
| Stratum[a] | rs[b] | minor | major | beta[c] | se[d] | P[e] | beta[c] | se[d] | P[e] | beta[c] | se[d] | P[e] | beta[c] | se[d] | P[e] | beta[c] | se[d] | P[e] |
| ALL | rs62256378 | A | G | 0.57 | 0.32 | 0.08 | 0.66 | 0.64 | 0.15 | 0.24 | 0.21 | 0.11 | 0.28 | 0.16 | 0.08 | 0.32 | 0.12 | $3.1 \times 10^{-3}$ |
| APOE-$\varepsilon$4 noncarriers | rs62256378 | A | G | 0.57 | 0.46 | 0.23 | 0.26 | 0.93 | 0.39 | 0.01 | 0.23 | 0.50 | 0.61 | 0.25 | 0.02 | 0.32 | 0.16 | 0.021 |
| APOE-$\varepsilon$4 heterozygous | rs62256378 | A | G | 0.38 | 0.56 | 0.25 | 1.07 | 1.03 | 0.16 | 0.43 | 0.42 | 0.15 | 0.18 | 0.28 | 0.29 | 0.31 | 0.21 | 0.072 |
| APOE-$\varepsilon$4 homozygous | rs62256378 | A | G | -0.36 | 1.10 | 1.00 | 1.38 | 2.15 | 0.25 | 1.23 | 0.92 | 0.14 | 0.29 | 0.45 | 0.26 | 0.40 | 0.37 | 0.141 |

[a] Analysis of all patients and within strata defined by APOE-e4 carrier status; [b] Physical position in base pairs, according to dbSNP build 37; [c] Effect obtained from regression analysis. The beta represents attenuation of memory decline in standard deviations of mini-mental states examination decline; [d] Standard error of effect estimate; [e] One-sided p-value for rs62256378-A as protective allele

Table 11. Explained variance of Aβ$_{1\text{-}42}$

| | All samples | AD patients | | | |
| --- | --- | --- | --- | --- | --- |
| | | Bonn1CSF | Bonn2CSF | ADNI | ADC |
| rs62256378 | 6.06% | 6.90% | 7.52% | 5.20% | 3.40% |
| rs62256378 / APOE-ε4 noncarrier | 10.69% | 14.86% | 10.15% | 6.30% | 11.60% |
| rs62256378 / Heterozygous APOE-ε4 | 2.98% | 0.82%* | 6.30% | 8.01%* | 1.74% |
| rs62256378 / Homozygous APOE-ε4 | 0.21%* | ** | 0.02%* | 2.3%* | 0.7%* |
| APOE | 7.70% | 5.86% | 12.43% | 16.86% | 6.00% |

*Non-significant groups.
** Too few observations
ADNI: Alzheimer's Disease Neuroimaging Initiative cohort; ADC: Amsterdam Dementia Cohort

## Claims

1. A method for establishing a clinical prognosis of a patient suffering from a disease or condition associated with the formation of aggregates of $A\beta_{1-42}$ comprising the steps of

   a) detecting whether an adenine or another nucleotide, preferably guanine is present at single nucleotide polymorphism (SNP) rs62256378 and/or whether a protective allele or another allele is present at at least one SNP in linkage disequilibrium with rs62256378;
   b) establishing a clinical prognosis based on the type of nucleotide present at the site of the SNP, wherein

   (i) the presence of an adenine at rs62256378 and/or the presence of a protective allele at at least one SNP in linkage disequilibrium with rs62256378 is indicative of a good prognosis or
   (ii) the presence of another nucleotide than adenine at rs62256378 and/or the presence of a another allele at at least one SNP in linkage disequilibrium with rs62256378 is indicative of a bad prognosis,

   wherein the at least one SNP in linkage disequilibrium with rs62256378 is selected from the group consisting ofrs74727963, rs78609144, rs79963093, rs80028595, rs77072359, rs74680427, rs111796700, rs115298081, rs72626930, rs76856038, rs77836520, rs116341583, rs114506918 and rs745742418, and
   wherein the SNP in linkage disequilibrium with rs62256378 displays an $r^2$ of at least 0.5.

2. The method of claim 1, wherein the patient does not carry a gene for isoform 4 of apolipoprotein E or is heterozygous for said gene.

3. A suitable medicament for use in the treatment of a disease or condition associated with the formation of aggregates of $A\beta_{1-42}$ in patients having another nucleotide than adenine at rs62256378 and/or another allele at at least one SNP in linkage disequilibrium with rs62256378 as determined by the method of any one of claims 1 or 2, wherein the medicament is selected from the group consisting of donepezil, galantamine, memantine, rivastigmine, tacrine, vitamin E, citalopram, fluoxetine, paroxeine, sertraline, trazodone, lorazepam, oxazepam, aripiprazole, clozapine, haloperidol, olanzapine, quetiapine, risperidone, and ziprasidone.

4. Use of a kit for establishing a clinical prognosis of a patient suffering from a disease or condition associated with the formation of aggregates of $A\beta_{1-42}$, which comprises at least one means for detecting the presence of adenine or guanine at SNP rs62256378, comprising an oligonucleotide comprising at least 8 bases of SEQ ID NO: 1 or a complement thereof, wherein said at least 8 bases comprise position 27.

5. A method for screening compounds to identify therapeutic candidates for the modulation of a disease or condition associated with the formation of aggregates of $A\beta_{1-42}$ comprising the steps of

   a) contacting (i) a brain, blood, or liver cell which is capable of expressing the GTP-specific beta subunit of the succinyl-CoA ligase (SUCLG2) *in vitro* or (ii) a cell which expresses SUCLG2 *in vitro* or (iii) a solution comprising SUCLG2 with a test compound or (iv) administering the test compound to an experimental animal, wherein the animal is a non-human animal;
   b) detecting in case of (i) whether said test compound increases the expression level or the activity of SUCLG2 *in vitro,* in case of (ii) whether said test compound increases the expression level or the activity of SUCLG2 *in vitro,* in case of (iii) whether said test compound increases the activity of SUCLG2, in case (iv) whether said test compound increases the expression level or activity of SUCLG2, wherein an increase of the expression level of activity indicates that said test compound is a candidate compound for treating the disease or condition *in vivo.*

6. The method of claim 5, wherein the brain cell is a microglial cell.

7. The method of claim 5 or 6, wherein the cell does not carry a gene for isoform 4 of apolipoprotein E or is heterozygous for said gene.

8. The method of any one of claims 5 to 7, wherein (i) the cell is further capable of expressing the alpha subunit of SUCL (SUCLG1) or expresses SUCLG1 or (ii) the solution further comprises SUCLG1.

9. The method of any one of claims 5 to 8, wherein the activity of SUCLG2 is the catalysis of the reaction between succinate, GTP and Coenzyme A to generate succinyl-CoA and GDP.

10. The method of claims 5 to 9, wherein the test compound is a small molecule, a peptide or an agonistic antibody.

**Patentansprüche**

1. Verfahren zum Feststellen einer klinischen Prognose eines Patienten, der an einer Krankheit oder einem Zustand leidet, die mit der Bildung von Aggregaten von $A\beta_{1-42}$ assoziiert sind, umfassend die Schritte:

   a) Nachweisen, ob ein Adenin oder ein anderes Nukleotid, vorzugsweise Guanin, am einzelnen Nukleotidpolymorphismus (SNP) rs62256378 vorliegt und/oder ob ein schützendes Allel oder ein anderes Allel an wenigstens einem SNP in Kopplungsungleichgewicht mit rs62256378 vorliegt;
   b) Feststellen einer klinischen Prognose basierend auf dem Nukleotidtyp, der an der Stelle des SNP vorliegt, wobei

   (i) das Vorliegen eines Adenins an rs62256378 und/oder das Vorliegen eines schützenden Allels an wenigstens einem SNP im Kopplungsungleichgewicht mit rs62256378 auf eine gute Prognose hinweist oder
   (ii) das Vorliegen eines anderen Nukleotids als Adenin an rs62256378 und/oder das Vorliegen eines anderen Allels an wenigstens einem SNP im Kopplungsungleichgewicht mit rs62256378 auf eine schlechte Prognose hinweist,

   wobei der wenigstens eine SNP im Kopplungsungleichgewicht mit rs62256378 ausgewählt ist aus der Gruppe bestehend aus rs74727963, rs78609144, rs79963093, rs80028595, rs77072359, rs74680427, rs111796700, rs115298081, rs72626930, rs76856038, rs77836520, rs116341583, rs114506918 und rs745742418, und wobei der SNP im Kopplungsungleichgewicht mit rs62256378 ein $r^2$ von wenigstens 0,5 aufweist.

2. Verfahren nach Anspruch 1, wobei der Patient kein Gen für die Isoform 4 von Apolipoprotein E trägt oder für dieses Gen heterozygot ist.

3. Geeignetes Medikament zur Verwendung bei der Behandlung einer Krankheit oder eines Zustands, die mit der Bildung von Aggregaten von $A\beta_{1-42}$ bei Patienten assoziiert sind, die ein anderes Nukleotid als Adenin an rs62256378 und/oder ein anderes Allel an wenigstens einem SNP in Kopplungsungleichgewicht mit rs62256378 aufweisen, wie durch das Verfahren nach einem der Ansprüche 1 oder 2 bestimmt,
wobei das Medikament ausgewählt ist aus der Gruppe bestehend aus Donepezil, Galantamin, Memantin, Rivastigmin, Tacrin, Vitamin E, Citalopram, Fluoxetin, Paroxein, Sertralin, Trazodon, Lorazepam, Oxazepam, Aripiprazol, Clozapin, Haloperidol, Olanzapin, Caipan, Ziprasidon.

4. Verwendung eines Kits zum Feststellen einer klinischen Prognose eines Patienten, der an einer Krankheit oder einem Zustand leidet, die mit der Bildung von Aggregaten von $A\beta_{1-42}$ assoziiert sind, das wenigstens ein Mittel zum Nachweisen des Vorliegens von Adenin oder Guanin bei SNP rs62256378 umfasst, umfassend ein Oligonukleotid, das wenigstens 8 Basen der SEQ ID NO: 1 oder einem Komplement davon umfasst, wobei die wenigstens 8 Basen Position 27 umfassen.

5. Verfahren zum Screenen von Verbindungen zum Identifizieren therapeutischer Kandidaten für die Modulation einer Krankheit oder eines Zustands, die mit der Bildung von Aggregaten von $A\beta_{1-42}$ assoziiert sind, umfassend die Schritte:

   a) Kontaktieren (i) einer Gehirn-, Blut- oder Leberzelle, die in der Lage sind, die GTP-spezifische beta-Untereinheit der Succinyl-CoA-Ligase (SUCLG2) *in vitro* exprimieren, oder (ii) einer Zelle, die SUCLG2 *in vitro* exprimiert, oder (iii) einer Lösung, die SUCLG2 umfasst, mit einer Testverbindung oder (iv) Verabreichen der Testverbindung an ein Versuchstier, wobei das Tier ein nicht-menschliches Tier ist;
   b) Nachweisen im Fall von (i), ob die Testverbindung das Expressionsniveau oder die Aktivität von SUCLG2 *in vitro* erhöht, im Fall von (ii), ob die Testverbindung das Expressionsniveau oder die Aktivität von SUCLG2 *in vitro* erhöht, im Fall von (iii), ob die Testverbindung die Aktivität von SUCLG2 erhöht, in dem Fall von (iv), ob die Testverbindung das Expressionsniveau oder die Aktivität von SUCLG2 erhöht, wobei ein Anstieg des Expressionsniveaus der Aktivität anzeigt, dass die Testverbindung eine Kandidatenverbindung ZUR Behandlung der Krankheit oder des Zustands *in vivo* ist.

6. Verfahren nach Anspruch 5, wobei die Gehirnzelle eine Mikrogliazelle ist.

7. Verfahren nach Anspruch 5 oder 6, wobei die Zelle kein Gen für die Isoform 4 von Apolipoprotein E trägt oder für dieses Gen heterozygot ist.

8. Verfahren nach einem der Ansprüche 5 bis 7, wobei (i) die Zelle ferner in der Lage ist, die alpha-Untereinheit von SUCL (SUCLG1) oder SUCLG1 zu exprimieren oder (ii) die Lösung ferner SUCLG1 umfasst.

9. Verfahren nach einem der Ansprüche 5 bis 8, wobei die Aktivität von SUCLG2 die Katalyse der Reaktion zwischen Succinat, GTP und Coenzym A ist, um Succinyl-CoA und BIP zu erzeugen.

10. Verfahren nach den Ansprüchen 5 bis 9, wobei die Testverbindung ein kleines Molekül, ein Peptid oder ein agonistischer Antikörper ist.


**Revendications**

1. Procédé pour établir le pronostic clinique d'un patient souffrant d'une maladie ou d'un état associé(e) à la formation d'agrégats d'$A\beta_{1-42}$, lequel procédé comporte les étapes suivantes :

   a) déterminer si une adénine ou un autre nucléotide, de préférence une guanine, est présent au niveau du SNP (polymorphisme à un seul nucléotide) rs62256378 et/ou si un allèle protecteur ou un autre allèle est présent en au moins un SNP en déséquilibre de liaison avec rs62256378 ;
   b) et établir le pronostic clinique en se basant sur le type du nucléotide présent au site du SNP, étant entendu

      i) que la présence d'une adénine en rs62256378 et/ou la présence d'un allèle protecteur en au moins un SNP en déséquilibre de liaison avec rs62256378 est ou sont l'indice d'un pronostic favorable,
      ii) et que la présence d'un nucléotide autre qu'une adénine en rs62256378 et/ou la présence d'un autre allèle en au moins un SNP en déséquilibre de liaison avec rs62256378 est ou sont l'indice d'un pronostic défavorable,

et étant entendu que le SNP, au nombre d'au moins un, en déséquilibre de liaison avec rs62256378 est choisi dans l'ensemble constitué par les suivants : rs74727963, rs78609144, rs79963093, rs80028595, rs77072359, rs74680427, rs111796700, rs115298081, rs72626930, rs76856038, rs77836520, rs116341583, rs114506918 et rs745742418, et que le SNP en déséquilibre de liaison avec rs62256378 affiche un coefficient $r^2$ valant au moins 0,5.

2. Procédé conforme à la revendication 1, dans lequel le patient n'est pas porteur d'un gène de l'isoforme 4 de l'apolipoprotéine E, ou est hétérozygote pour ledit gène.

3. Médicament approprié pour utilisation dans le traitement d'une maladie ou d'un état associé(e) à la formation d'agrégats d'A$\beta_{1-42}$ chez des patients porteurs d'un nucléotide autre qu'une adénine en rs62256378 et/ou d'un autre allèle en au moins un SNP en déséquilibre de liaison avec rs62256378, comme déterminé par un procédé conforme à l'une des revendications 1 et 2,
lequel médicament est choisi dans l'ensemble formé par les suivants : donépézil, galantamine, mémantine, rivastigmine, tacrine, vitamine E, citalopram, fluoxétine, paroxétine, sertraline, trazodone, lorazépam, oxazépam, aripiprazole, clozapine, halopéridol, olanzapine, quétiapine, rispéridone, et ziprasidone.

4. Utilisation d'un nécessaire pour établir le pronostic clinique d'un patient souffrant d'une maladie ou d'un état associé(e) à la formation d'agrégats d'A$\beta_{1-42}$, lequel nécessaire comprend au moins un moyen permettant de détecter la présence d'une adénine ou d'une guanine en le SNP rs62256378, comprenant un oligonucléotide comportant au moins huit bases de la Séquence N° 1 ou un complémentaire d'un tel oligonucléotide, dans lequel ces bases au nombre d'au moins huit comprennent celle de la position 27.

5. Procédé de passage au crible de composés pour identifier des candidats thérapeutiques permettant de moduler une maladie ou un état associé(e) à la formation d'agrégats d'A$\beta_{1-42}$, lequel procédé comporte les étapes suivantes :

   a) mettre :

      i) une cellule de cerveau, du sang ou de foie, capable d'exprimer la SUCLG2 (sous-unité $\beta$ GTP-spécifique de la succinyl-CoA ligase) *in vitro,*
      ii) ou une cellule qui exprime la SUCLG2 *in vitro,*
      iii) ou une solution contenant de la SUCLG2,
      en contact avec le composé testé,
      iv) ou administrer le composé testé à un animal de test, lequel animal est un animal non-humain,

   b) et déterminer :

      dans le cas (i), si ledit composé testé augmente le niveau d'expression ou l'activité de SUCLG2 *in vitro,*
      dans le cas (ii), si ledit composé testé augmente le niveau d'expression ou l'activité de SUCLG2 *in vitro,*
      dans le cas (iii), si ledit composé testé augmente l'activité de SUCLG2,
      et dans le cas (iv), si ledit composé testé augmente le niveau d'expression ou l'activité de SUCLG2,
      étant entendu qu'une augmentation de ce niveau d'expression ou de cette activité est l'indice de ce que ledit composé testé est un bon candidat pour le traitement *in vivo* de ladite maladie ou dudit état.

6. Procédé conforme à la revendication 5, dans lequel la cellule de cerveau est une cellule microgliale.

7. Procédé conforme à la revendication 5 ou 6, dans lequel la cellule n'est pas porteuse d'un gène de l'isoforme 4 de l'apolipoprotéine E, ou est hétérozygote pour ledit gène

8. Procédé conforme à l'une des revendications 5 à 7, dans lequel i) la cellule est en outre capable d'exprimer la SUCLG1 (sous-unité $\alpha$ de la SUCL), ou exprime en outre la SUCLG1, ou ii) la solution contient en outre de la SUCLG1.

9. Procédé conforme à l'une des revendications 5 à 8, dans lequel l'activité de SUCLG2 est de catalyser la réaction entre succinate, GTP et Coenzyme A qui produit du succinyl-CoA et du GDP.

10. Procédé conforme à l'une des revendications 5 à 9, dans lequel le composé testé est une petite molécule, un peptide ou un anticorps agoniste.

Figure 1

Figure 2

Figure 3

Figure 4

60

Figure 5

SUCLG2-A(+)

SUCLG2-A(-)

MMSE score

Time (months)

Figure 6

EP 3 077 533 B1

Figure 7

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006028586 A2
- WO 2011007155 A1
- US 20030118592 A
- US 20030133939 A
- EP 13196119 A

### Non-patent literature cited in the description

- A multilingual glossary of biotechnological terms: (IUPAC Recommendations). Helvetica Chimica Acta, 1995
- Molecular Cloning: A Laboratory Manual. Cold Spring Harbor Laboratory Press, 1989
- **VANLIERE ; ROSENBERG.** *Theoretical Population Biology,* 2008, vol. 74, 130-137
- **KITAZAWA et al.** *The Journal of Neuroscience,* 2009, vol. 29, 6131-611
- **FOLSTEIN MF ; FOLSTEIN SE ; MCHUGH PR.** *Journal of Psychiatric Research,* 1975, vol. 12 (3), 189-98
- **DOWDY ; WEARDEN.** Statistics for Research. John Wiley & Sons, 1983
- **PADLAN et al.** *FASEB J.,* 1995, vol. 9, 133-139
- **VAJDOS et al.** *J Mol Biol,* 2002, vol. 320, 415-428
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **HEAP CJ et al.** *J. Gen. Virol.,* 2005, vol. 86, 1791-1800
- **QIU X-Q et al.** *Nature biotechnology,* 2007, vol. 25 (8), 921-929
- **KIM, S. et al.** Genome-wide association study of CSF biomarkers Abeta1-42, t-tau, and p-tau181p in the ADNI cohort. *Neurology,* 2011, vol. 76, 69-79
- **SCHJEIDE, B.M. et al.** The role of clusterin, complement receptor 1, and phosphatidylinositol binding clathrin assembly protein in Alzheimer disease risk and cerebrospinal fluid biomarker levels. *Arch Gen Psychiatry,* 2011, vol. 68, 207-13
- **ELIAS-SONNENSCHEIN, L.S. et al.** Genetic loci associated with Alzheimer's disease and cerebrospinal fluid biomarkers in a Finnish case-control cohort. *PLoS One,* 2013, vol. 8, e59676
- **HAN, M.R. ; SCHELLENBERG, G.D. ; WANG, L.S.** Genome-wide association reveals genetic effects on human Abeta42 and tau protein levels in cerebrospinal fluids: a case control study. *BMC Neurol,* 2010, vol. 10, 90
- **CRUCHAGA, C. et al.** GWAS of cerebrospinal fluid tau levels identifies risk variants for Alzheimer's disease. *Neuron,* 2013, vol. 78, 256-68
- **MATTIACE, L.A. ; DAVIES, P. ; DICKSON, D.W.** Detection of HLA-DR on microglia in the human brain is a function of both clinical and technical factors. *Am J Pathol,* 1990, vol. 136, 1101-14
- **JOHNSON, J.D. ; MEHUS, J.G. ; TEWS, K. ; MILAVETZ, B.I. ; LAMBETH, D.O.** Genetic evidence for the expression of ATP- and GTP-specific succinyl-CoA synthetases in multicellular eucaryotes. *J Biol Chem,* 1998, vol. 273, 27580-6
- **OSTERGAARD, E.** Disorders caused by deficiency of succinate-CoA ligase. *J Inherit Metab Dis,* 2008, vol. 31, 226-9
- **SILVA, D.F. et al.** Mitochondrial abnormalities in Alzheimer's disease: possible targets for therapeutic intervention. *Adv Pharmacol,* 2012, vol. 64, 83-126
- **MILLER, C. ; WANG, L. ; OSTERGAARD, E. ; DAN, P. ; SAADA, A.** The interplay between SUCLA2, SUCLG2, and mitochondrial DNA depletion. *Biochim Biophys Acta,* 2011, vol. 1812, 625-9
- **MAWUENYEGA, K.G. et al.** Decreased clearance of CNS beta-amyloid in Alzheimer's disease. *Science,* 2010, vol. 330, 1774
- **BRADSHAW, E.M. et al.** CD33 Alzheimer's disease locus: altered monocyte function and amyloid biology. *Nat Neurosci,* 2013, vol. 16, 848-50
- **HENEKA, M.T. et al.** NLRP3 is activated in Alzheimer's disease and contributes to pathology in APP/PS1 mice. *Nature,* 2013, vol. 493, 674-8
- **BALOYANNIS, S.J.** Mitochondrial alterations in Alzheimer's disease. *J Alzheimers Dis,* 2006, vol. 9, 119-26

- **VAN DER FLIER, W.M. ; PIJNENBURG, Y.A. ; FOX, N.C. ; SCHELTENS, P.** Early-onset versus late-onset Alzheimer's disease: the case of the missing APOE varepsilon4 allele. *Lancet Neurol,* 2011, vol. 10, 280-8
- **MCKHANN, G. et al.** Clinical diagnosis of Alzheimer's disease: report of the NINCDS-ADRDA Work Group under the auspices of Department of Health and Human Services Task Force on Alzheimer's Disease. *Neurology,* 1984, vol. 34, 939-44
- **KORNHUBER, J. et al.** Early and differential diagnosis of dementia and mild cognitive impairment: design and cohort baseline characteristics of the German Dementia Competence Network. *Dement Geriatr Cogn Disord,* 2009, vol. 27, 404-17
- **JESSEN, F. et al.** Prediction of dementia in primary care patients. *PLoS One,* 2011, vol. 6, e16852
- **LUCK, T. et al.** Mild cognitive impairment in general practice: age-specific prevalence and correlate results from the German study on ageing, cognition and dementia in primary care patients (AgeCoDe). *Dement Geriatr Cogn Disord,* 2007, vol. 24, 307-16
- **ZAUDIG, M. et al.** SIDAM--A structured interview for the diagnosis of dementia of the Alzheimer type, multi-infarct dementia and dementias of other aetiology according to ICD-10 and DSM-III-R. *Psychol Med,* 1991, vol. 21, 225-36
- **MCKHANN, G.M. et al.** The diagnosis of dementia due to Alzheimer's disease: recommendations from the National Institute on Aging-Alzheimer's Association workgroups on diagnostic guidelines for Alzheimer's disease. *Alzheimers Dement,* 2011, vol. 7, 263-9
- **KEMPEN, G.I. ; MEIER, A.J. ; BOUWENS, S.F. ; VAN DEURSEN, J. ; VERHEY, F.R.** The psychometric properties of the Dutch version of the Telephone Interview Cognitive Status (TICS). *Tijdschr Gerontol Geriatr,* 2007, vol. 38, 38-45
- **SCHMERMUND, A. et al.** Assessment of clinically silent atherosclerotic disease and established and novel risk factors for predicting myocardial infarction and cardiac death in healthy middle-aged subjects: rationale and design of the Heinz Nixdorf RECALL Study. Risk Factors, Evaluation of Coronary Calcium and Lifestyle. *Am Heart J,* 2002, vol. 144, 212-8
- **KRAWCZAK, M. et al.** PopGen: population-based recruitment of patients and controls for the analysis of complex genotype-phenotype relationships. *Community Genet,* 2006, vol. 9, 55-61
- **WICHMANN, H.E. ; GIEGER, C. ; ILLIG, T.** KORA-gen--resource for population genetics, controls and a broad spectrum of disease phenotypes. *Gesundheitswesen,* 2005, vol. 67 (1), 26-30
- **POPP, J. et al.** Cerebrospinal fluid markers for Alzheimer's disease over the lifespan: effects of age and the APOEepsilon4 genotype. *J Alzheimers Dis,* 2010, vol. 22, 459-68
- **MULDER, C. et al.** Amyloid-beta(1-42), total tau, and phosphorylated tau as cerebrospinal fluid biomarkers for the diagnosis of Alzheimer disease. *Clin Chem,* 2010, vol. 56, 248-53
- **HAROLD, D. et al.** Genome-wide association study identifies variants at CLU and PICALM associated with Alzheimer's disease. *Nat Genet,* 2009, vol. 41, 1088-93
- **HOLLINGWORTH, P. et al.** Common variants at ABCA7, MS4A6A/MS4A4E, EPHA1, CD33 and CD2AP are associated with Alzheimer's disease. *Nat Genet,* 2011, vol. 43, 429-35
- **LAMBERT, J.C. et al.** Genome-wide association study identifies variants at CLU and CR1 associated with Alzheimer's disease. *Nat Genet,* 2009, vol. 41, 1094-9
- **NAJ, A.C. et al.** Common variants at MS4A4/MS4A6E, CD2AP, CD33 and EPHA1 are associated with late-onset Alzheimer's disease. *Nat Genet,* 2011, vol. 43, 436-41
- **MANGOLD, E. et al.** Genome-wide association study identifies two susceptibility loci for nonsyndromic cleft lip with or without cleft palate. *Nat Genet,* 2010, vol. 42, 24-6
- **ABECASIS, G.R. et al.** A map of human genome variation from population-scale sequencing. *Nature,* 2010, vol. 467, 1061-73
- **HOWIE, B.N. ; DONNELLY, P. ; MARCHINI, J.** A flexible and accurate genotype imputation method for the next generation of genome-wide association studies. *PLoS Genet,* 2009, vol. 5, e1000529
- **PURCELL, S. et al.** PLINK: a tool set for whole-genome association and population-based linkage analyses. *Am J Hum Genet,* 2007, vol. 81, 559-75
- **DE BAKKER, P.I. et al.** Practical aspects of imputation-driven meta-analysis of genome-wide association studies. *Hum Mol Genet,* 2008, vol. 17, R122-8
- **MEESTERS, C. et al.** Quick, "imputation-free" meta-analysis with proxy-SNPs. *BMC Bioinformatics,* 2012, vol. 13, 231
- **HUEDO-MEDINA, T.B. ; SANCHEZ-MECA, J. ; MARIN-MARTINEZ, F. ; BOTELLA, J.** Assessing heterogeneity in meta-analysis: Q statistic or I2 index?. *Psychol Methods,* 2006, vol. 11, 193-206
- **HEROLD, C. ; STEFFENS, M. ; BROCKSCHMIDT, F.F. ; BAUR, M.P. ; BECKER, T.** INTERSNP: genome-wide interaction analysis guided by a priori information. *Bioinformatics,* 2009, vol. 25, 3275-81

- **CORDELL, H.J. ; CLAYTON, D.G.** A unified stepwise regression procedure for evaluating the relative effects of polymorphisms within a gene using case/control or family data: application to HLA in type 1 diabetes. *Am J Hum Genet,* 2002, vol. 70, 124-41

SEQUENCE LISTING

<110> Life & Brain GmbH Rheinische Friedrich-Wilhelms Universität Bonn

<120> Means and methods for establishing a clinical prognosis of diseases associated with the formation of aggregates of Abetal-42

<130> 456-12 PCT

<150> EP 13 196 119.5
<151> December 6, 2013

<160> 38

<170> BiSSAP 1.0

<210> 1
<211> 52
<212> DNA
<213> Homo sapiens

<220>
<221> source
<222> 1..52
<223> /mol_type="DNA" /organism="Homo sapiens"

<400> 1
gtatacaggt ggaggaacag aaaagaatgt ggtgcatttg agaaactgaa ca       52

<210> 2
<211> 52
<212> DNA
<213> Homo sapiens

<220>
<221> source
<222> 1..52
<223> /mol_type="DNA" /organism="Homo sapiens"

<400> 2
gtatacaggt ggaggaacag aaaagagtgt ggtgcatttg agaaactgaa ca       52

<210> 3
<211> 52
<212> DNA
<213> Homo sapiens

<220>
<221> source
<222> 1..52
<223> /mol_type="DNA" /organism="Homo sapiens"

<400> 3
gcccgtctct gtcacaacta cttacctttg ttgcagcatg aaagcaatca ta       52

<210> 4
<211> 52
<212> DNA
<213> Homo sapiens

<220>
<221> source
<222> 1..52
<223> /mol_type="DNA" /organism="Homo sapiens"

<400> 4
gcccgtctct gtcacaacta cttacccttg ttgcagcatg aaagcaatca ta       52

<210> 5
<211> 52

<212> DNA
<213> Homo sapiens

<220>
<221> source
<222> 1..52
<223> /mol_type="DNA" /organism="Homo sapiens"

<400> 5
caatctgtcc ttattctgtc tattcttttc ttcctaaatt cttcacttta ct          52

<210> 6
<211> 52
<212> DNA
<213> Homo sapiens

<220>
<221> source
<222> 1..52
<223> /mol_type="DNA" /organism="Homo sapiens"

<400> 6
caatctgtcc ttattctgtc tattctcttc ttcctaaatt cttcacttta ct          52

<210> 7
<211> 52
<212> DNA
<213> Homo sapiens

<220>
<221> source
<222> 1..52
<223> /mol_type="DNA" /organism="Homo sapiens"

<400> 7
atgcttctgt agttgtcagc agacagagta gtagtggtta agtcaggtat ta          52

<210> 8
<211> 52
<212> DNA
<213> Homo sapiens

<220>
<221> source
<222> 1..52
<223> /mol_type="DNA" /organism="Homo sapiens"

<400> 8
atgcttctgt agttgtcagc agacagggta gtagtggtta agtcaggtat ta          52

<210> 9
<211> 52
<212> DNA
<213> Homo sapiens

<220>
<221> source
<222> 1..52

<223> /mol_type="DNA" /organism="Homo sapiens"

<400> 9
ttcctttaac cccacatata ttgtcactat caatattttt aaaaataaac tt          52

<210> 10
<211> 52
<212> DNA
<213> Homo sapiens

<220>
<221> source
<222> 1..52
<223> /mol_type="DNA" /organism="Homo sapiens"

<400> 10
ttcctttaac cccacatata ttgtcattat caatattttt aaaaataaac tt          52

<210> 11
<211> 52
<212> DNA
<213> Homo sapiens

<220>
<221> source
<222> 1..52
<223> /mol_type="DNA" /organism="Homo sapiens"

<400> 11
atcactcact ctgggcaagc caactgtcaa gccataagga cggacactca ag          52

<210> 12
<211> 52
<212> DNA
<213> Homo sapiens

<220>
<221> source
<222> 1..52
<223> /mol_type="DNA" /organism="Homo sapiens"

<400> 12
atcactcact ctgggcaagc caactgccaa gccataagga cggacactca ag          52

<210> 13
<211> 52
<212> DNA
<213> Homo sapiens

<220>
<221> source
<222> 1..52
<223> /mol_type="DNA" /organism="Homo sapiens"

<400> 13
acatactggc ttcccatctg ctcctcttcc caaatcttac agaaaacact tt          52

<210> 14

<211> 52
<212> DNA
<213> Homo sapiens

<220>
<221> source
<222> 1..52
<223> /mol_type="DNA" /organism="Homo sapiens"

<400> 14
acatactggc ttcccatctg ctcctcctcc caaatcttac agaaaacact tt          52

<210> 15
<211> 52
<212> DNA
<213> Homo sapiens

<220>
<221> source
<222> 1..52
<223> /mol_type="DNA" /organism="Homo sapiens"

<400> 15
tctttttttt tttttagtag agatggaatt tcaccatatt ggccaggctg gt          52

<210> 16
<211> 52
<212> DNA
<213> Homo sapiens

<220>
<221> source
<222> 1..52
<223> /mol_type="DNA" /organism="Homo sapiens"

<400> 16
tctttttttt tttttagtag agatgggatt tcaccatatt ggccaggctg gt          52

<210> 17
<211> 52
<212> DNA
<213> Homo sapiens

<220>
<221> source
<222> 1..52
<223> /mol_type="DNA" /organism="Homo sapiens"

<400> 17
aaggttgtaa gtttcaagta ttttccttgt gtggccatga attatgccta tt          52

<210> 18
<211> 52
<212> DNA
<213> Homo sapiens

<220>
<221> source

<222> 1..52
<223> /mol_type="DNA" /organism="Homo sapiens"

<400> 18
aaggttgtaa gtttcaagta ttttccatgt gtggccatga attatgccta tt        52

<210> 19
<211> 52
<212> DNA
<213> Homo sapiens

<220>
<221> source
<222> 1..52
<223> /mol_type="DNA" /organism="Homo sapiens"

<400> 19
tgcagcttgg ataattgatg tttgtacgcc tggatttaat aaaaaatact ct        52

<210> 20
<211> 52
<212> DNA
<213> Homo sapiens

<220>
<221> source
<222> 1..52
<223> /mol_type="DNA" /organism="Homo sapiens"

<400> 20
tgcagcttgg ataattgatg tttgtatgcc tggatttaat aaaaaatact ct        52

<210> 21
<211> 52
<212> DNA
<213> Homo sapiens

<220>
<221> source
<222> 1..52
<223> /mol_type="DNA" /organism="Homo sapiens"

<400> 21
ataatgcagt atctatattt tatttgcaat gggagctaca ttggtgcaga tt        52

<210> 22
<211> 52
<212> DNA
<213> Homo sapiens

<220>
<221> source
<222> 1..52
<223> /mol_type="DNA" /organism="Homo sapiens"

<400> 22
ataatgcagt atctatattt tatttgtaat gggagctaca ttggtgcaga tt        52

<210> 23
<211> 52
<212> DNA
<213> Homo sapiens

<220>
<221> source
<222> 1..52
<223> /mol_type="DNA" /organism="Homo sapiens"

<400> 23
agaaagctat tttgattctc tgaaaataaa gcttcagagg ataagaaaga tg          52

<210> 24
<211> 52
<212> DNA
<213> Homo sapiens

<220>
<221> source
<222> 1..52
<223> /mol_type="DNA" /organism="Homo sapiens"

<400> 24
agaaagctat tttgattctc tgaaaagaaa gcttcagagg ataagaaaga tg          52

<210> 25
<211> 52
<212> DNA
<213> Homo sapiens

<220>
<221> source
<222> 1..52
<223> /mol_type="DNA" /organism="Homo sapiens"

<400> 25
atcctgtcat ttaatactca tagcaatcct gctgagttgt ttctattaat at          52

<210> 26
<211> 52
<212> DNA
<213> Homo sapiens

<220>
<221> source
<222> 1..52
<223> /mol_type="DNA" /organism="Homo sapiens"

<400> 26
atcctgtcat ttaatactca tagcaaccct gctgagttgt ttctattaat at          52

<210> 27
<211> 52
<212> DNA
<213> Homo sapiens

<220>

<210> source
<222> 1..52
<223> /mol_type="DNA" /organism="Homo sapiens"

<400> 27
ggatacacaa gtatcaatag ctagattgat caagtggaag aaaggatatc ag          52

<210> 28
<211> 52
<212> DNA
<213> Homo sapiens

<220>
<221> source
<222> 1..52
<223> /mol_type="DNA" /organism="Homo sapiens"

<400> 28
ggatacacaa gtatcaatag ctagatcgat caagtggaag aaaggatatc ag          52

<210> 29
<211> 52
<212> DNA
<213> Homo sapiens

<220>
<221> source
<222> 1..52
<223> /mol_type="DNA" /organism="Homo sapiens"

<400> 29
gtctcacagt gtttgcaggt cagaagcctg gcaggatgta actggatttt ct          52

<210> 30
<211> 52
<212> DNA
<213> Homo sapiens

<220>
<221> source
<222> 1..52
<223> /mol_type="DNA" /organism="Homo sapiens"

<400> 30
gtctcacagt gtttgcaggt cagaagtctg gcaggatgta actggatttt ct          52

<210> 31
<211> 42
<212> PRT
<213> Homo sapiens

<220>
<221> SOURCE
<222> 1..42
<223> /mol_type="protein" /organism="Homo sapiens"

<400> 31

```
        Asp Ala Glu Phe Arg His Asp Ser Gly Tyr Glu Val His His Gln Lys
        1               5                   10                      15
        Leu Val Phe Phe Ala Glu Asp Val Gly Ser Asn Lys Gly Ala Ile Ile
                    20                  25                  30
        Gly Leu Met Val Gly Gly Val Val Ile Ala
                    35                  40
```

<210> 32
<211> 43
<212> PRT
<213> Homo sapiens

<220>
<221> SOURCE
<222> 1..43
<223> /mol_type="protein" /organism="Homo sapiens"

<400> 32

```
        Asp Ala Glu Phe Arg His Asp Ser Gly Tyr Glu Val His His Gln Lys
        1               5                   10                      15
        Leu Val Phe Phe Ala Glu Asp Val Gly Ser Asn Lys Gly Ala Ile Ile
                    20                  25                  30
        Gly Leu Met Val Gly Gly Val Val Ile Ala Thr
                    35                  40
```

<210> 33
<211> 432
<212> PRT
<213> Homo sapiens

<220>
<221> SOURCE
<222> 1..432
<223> /mol_type="protein" /organism="Homo sapiens"

<400> 33

```
Met Ala Ser Pro Val Ala Ala Gln Ala Gly Lys Leu Leu Arg Ala Leu
1               5                   10                  15
Ala Leu Arg Pro Arg Phe Leu Ala Ala Gly Ser Gln Ala Val Gln Leu
        20                  25                  30
Thr Ser Arg Arg Trp Leu Asn Leu Gln Glu Tyr Gln Ser Lys Lys Leu
        35                  40                  45
Met Ser Asp Asn Gly Val Arg Val Gln Arg Phe Phe Val Ala Asp Thr
    50                  55                  60
Ala Asn Glu Ala Leu Glu Ala Ala Lys Arg Leu Asn Ala Lys Glu Ile
65                  70                  75                  80
Val Leu Lys Ala Gln Ile Leu Ala Gly Gly Arg Gly Lys Gly Val Phe
                85                  90                  95
Asn Ser Gly Leu Lys Gly Gly Val His Leu Thr Lys Asp Pro Asn Val
            100                 105                 110
Val Gly Gln Leu Ala Lys Gln Met Ile Gly Tyr Asn Leu Ala Thr Lys
            115                 120                 125
Gln Thr Pro Lys Glu Gly Val Lys Val Asn Lys Val Met Val Ala Glu
    130                 135                 140
Ala Leu Asp Ile Ser Arg Glu Thr Tyr Leu Ala Ile Leu Met Asp Arg
145                 150                 155                 160
Ser Cys Asn Gly Pro Val Leu Val Gly Ser Pro Gln Gly Gly Val Asp
            165                 170                 175
Ile Glu Glu Val Ala Ala Ser Asn Pro Glu Leu Ile Phe Lys Glu Gln
            180                 185                 190
Ile Asp Ile Phe Glu Gly Ile Lys Asp Ser Gln Ala Gln Arg Met Ala
            195                 200                 205
Glu Asn Leu Gly Phe Val Gly Pro Leu Lys Ser Gln Ala Ala Asp Gln
    210                 215                 220
Ile Thr Lys Leu Tyr Asn Leu Phe Leu Lys Ile Asp Ala Thr Gln Val
225                 230                 235                 240
Glu Val Asn Pro Phe Gly Glu Thr Pro Glu Gly Gln Val Val Cys Phe
            245                 250                 255
Asp Ala Lys Ile Asn Phe Asp Asp Asn Ala Glu Phe Arg Gln Lys Asp
            260                 265                 270
Ile Phe Ala Met Asp Asp Lys Ser Glu Asn Glu Pro Ile Glu Asn Glu
    275                 280                 285
Ala Ala Lys Tyr Asp Leu Lys Tyr Ile Gly Leu Asp Gly Asn Ile Ala
    290                 295                 300
Cys Phe Val Asn Gly Ala Gly Leu Ala Met Ala Thr Cys Asp Ile Ile
305                 310                 315                 320
Phe Leu Asn Gly Gly Lys Pro Ala Asn Phe Leu Asp Leu Gly Gly Gly
            325                 330                 335
Val Lys Glu Ala Gln Val Tyr Gln Ala Phe Lys Leu Leu Thr Ala Asp
            340                 345                 350
Pro Lys Val Glu Ala Ile Leu Val Asn Ile Phe Gly Gly Ile Val Asn
            355                 360                 365
Cys Ala Ile Ile Ala Asn Gly Ile Thr Lys Ala Cys Arg Glu Leu Glu
    370                 375                 380
Leu Lys Val Pro Leu Val Val Arg Leu Glu Gly Thr Asn Val Gln Glu
385                 390                 395                 400

Ala Gln Lys Ile Leu Asn Asn Ser Gly Leu Pro Ile Thr Ser Ala Ile
            405                 410                 415
Asp Leu Glu Asp Ala Ala Lys Lys Ala Val Ala Ser Val Ala Lys Lys
        420                 425                 430
```

<210> 34

<211> 440

<212> PRT

<213> Homo sapiens

<220>
<221> SOURCE
<222> 1..440
<223> /mol_type="protein" /organism="Homo sapiens"

<400> 34

```
Met Ala Ser Pro Val Ala Ala Gln Ala Gly Lys Leu Leu Arg Ala Leu
1               5                   10                  15
Ala Leu Arg Pro Arg Phe Leu Ala Ala Gly Ser Gln Ala Val Gln Leu
            20                  25                  30
Thr Ser Arg Arg Trp Leu Asn Leu Gln Glu Tyr Gln Ser Lys Lys Leu
        35                  40                  45
Met Ser Asp Asn Gly Val Arg Val Gln Arg Phe Phe Val Ala Asp Thr
    50                  55                  60
Ala Asn Glu Ala Leu Glu Ala Ala Lys Arg Leu Asn Ala Lys Glu Ile
65                  70                  75                  80
Val Leu Lys Ala Gln Ile Leu Ala Gly Gly Arg Gly Lys Gly Val Phe
                85                  90                  95
Asn Ser Gly Leu Lys Gly Gly Val His Leu Thr Lys Asp Pro Asn Val
            100                 105                 110
Val Gly Gln Leu Ala Lys Gln Met Ile Gly Tyr Asn Leu Ala Thr Lys
            115                 120                 125
Gln Thr Pro Lys Glu Gly Val Lys Val Asn Lys Val Met Val Ala Glu
    130                 135                 140
Ala Leu Asp Ile Ser Arg Glu Thr Tyr Leu Ala Ile Leu Met Asp Arg
145                 150                 155                 160
Ser Cys Asn Gly Pro Val Leu Val Gly Ser Pro Gln Gly Gly Val Asp
            165                 170                 175
Ile Glu Glu Val Ala Ala Ser Asn Pro Glu Leu Ile Phe Lys Glu Gln
            180                 185                 190
Ile Asp Ile Phe Glu Gly Ile Lys Asp Ser Gln Ala Gln Arg Met Ala
    195                 200                 205
Glu Asn Leu Gly Phe Val Gly Pro Leu Lys Ser Gln Ala Ala Asp Gln
    210                 215                 220
Ile Thr Lys Leu Tyr Asn Leu Phe Leu Lys Ile Asp Ala Thr Gln Val
225                 230                 235                 240
Glu Val Asn Pro Phe Gly Glu Thr Pro Glu Gly Gln Val Val Cys Phe
            245                 250                 255
Asp Ala Lys Ile Asn Phe Asp Asp Asn Ala Glu Phe Arg Gln Lys Asp
            260                 265                 270
Ile Phe Ala Met Asp Asp Lys Ser Glu Asn Glu Pro Ile Glu Asn Glu
    275                 280                 285
Ala Ala Lys Tyr Asp Leu Lys Tyr Ile Gly Leu Asp Gly Asn Ile Ala
    290                 295                 300
Cys Phe Val Asn Gly Ala Gly Leu Ala Met Ala Thr Cys Asp Ile Ile
305                 310                 315                 320
Phe Leu Asn Gly Gly Lys Pro Ala Asn Phe Leu Asp Leu Gly Gly Gly
            325                 330                 335
Val Lys Glu Ala Gln Val Tyr Gln Ala Phe Lys Leu Leu Thr Ala Asp
            340                 345                 350
Pro Lys Val Glu Ala Ile Leu Val Asn Ile Phe Gly Gly Ile Val Asn
            355                 360                 365
```

```
Cys Ala Ile Ile Ala Asn Gly Ile Thr Lys Ala Cys Arg Glu Leu Glu
    370                 375                 380
Leu Lys Val Pro Leu Val Val Arg Leu Glu Gly Thr Phe Met Glu Lys
385                 390                 395                 400
Lys Gly Ser Tyr Met His Ile Lys Gln Glu Thr Gly Asn Ser Asn Glu
                405                 410                 415
Asn Ile Thr Gly Ile Gln Glu Asn Val Ala His Gln Asn Leu Ser Lys
                420                 425                 430
Cys Ala Ile Ser Ile Phe Leu Cys
                435                 440
```

<210> 35
<211> 346
<212> PRT
<213> Homo sapiens

<220>
<221> SOURCE
<222> 1..346
<223> /mol_type="protein" /organism="Homo sapiens"

<400> 35

EP 3 077 533 B1

```
Met Thr Ala Thr Leu Ala Ala Ala Ala Asp Ile Ala Thr Met Val Ser
1               5                   10                  15
Gly Ser Ser Gly Leu Ala Ala Ala Arg Leu Leu Ser Arg Ser Phe Leu
            20                  25                  30
Leu Pro Gln Asn Gly Ile Arg His Cys Ser Tyr Thr Ala Ser Arg Gln
            35                  40                  45
His Leu Tyr Val Asp Lys Asn Thr Lys Ile Ile Cys Gln Gly Phe Thr
        50                  55                  60
Gly Lys Gln Gly Thr Phe His Ser Gln Gln Ala Leu Glu Tyr Gly Thr
65                  70                  75                  80
Lys Leu Val Gly Gly Thr Thr Pro Gly Lys Gly Gly Gln Thr His Leu
                85                  90                  95
Gly Leu Pro Val Phe Asn Thr Val Lys Glu Ala Lys Glu Gln Thr Gly
            100                 105                 110
Ala Thr Ala Ser Val Ile Tyr Val Pro Pro Phe Ala Ala Ala Ala
            115                 120                 125
Ile Asn Glu Ala Ile Glu Ala Glu Ile Pro Leu Val Val Cys Ile Thr
    130                 135                 140
Glu Gly Ile Pro Gln Gln Asp Met Val Arg Val Lys His Lys Leu Leu
145                 150                 155                 160
Arg Gln Glu Lys Thr Arg Leu Ile Gly Pro Asn Cys Pro Gly Val Ile
                165                 170                 175
Asn Pro Gly Glu Cys Lys Ile Gly Ile Met Pro Gly His Ile His Lys
            180                 185                 190
Lys Gly Arg Ile Gly Ile Val Ser Arg Ser Gly Thr Leu Thr Tyr Glu
            195                 200                 205
Ala Val His Gln Thr Thr Gln Val Gly Leu Gly Gln Ser Leu Cys Val
    210                 215                 220
Gly Ile Gly Gly Asp Pro Phe Asn Gly Thr Asp Phe Ile Asp Cys Leu
225                 230                 235                 240
Glu Ile Phe Leu Asn Asp Ser Ala Thr Glu Gly Ile Ile Leu Ile Gly
            245                 250                 255
Glu Ile Gly Gly Asn Ala Glu Glu Asn Ala Ala Glu Phe Leu Lys Gln
            260                 265                 270
His Asn Ser Gly Pro Asn Ser Lys Pro Val Val Ser Phe Ile Ala Gly
    275                 280                 285
Leu Thr Ala Pro Pro Gly Arg Arg Met Gly His Ala Gly Ala Ile Ile
    290                 295                 300
Ala Gly Gly Lys Gly Gly Ala Lys Glu Lys Ile Ser Ala Leu Gln Ser
305                 310                 315                 320

Ala Gly Val Val Val Ser Met Ser Pro Ala Gln Leu Gly Thr Thr Ile
                325                 330                 335
Tyr Lys Glu Phe Glu Lys Arg Lys Met Leu
    340                 345
```

<210> 36
<211> 24
<212> RNA
<213> artificial sequences

<220>
<221> source
<222> 1..24
<223> /mol_type="RNA" /note="siRNA" /organism="artificial sequences"

<400> 36
gcgguuugaa aggagguguu cauu          24

<210> 37

52

<211> 25
<212> RNA
<213> artificial sequences

<220>
<221> source
<222> 1..25
<223> /mol_type="RNA" /note="siRNA" /organism="artificial sequences"

<400> 37
cccuggauau uucuagagaa acaua           25

<210> 38
<211> 25
<212> RNA
<213> artificial sequences

<220>
<221> source
<222> 1..25
<223> /mol_type="RNA" /note="siRNA" /organism="artificial sequences"

<400> 38
accaggcugc agaucagauu acaaa           25